(19) Europäisches Patentamt
European Patent Office
Office européen des brevets

(11) EP 1 616 016 B1

(12) **EUROPEAN PATENT SPECIFICATION**

(45) Date of publication and mention of the grant of the patent:
**14.11.2012 Bulletin 2012/46**

(21) Application number: **04757661.6**

(22) Date of filing: **19.03.2004**

(51) Int Cl.:
***C12N 15/861*** *(2006.01)*

(86) International application number:
**PCT/US2004/008567**

(87) International publication number:
**WO 2004/083404 (30.09.2004 Gazette 2004/40)**

(54) **GENE THERAPY VECTORS HAVING REDUCED IMMUNOGENICITY BASED ON CD8 ALPHA-CHAIN**

GENTHERAPIE-VEKTOREN MIT REDUZIERTER IMMUNITÄT, DIE AUF DER VERWENDUNG VON DER CD8 ALPHA KETTE BASIEREN

VECTEURS DE THERAPIE GENIQUE PRESENTANT UNE ANTIGENICITE REDUITE, BASEE SUR LA CHAINE ALPHA DU CD8

(84) Designated Contracting States:
**AT BE BG CH CY CZ DE DK EE ES FI FR GB GR HU IE IT LI LU MC NL PL PT RO SE SI SK TR**

(30) Priority: **19.03.2003 US 456378 P**

(43) Date of publication of application:
**18.01.2006 Bulletin 2006/03**

(73) Proprietor: **Isogenis, Inc.**
**Denver, CO 80206 (US)**

(72) Inventors:
- **QI, Yan**
  **Highlands Ranch, CO 80126 (US)**
- **ZHANG, Xianghua**
  **Aurora, CO 80013 (US)**
- **KONIGSBERG, Paula, J.**
  **Denver, CO 80205 (US)**

(74) Representative: **Dzieglewska, Hanna Eva**
**Dehns**
**St Bride's House**
**10 Salisbury Square**
**London**
**EC4Y 8JD (GB)**

(56) References cited:
**WO-A-99/23229** **US-A- 5 601 828**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 616 016 B1

## Description

CROSS-REFERENCE TO RELATED APPLICATION(S)

This application claims the benefit of provisional application serial number 60/456,378, filed March 19, 2003.

FIELD OF THE INVENTION

The present invention relates generally to the field of gene therapy, and more specifically, provides methods and compositions for reducing the immunogenicity of gene therapy vectors.

BACKGROUND OF THE INVENTION

Gene delivery or gene therapy is a promising method for the treatment of acquired and inherited diseases. An ever-expanding array of genes for which abnormal expression is associated with life-threatening human diseases are being cloned and identified. The ability to express such cloned genes in humans will ultimately permit the prevention and/or cure of many important human diseases, diseases for which current therapies are either inadequate or non-existent. As an example, *in vivo* expression of cholesterol-regulating genes, genes which selectively block the replication of HIV, or of tumor-suppressing genes in human patients should dramatically improve treatment of heart disease, HIV, and cancer, respectively.

Unfortunately, however, gene therapy protocols described to date have been plagued by a variety of problems, including in particular the short period of gene expression from the vector and the inability to effectively readminister the same vector a second time, both of which are caused by the host immune response against antigens associated with the vector and its therapeutic payload. Tissues that have incorporated the viral and/or therapeutic genes are initially attacked by the host's cellular immune response, mediated by CD8+ cytotoxic T cells as well as CD4+ helper T cells, which dramatically limits the persistence of gene expression from the vectors. Moreover, the host's humoral immune response mediated by the CD4+ T cells further limits the effectiveness of current gene therapy protocols by inhibiting the successful readministration of the same vector.

For example, following an initial administration of an adenoviral vector, serotype-specific antibodies are generated against epitopes of the major viral capsid proteins, namely the penton, hexon and fiber. Given that such capsid proteins are the means by which the adenovirus attaches itself to a cell and subsequently infects the cell, such antibodies are then able to block or "neutralize" reinfection of a cell by the same serotype of adenovirus. This necessitates using a different serotype of adenovirus in order to administer one or more subsequent doses of exogenous therapeutic DNA in the context of gene therapy. In addition, both therapeutic and viral gene products are expressed on the target cells making them susceptible to cellular immune responses. Thus, they are rejected and the beneficial effect of the gene therapy is negated and the target organ or tissue may be destroyed. As a result of these immune-related obstacles, progress in gene therapy protocols has been stymied.

Accordingly, there exists a significant need in the art for effective methods of specifically inhibiting immune responses directed against gene therapy expression vectors and cells transfected by such vectors. In addition, there exists a need for improved methods and composition for administering or delivering gene therapy payloads. It is therefore an object of the present invention to specifically inhibit both the cellular and humoral immune responses directed against such gene therapy vectors and their therapeutic products, and thereby increase exogenous gene expression from cells transfected by such vectors.

SUMMARY OF THE RELEVANT LITERATURE

It is known that the activity of MHC class I-restricted T cells (*e.g.*, CD8+ CTLs) can be suppressed when a CTL that has received a signal through its T cell receptor complex also receives a signal through the a3 domain of its class I MHC molecule. This so-called veto signal may be delivered by a CD8 molecule expressed by the stimulator or "veto" cell. Sambhara and Miller, Science 252:1424-1427 (1991). The resulting immune suppression is both antigen-specific and MHC-restricted, and results from the unidirectional recognition of the veto cell by the responding CTL, but not vice versa. Rammensee et al., Eur. J. Immunol. 12:930-934 (1982); Fink et al., J. Exp. Med. 157:141-154 (1983); Rammensee et al., J. Immunol. 132:668-672 (1984). Veto activity has since been linked to the presence of the CD8 $\alpha$ chains, such that the veto function is lost if expression of CD8 is deleted and established when the CD8 $\alpha$ chain is expressed. Hambor et al., J. Immunol. 145:1646-1652 (1990); Hambor. et al., Intern. Immunol. 2:8856-8879 (1990); Kaplan et al., Proc. Natl. Acad. Sci. USA 86:8512-8515 (1989).

Numerous strategies have been proposed to exploit this antigen-specific suppressive pathway to eliminate unwanted cytotoxic T cell responses. One such strategy involves the use of polypeptide conjugates covalently linking CD8 or a functional domain thereof to secondary ligands that direct CD8's veto activity to specific target cells. *See, e.g.*, U.S. Patent Nos. 5,242,687, 5,601,828 and 5,623,056. Alternatively, hybrid antibody molecules have been investigated having a monoclonal antibody binding site with specificity to MHC class I molecules linked to the extracellular domain of the CD8 α chain. Qi et al., J. Exp. Med. 183:1973-1980 (1996). Such molecules, however, have several shortcomings and have yet to find actual clinical utility.

More recently, WO 02/102852 describes the inhibition of CTL using soluble C8α chain variants having amino acid modifications designed to increased affinity for MHC class I. Significantly, it is taught therein that the proposed CD8α compositions are specific for class I MHC molecules and are therefore expected to inhibit only the response of CTL, and further that combinations with other immunosuppressive agents will be required in situations involving other elements of the cellular and humoral immune responses, e.g., MHC class II-restricted T cells such as CD4+ T cells. Id. pp. 27-28.

SUMMARY OF THE INVENTION

The present invention is based on the surprising discovery that the veto effect mediated by targeted expression of immunomodulatory molecules such as CD8 can effectively and specifically inhibit the host immune response directed against antigens associated with an expression vector, including its exogenous genetic payload, as well as against antigens associated with the transfected target cell. The present invention is also based on the additional surprising discovery that the veto effect mediated by targeted expression of CD8a can effectively and specifically suppress responding CD4+ T cells (MHC class II-restricted) as well as CD8+ T cells (MHC class I-restricted), and the resulting determination that both the cellular and humoral components of the host immune response directed against such vector-associated antigens can be inhibited. Thus, by utilizing the methods and compositions described herein one may synergistically enhance gene therapy protocols by inhibiting the host immune responses against vector-associated antigens that currently limit gene expression from the vectors and prevent gene therapy from reaching its full potential.

Accordingly, the present invention provides compositions and methods for specifically inhibiting host immune responses directed against expression vectors as well as the target cells transfected with such vectors, wherein the vectors comprise a nucleic acid sequence encoding for an immunomodulatory molecule capable of eliciting a veto effect, preferably a CD8 polypeptide, more preferably the CD8 α-chain, and most preferably both the extracellular and transmembrane domains of the CD8 α-chain. Given the nature of the subject compositions and methods, as well as the apparent inadequacies of the prior art soluble forms of CD8 α-chain described above, the presence of the CD8 α-chain transmembrane domain or a suitable alternative transmembrane region is deemed essential.

In one aspect, the present invention provides a method for inhibiting an immune response against an expression vector, comprising contacting a target cell of the host *in vivo* or *ex vivo* with an expression vector encoding all or a functional portion of a CD8 polypeptide, preferably the CD8 α-chain, and most preferably both the extracellular and transmembrane domains of the CD8 α-chain, wherein said CD8 polypeptide is expressed on the surface of the target cell and whereby an immune response against the expression vector and the target cell is specifically inhibited. The recombinant vector preferably further comprises one or more additional transgenes encoding therapeutic proteins or molecules of interest. As described and exemplified herein, both the humoral and cellular components of the immune response are inhibited utilizing the methods and compositions of the present invention.

In another aspect, a method for the specific inhibition of a host immune response directed against vector-associated antigens is provided, comprising contacting a target cell of the host *in vivo* or *ex vivo* with an expression vector comprising a nucleic acid encoding all or a functional portion of a CD8 polypeptide, preferably a CD8 α-chain, and most preferably both the extracellular and transmembrane domains of the CD8 α-chain, wherein the CD8 polypeptide is expressed on the surface of the target cell and whereby the host immune response to vector-associated antigens is specifically inhibited.

In a further aspect the invention provides a method for improving the expression of a therapeutic transgene in a host, comprising administering to a host an expression vector comprising a nucleic acid sequence encoding for encoding all or a functional portion of a CD8 polypeptide, preferably a CD8 α-chain, and most preferably both the extracellular and transmembrane domains of the CD8 α-chain, wherein the CD8 polypeptide is expressed on the surface of a host cell and whereby the host immune response to vector-associated antigens is specifically inhibited. In one embodiment, the therapeutic transgene is included in the same vector as the CD8 polypeptide. In alternative embodiments, the CD8 polypeptide and the therapeutic molecule are encoded by separate expression vectors. As described herein, the subject method improves expression of the therapeutic transgene by inhibiting both the cellular and humoral components of the host immune response to vector-associated antigens, thereby increasing the persistence of the therapeutic transgene in the host, and enabling readministration of the expression vector for subsequent rounds of transgene expression.

In a further aspect, the invention provides improved viral expression vectors having reduced immunogenicity, wherein the expression vectors comprise non-viral nucleic acid consisting essentially of nucleic acid encoding for a CD8 polypeptide as disclosed herein and nucleic acid encoding for at least one therapeutic transgene of interest. In one embodiment, the therapeutic transgene is other than an immunomodulatory molecule. In preferred embodiments, the CD8 polypeptide comprises all or a functional portion of the CD8 α-chain. Preferably, the functional portion of the CD8 α-chain comprises at least the extracellular domain of the CD8 α-chain, and more preferably both the extracellular domain and the transmembrane domain of the CD8 α-chain. Generally, the immunomodulatory molecules provided for herein are associated with the target cell surface membrane, e.g., inserted within the membrane or covalently or non-covalently bound thereto, after transfection of the target cell.

Suitable expression vectors contemplated for use herein include recombinant and non-recombinant vectors, and viral (e.g., adenoviral, retroviral, adeno-associated viral vectors and the like) as well as non-viral (e.g., bacterial plasmids, phages, liposomes and the like) vectors. Viral vectors are preferred, and adenoviral vectors most preferred.

While multiple embodiments are disclosed, still other embodiments of the present invention will become apparent to those skilled in the art from the following detailed description, which shows and describes illustrative embodiments of the invention. As will be realized, the invention is capable of modifications in various obvious aspects, all without departing from the spirit and scope of the present invention. Accordingly, the drawings and detailed description are to be regarded as illustrative in nature and not restrictive.

## BRIEF DESCRIPTION OF THE DRAWINGS

FIG. 1 depicts CD8 α-chain protein and nucleic acid sequences from various species. Also included are accession numbers for the noted sequences.

FIGS. 2A-B depict the amino acid and nucleic acid sequences for the wild-type CD8 α-chain, including a demarcation of the different domains of the protein for the human and mouse

FIG. 3 depicts Balb/c spleen cells that were stimulated with C57BU6 spleen cells. Cultures were supplemented with normal fibroblasts (●), medium (■), or fibroblasts with CD8 (▲) of mouse (A) or human (B) origin. Cultures were harvested and tested for their lytic ability towards C57BU6-derived target cells.

FIG. 4 depicts Balb/c (H-2d) mice that were injected with control fibroblasts (■ and ▲) or mCD8-transfected C57BU6-(H-2b) derived (○ and ●) fibroblasts. After two weeks animals were sacrificed, spleen cells were harvested, stimulated with C57BL/6 (H-2b) (■ and ○) or CBA/J (H-2k) (● and ▲) spleen cells and tested for their lytic ability on EL4 (H-2b) (■ and ○) or S.AKR (H-2k) (● and ▲) target cells.

FIG. 5 depicts target cells (▲) or CD8-expressing targets (■) that were tested for their susceptibility to lysis by alloreactive T cells (A) or by antigen-specific CTLs (B).

FIG. 6 depicts MLCs (Balb/c anti-C57B/6) that were set up in the presence of normal fibroblasts (●) and fibroblasts transduced with mAdCD8 (A, ▲) or HAdCD8 (B, ▲). No fibroblasts were added to control cultures (■). The lytic activity of these cultures towards an C57BU6-derived target was determined at the end of the culture period.

FIG. 7 depicts immunization with an adenoviral veto transfer vector, mAdCD8. C57BL/6 mice were infected with the vectors indicated above. After 10 days, spleen cells were harvested and cultured in the presence of the Adβgal virus. The number of blast cells is given.

FIG. 8 depicts negative immunization with mAdCD8 (A) C57BU6 mice were once immunized i.v. with Adβgal or mAdCD8. (B) Animals treated as in (A) were re-immunized with Adβgal after 5 days. Seven days after the last injection animals were sacrificed, and their spleen cells were cultured in the presence of Adβgal. After 5 days of culture, cells were tested for their lytic ability of Adβgal-infected syngeneic target cells.

FIG. 9 depicts $3x10^6$ C7BI/6 spleen cells that were incubated with $1x10^6$ (or no) stimulator cells, transduced as indicated. After 4 days the cultures were analyzed for presence $CD4^+$ T lymphoblasts by immunofluorescence.

FIGS. 10A-D depicts surface expression of mouse and human CD8 α-chains after infection with the different virus constructs. A. Infected cells: Mc57T Fibroblasts; Panel 1: Mock-Infection; Panel 2: Infection with hAdCD8. B. Infected cells: MC57T Fibroblasts; Panel 1: Mock Infection; Panel 2: Infection with mAdCD8. C. Infected cells: Balbc unselected bone marrow cells; Panel 1: Infection with lacZ Adenoviral Vector (AdLacZ); Panel 12: Infection with mAdCD8. D. Infected Cells: MC57T Fibroblasts; Panel 1: Mock-Infection; Panel 2: Infection with pAAV-mCD8; Panel 3: Infection with pAAV-hCD8.

FIG 11 depicts MLCs (Balb/c anti-C57BL/6) were set up in the presence of these fibroblasts that had been cultured for 0 or 5 hours after transduction before they were added to the MLCs. At the end of the cultures, the number of lymphoblasts was determined on a fluorescence activated cell analyzer.

FIG 12 depicts *in vitro* inhibition with veto transfer vector. A BALB/c antiC57BU6 mixed lymphocyte culture (MLC) was established in the absence or presence of uninfected or mAdCD8-infected MC57 fibroblasts (H-2b) (X). CTL responses were measured in EL4 (H-2b) target cells.

FIG 13 depicts Balb/c mice that were immunized with AdLacZ or mAdCD8. Their spleen cells were cultured in the presence of AdLacZ and tested for specific lytic activity against AdLacZ-infected syngeneic P815 target cells.

FIG 14 depicts (A) C57BU6 animals that were immunized with AdLacZ (■) or mAdCD8 (▲). The lytic activity of their spleen cells towards syngeneic AdLacZ EL4 target cells was tested. (B) Such animals were re-immunized with AdLacZ prior to testing their lytic activity against AdLacz-infected EL4 targets.

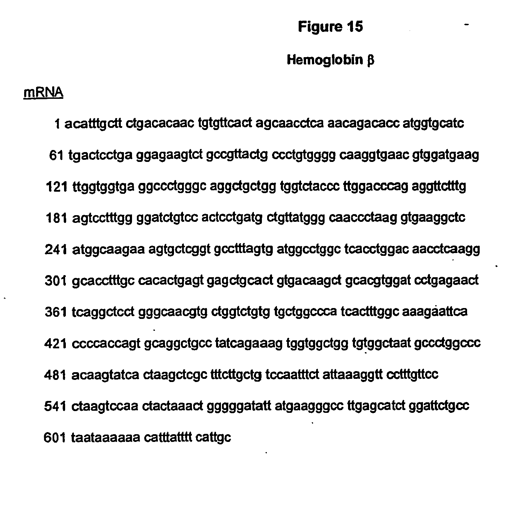

FIG 15 Depicts the mRNA sequence of Hemoglobin β.

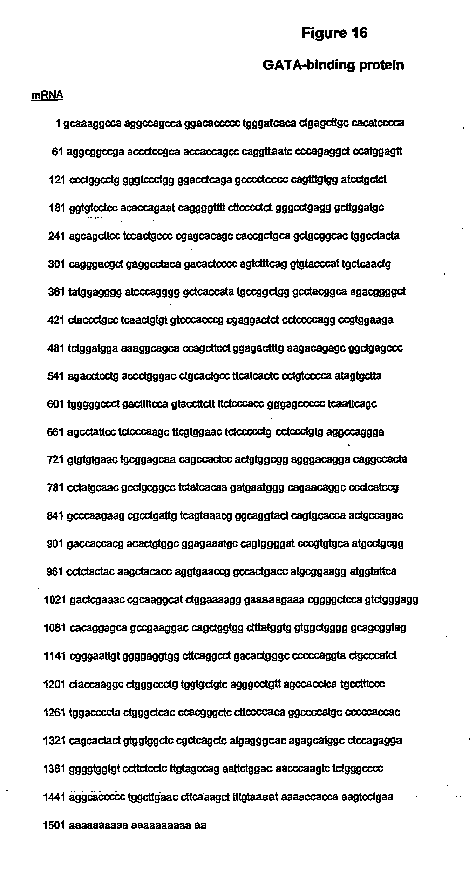

FIG 16 Depicts the mRNA sequence of GATA binding protein.

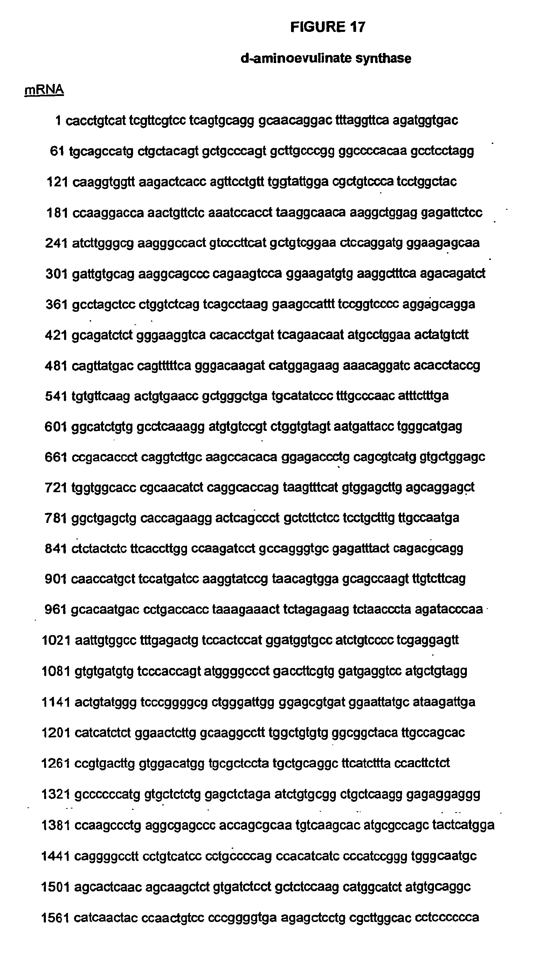

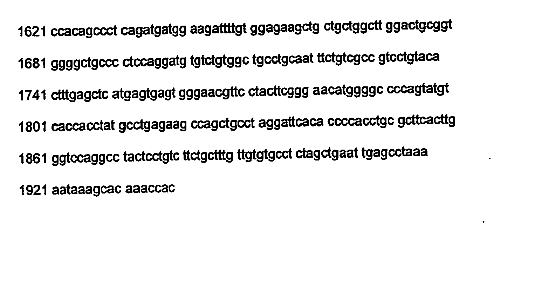

FIG 17 Depicts the mRNA sequence of d-aminoevulinate synthase.

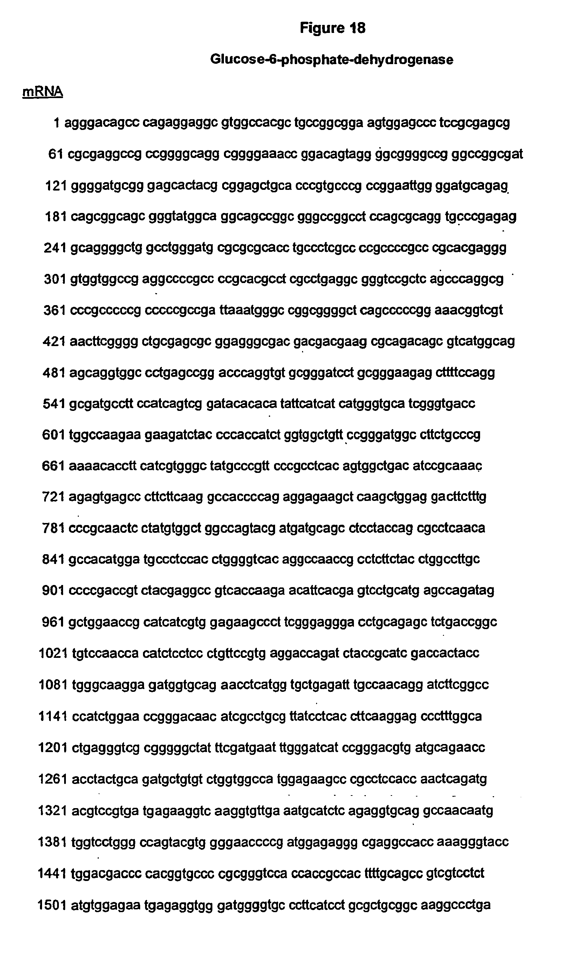

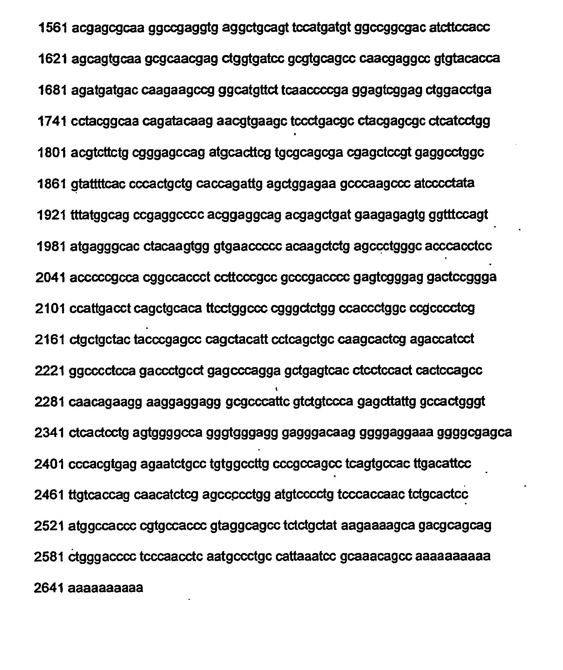

FIG 18 Depicts the mRNA sequence of Glucose-6-phosphate-dehydrogenase.

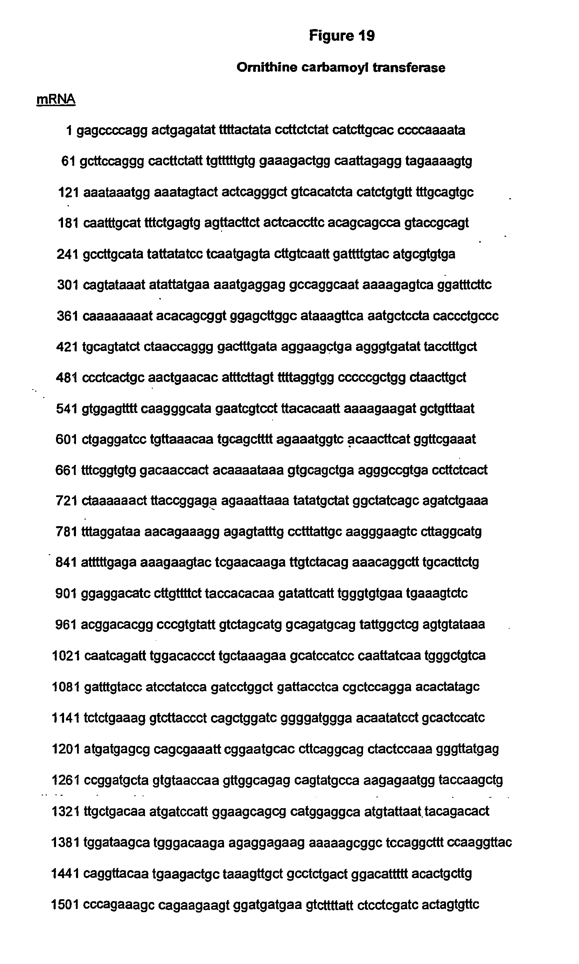

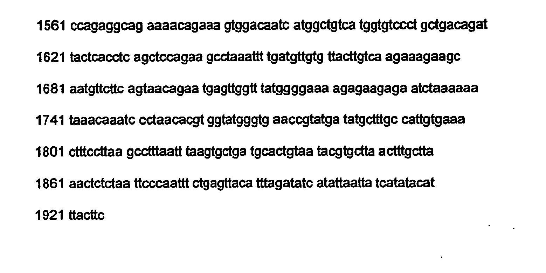

FIG 19 Depicts the mRNA sequence of Ornithine carbamoyl transferase.

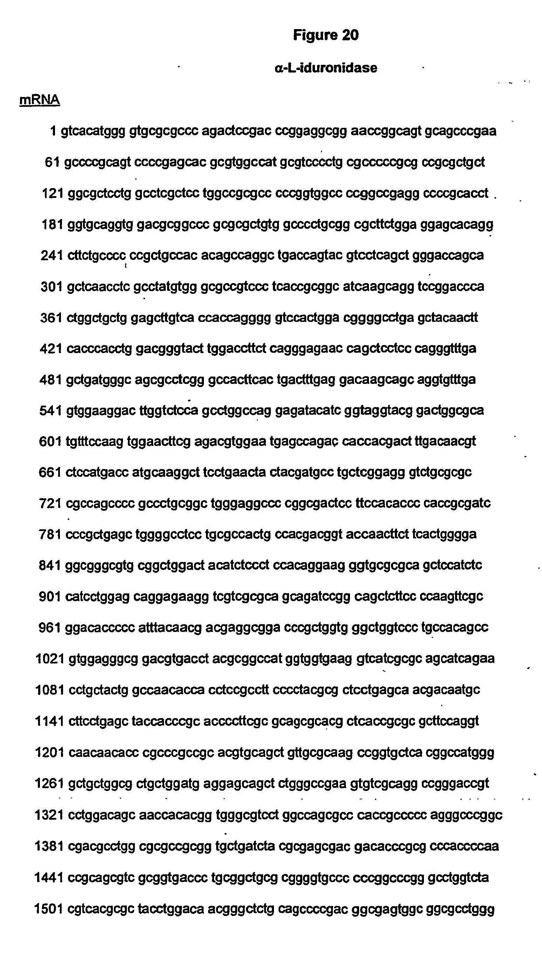

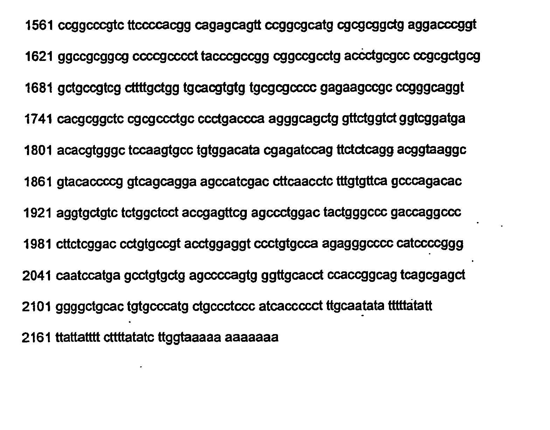

FIG 20 Depicts the mRNA sequence of α-L-iduronidase.

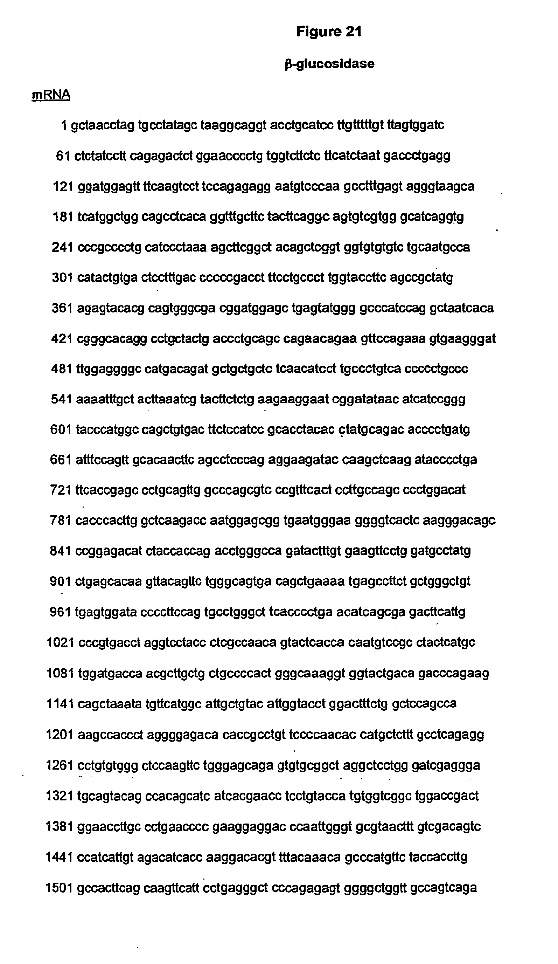

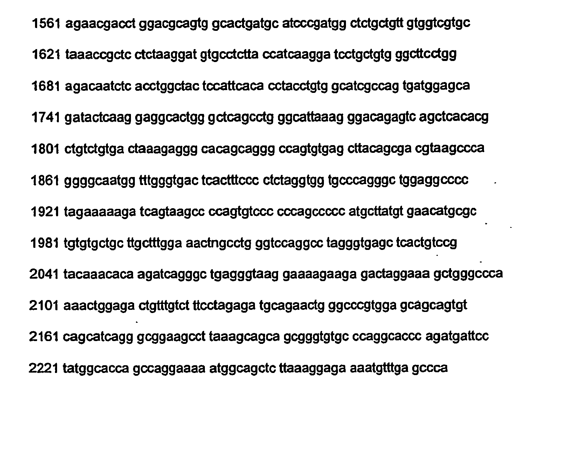

FIG 21 Depicts the mRNA sequence of β-glucosidase.

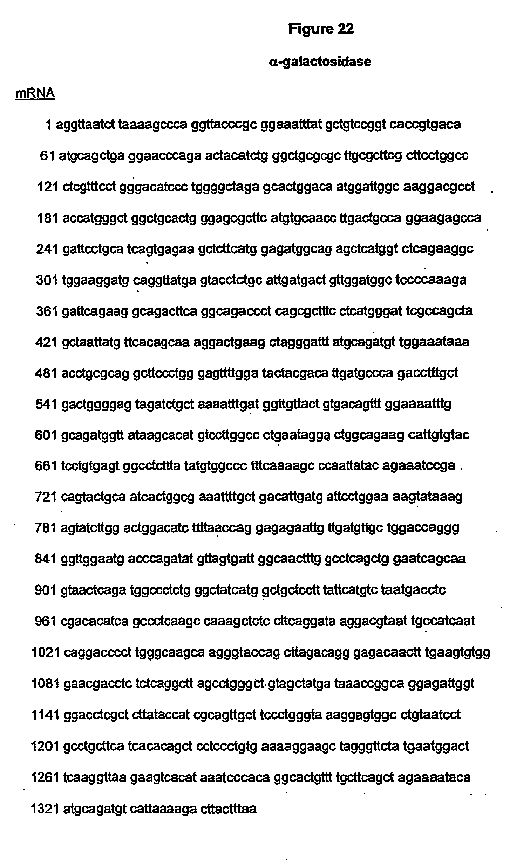

FIG 22 Depicts the mRNA sequence of α-galactosidase.

## DETAILED DESCRIPTION

Host immune responses directed against proteins associated with expression vectors have plagued the development of gene therapy techniques, wherein the cellular components of the response severely limit the expression of genes contained within the vector and the humoral component of the response complicates readministration of the same vector in immune competent animals. The success of the present invention stems from the surprising discovery that the expression of an immunomodulatory molecule such as CD8 on a target cell transfected with an expression vector suppresses both responding $CD4^+$ T cells and $CD8^+$ T cells, thereby effectively and specifically inhibiting both the humoral and the cellular components of the host immune response directed against vector-associated antigens.

Thus, the compositions and methods described herein are capable of dramatically improving *in vivo* and *ex vivo* gene therapy protocols by increasing the persistence of an expression vector in a host cell and thereby improving expression of a therapeutic transgene contained within the vector, as well as enabling the successful readministration of the same vector (e.g., a recombinant adenoviral vector of the same serotype) to the host cell. In one embodiment, expression vectors are provided comprising a nucleic acid encoding for an immunomodulatory molecule, most preferably both the extracellular domain and the transmembrane domain of the CD8 α-chain, as well as a nucleic acid sequence encoding for one or more therapeutic molecules of interest. In an alternative embodiment separate expression vectors are provided, one of which encodes for the CD8 polypeptide and one of which encodes for the desired therapeutic molecule(s), for co-administration to the host.

The present disclosure also provides a method for inhibiting an immune response to an expression vector, in particular a recombinant vector, such as an adenoviral vector, an adeno-associated viral vector, a herpes viral vector or a retroviral vector, comprising contacting a target cell with an expression vector encoding for an immunomodulatory molecule and one or more therapeutic molecules of interest, such as in the context of *in vivo* and *ex vivo* gene therapy. As described and exemplified herein, the antigen-specific inhibition of the host immune response achieved by the present invention enables a more persistent presence of the expression vector in the cell and concomitant improved expression of therapeutic transgene(s) contained within the vector, as well as successful readministration of the same vector for continuing gene therapy.

Accordingly, the present disclosure provides compositions and methods for gene therapy wherein the cellular and humoral immune responses against antigens associated with the gene therapy delivery vehicle are abolished or diminished. Generally, the present invention is directed to methods and compositions for reducing or diminishing both cellular and/or humoral immune responses against an expression vector, gene therapy vector, target cell or progeny of a target cell infected with a gene therapy vector.

*"In vivo* gene therapy" and *"in vitro* gene therapy" are intended to encompass all past, present and future variations and modifications of what is commonly known and referred to by those of ordinary skill in the art as "gene therapy", including *ex vivo* applications.

By "expression vector" is meant any vehicle for delivery of a nucleic acid to a target cell. Expression vectors can be generally divided into viral vectors and non-viral vectors. By viral vectors is meant, but not limited to adenoviral vectors, adeno-associated vectors, retroviral vectors, lentiviral vectors, and the like. By non-viral vectors is meant plasmid vectors, naked DNA, naked DNA coupled to different carriers, or associated with liposomes or other lipid preparation. Generally, expression vectors are recombinant, although in some embodiments, for example when liposomes or cell ablation, e.g. biolistic techniques, are used, they are not. Preferred recombinant vectors for use herein are plasmid vectors as well as viral vectors selected from the group consisting of an adenoviral vector, an adeno-associated viral vector, a herpes viral vector and a retroviral vector. In some embodiments utilizing recombinant viral vectors, and in particular adenoviral vectors, the immunogenicity of the capsid, e.g., the hexon protein of an adenoviral capsid, may be reduced in accordance with methods known in the art, although such modifications are no longer a necessity in view of the improvements detailed herein.

By "gene therapy delivery vehicle" is meant a composition including an expression vector as described above, including but not limited to viral vectors and non-viral vectors.

By "inhibiting" is meant the direct or indirect, partial or complete, inhibition and/or reduction of an innate or acquired immune response, whether cellular (e.g., leukocyte recruitment) or humoral, to vector-associated antigens and/or to target cell-specific antigens. Vector-associated antigens include, e.g., antigens derived from the nucleic acid carrier or envelope (e.g. viral coat proteins and the like) as well as antigens derived from vector genes (e.g. bacterial or viral nucleic acids and proteins) and/or any therapeutic transgenes (e.g. mammalian nucleic acids and/or proteins) included in the vector.

By "specific immune inhibition" or "antigen-specific immune inhibition" is meant the inhibition of immune responses directed against antigens such as vector-associated antigens, as opposed to general immune inhibition which is not antigen-specific. Thus, by way of example, the absence of a host cellular and/or humoral immune response to vector-associated antigens, combined with evidence of *in vivo* immune competence to other foreign antigens, would demonstrate specific immune inhibition of vector-associated antigens.

By "immune response" is preferably meant an acquired immune response, such as a cellular or humoral immune response.

By "contacting" is meant administering the gene therapy expression vector to the cell in such a manner and in such an amount as to effect physical contact between the vector and cell. If the vector is a recombinant viral particle, desirably, attachment to and infection of the cell by the viral vector is effected by such physical contact. If the viral vector is other than a recombinant viral particle, such as a nonencapsulated viral nucleic acid or other nucleic acid, desirably, infection of the cell by the nucleic acid is effected.

Such "contacting" can be done by any means known to those skilled in the art, and described herein, by which the apparent touching or mutual tangency of the vector with the target cell can be effected. Optionally, the vector, such as an adenoviral vector, can be further complexed with a bispecific or multispecific molecule (e.g., an antibody or fragment thereof), in which case "contacting" involves the apparent touching or mutual tangency of the complex of the vector and the bispecific or multispecific molecule with the target cell. For example, the vector and the bispecific (multispecific) molecule can be covalently joined, *e.g.*, by chemical means known to those skilled in the art, or other means. Preferably, the vector and the bispecific (multispecific) molecule can be linked by means of noncovalent interactions (e.g., ionic bonds, hydrogen bonds, Van der Waals forces, and/or nonpolar interactions). Although the vector and the bispecific (multispecific) molecule can be brought into contact by mixing in a small volume of the same solution, the target cell and the complex need not necessarily be brought into contact in a small volume, as, for instance, in cases where the complex is administered to a host (*e.g.*, a human), and the complex travels by the bloodstream to the target cell to which it binds selectively and into which it enters. The contacting of the vector with a bispecific (multispecific) molecule preferably is done before the target cell is contacted with the complex of the vector and the bispecific (multispecific) molecule.

By "transgene" is meant a gene, which can be expressed in a cell contacted with an expression vector comprising the transgene and the expression of which is desirably prophylactically or therapeutically beneficial to the cell or the tissue, organ, organ system, organism or cell culture of which the cell is a part. Thus, a transgene can be a therapeutic gene, e.g. therapeutic gene of interest. A therapeutic gene can be one that exerts its effect at the level of RNA or protein. For instance, a protein encoded by a therapeutic gene can be employed in the treatment of an inherited disease, *e.g.*, the use of a cDNA encoding the cystic fibrosis transmembrane conductance regulator in the treatment of cystic fibrosis.

Moreover, the therapeutic gene can exert its effect at the level of RNA, for instance, by encoding an antisense message or ribozyme, an siRNA as is known in the art, an alternative RNA splice acceptor or donor, a protein that affects splicing or 3' processing (e.g., polyadenylation), or a protein that affects the level of expression of another gene within the cell (i.e., where gene expression is broadly considered to include all steps from initiation of transcription through production of a processed protein), perhaps, among other things, by mediating an altered rate of mRNA accumulation, an alteration of mRNA transport, and/or a change in post-transcriptional regulation.

In accordance with preferred aspects of the present invention, the expression vector optionally comprises one or more transgenes encoding therapeutic molecules of interest along with the CD8 polypeptide described herein. Diseases that may be treated by the present invention include, but are not limited to, prevalent genetic diseases such as Phenylketonuria (phenylalanine-L-monooxygenase), cystic fibrosis (cystic fibrosis conductance regulator), ornithine caramyltransferase deficiency (OTC), hemophilias (Factor XI-deficiency, Factor VIII-deficiency), Tay-Sachs (N-acetyl-hexosaminidase A) and other lipid storage diseases, etc. In addition, the gene encoding erythropoietin (EPO) can used. EPO is a glycoprotein hormone produced in fetal liver and adult kidney which acts on progenitor cells in the bone marrow and other hematopoietic tissue to stimulate the formation of red blood cells. Genes encoding human and other mammalian EPO have been cloned, sequenced and expressed, and show a high degree of sequence homology in the coding region across species. Wen et al. (1993) Blood 82:1507-1516. The sequence of the gene encoding native human EPO, as well as methods of obtaining the same, are described in, e.g., U.S. Pat. Nos. 4,954,437 and 4,703,008, . Gene therapy methods using EPO are disclosed in U.S. Patent No. 6,610,290,

Alternatively, a nucleotide sequence encoding the lysosomal enzyme acid alpha.-glucosidase (GAA) can be used. GAA functions to cleave .alpha.-1,4 and .alpha.-1,6 linkages of lysosomal glycogen to release monosaccharides. The sequence of the gene encoding human GAA, as well as methods of obtaining the same, have been previously described (GenBank Accession Numbers: M34424 and Y00839; Martiniuk et al. (1990) DNA Cell Biol. 9:85-94; Martiniuk et al. (1986) Proc. Natl. Acad. Sci. USA 83:9641-9644; Hoefsloot et al. (1988) Eur. Mol. Biol. Organ. 7:1697-1704),

Preferred diseases that may be treated by the methods and compositions disclosed herein are set forth in Table 1 below.

TABLE 1

| **Gene Therapy Targets** | | |
|---|---|---|
| **Disease Name** | **Defect** | **Accession Number/mRNA** |
| Sickle Cell Anemia | hemoglobin-β | NM_000518 |
| x-linked Dyserythropoietic Anemia | GATA-binding protein | NM_002049 |
| Sideroblastic Anemia | δ-aminoevulinate synthase | NM_000032 |
| Chronic Hemolytic Anemia (Favism) | glucose-6-phosphate-dehydrogenase | NM_000402 |
| Hemophilia A | Coagulation Factor VIII | NM_000132 |
| Hemophilia B | Coagulation Factor XI | NM_000133 |
| Cystic Fibrosis | cystic fibrosis transmembrane conductance regulator | NM_000492 |
| OTC-Deficiency | ornithine carbamoyl transferas | NM_000531 |
| Hurler Syndrome | α-L-iduronidase | NM_000203 |
| Hunter Syndrome | Iduronate-2-sulfatase | NM_000202 |
| Gaucher Disease | β-glucosidase | NM_000157 |
| Fabry Disease | α-galactosidase | NM_000169 |
| Krabbe Disease | galactosylceramidase | NM_000153 |
| Pompe Disease | acid α-glucosidase | NM_000152 |
| Tay-Sachs Disease | hexamidase A | NM_000520 |
| Phenylketonuria | phenylalanine hydroxylase | NM_000277 |
| Alport Syndrome | collagen type IV, α5 | NM_000495 |
| Bloom Syndrome | Bloom Sundrome Gene Product | NM_000057 |
| Familial Hypercholestrolemia | low density lipoprotein receptor | NM_000527 |

If the immunomodulatory CD8 molecule is encoded by a gene contained in a vector that is separate from the vector comprising and expressing the therapeutic transgene, the vector comprising the CD8 molecule can be brought into contact with the cell prior to, simultaneously with, or subsequent to contact of the cell with the vector comprising and expressing the gene, as long as similar or identical types of vectors are used and the timing of the contact effects is sufficient to inhibit an immune response to the vectors brought into contact with the cell.

A "target cell" can be present as a single entity, or can be part of a larger collection of cells. Such a "larger collection of cells" may comprise, for instance, a cell culture (either mixed or pure), a tissue (e.g., epithelial or other tissue), an organ (e.g., heart, lung, liver, gallbladder, urinary bladder, eye or other organ), an organ system (e.g., circulatory system, respiratory system, gastrointestinal system, urinary system, nervous system, integumentary system or other organ system), or an organism (e.g., a bird, mammal, particularly a human, or the like). Preferably, the organs/tissues/cells being targeted are of the circulatory system (e.g., including, but not limited to heart, blood vessels, and blood), respiratory system (e.g., nose, pharynx, larynx, trachea, bronchi, bronchioles, lungs, and the like), gastrointestinal system (e.g., including mouth, pharynx, esophagus, stomach, intestines, salivary glands, pancreas, liver, gallbladder, and others), urinary system (e.g., such as kidneys, ureters, urinary bladder, urethra, and the like), nervous system (e.g., including, but not limited to, brain and spinal cord, and special sense organs, such as the eye) and integumentary system (e.g., skin). Even more preferably, the cells are selected from the group consisting of heart, blood vessel, lung, liver, gallbladder, urinary bladder, eye cells and stem cells. Methods of culturing and using stem cells are disclosed in more detail in U.S. Patent Nos. 5,672,346, 6,143,292 and 6,534,052.

In some embodiments, a target cell with which an expression vector such as a viral vector or plasmid is contacted differs from another cell in that the contacted target cell comprises a particular cell-surface binding site that can be targeted by the expression vector. By "particular cell-surface binding site" is meant any site (i.e., molecule or combination of molecules) present on the surface of a cell with which the vector, e.g., adenoviral vector, can interact in order to attach to the cell and, thereby, enter the cell. A particular cell-surface binding site, therefore, encompasses a cell-surface receptor and, preferably, is a protein (including a modified protein), a carbohydrate, a glycoprotein, a proteoglycan, a lipid, a mucin molecule or mucoprotein, and the like. Examples of potential cell-surface binding sites include, but are not limited to: heparin and chondroitin sulfate moieties found on glycosaminoglycans; sialic acid moieties found on mucins, glycoproteins, and gangliosides; major histocompatability complex I (MHC I) glycoproteins; common carbohydrate molecules found in membrane glycoproteins, including mannose, N-acetyl-galactosamine, N-acetyl-glucosamine, fucose, and galactose; glycoproteins, such as ICAM-1, VCAM; E-selectin, P-selectin, L-selectin, and integrin molecules; and tumor-specific antigens present on cancerous cells, such as, for instance, MUC-1 tumor-specific epitopes. However, targeting an expression vector such as an adenovirus to a cell is not limited to any specific mechanism of cellular interaction (i.e., interaction with a given cell-surface binding site).

As used herein and further defined below, "polynucleotide" or "nucleic acid" may refer to either DNA or RNA, or molecules which contain both deoxy- and ribonucleotides. The nucleic acids include genomic DNA, cDNA and oligonucleotides including sense and anti-sense nucleic acids. Such nucleic acids may also contain modifications in the ribose-phosphate backbone to increase stability and half life of such molecules in physiological environments.

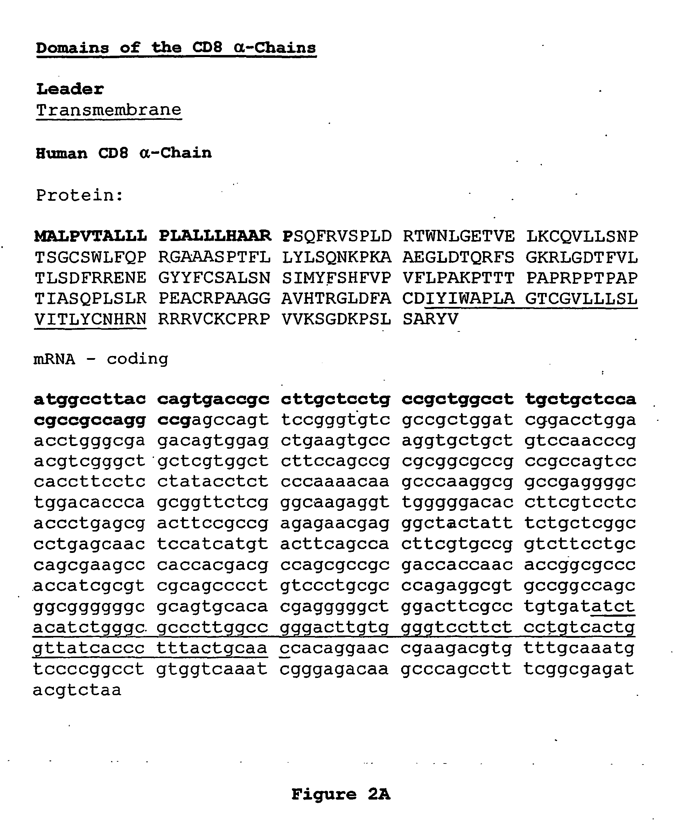

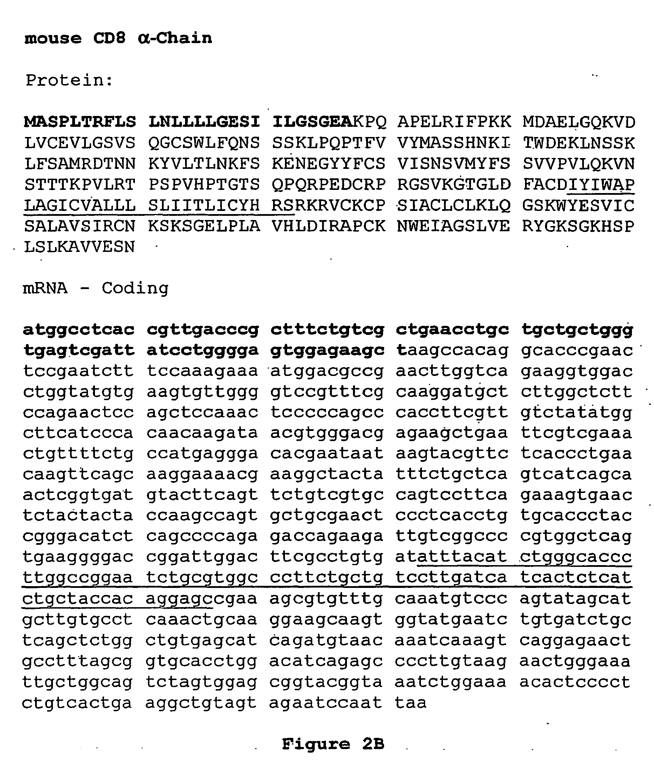

The nucleic acid may be double stranded, single stranded, or contain portions of both double stranded or single stranded sequence. As will be appreciated by those in the art, the depiction of a single strand ("Watson") also defines the sequence of the other strand ("Crick"); thus the sequences depicted in Figures 2, 4 and 6 also include the complement of the sequence. By the term "recombinant nucleic acid" herein is meant nucleic acid, originally formed *in vitro,* in general, by the manipulation of nucleic acid by endonucleases, in a form not normally found in nature. Thus an isolated nucleic acid, in a linear form, or an expression vector formed *in vitro* by ligating DNA molecules that are not normally joined, are both considered recombinant for the purposes of this invention. It is understood that once a recombinant nucleic acid is made and reintroduced into a host cell or organism, it may replicate non-recombinantly, i.e. using the *in vivo* cellular machinery of the host cell rather than *in vitro* or extrachromosomal manipulations; however, such nucleic acids, once produced recombinantly, although subsequently replicated non-recombinantly, are still considered recombinant for the purposes of the invention.

The terms "polypeptide" and "protein" may be used interchangeably throughout this application and mean at least two covalently attached amino acids, which includes proteins, polypeptides, oligopeptides and peptides. The protein may be made up of naturally occurring amino acids and peptide bonds, or synthetic peptidomimetic structures. Thus "amino acid", or "peptide residue", as used herein means both naturally occurring and synthetic amino acids. For example, homo-phenylalanine, citrulline and noreleucine are considered amino acids for the purposes of the invention. "Amino acid" also includes imino acid residues such as proline and hydroxyproline. The side chains may be in either the (R) or the (S) configuration. In the preferred embodiment, the amino acids are in the (S) or L-configuration. If non-naturally occurring side chains are used, non-amino acid substituents may be used, for example to prevent or retard *in vivo* degradation. Alterations of native amino acid sequences to produce variant proteins and peptides for targeting or expression as a transgene, for example, can be done by a variety of means known to those skilled in the art. A variant peptide is a peptide that is substantially homologous to a given peptide, but which has an amino acid sequence that differs from that peptide. The degree of homology (i.e., percent identity) can be determined, for instance, by comparing sequence information using a computer program optimized for such comparison (e.g., using the GAP computer program, version 6.0 or a higher version, described by Devereux et al. (Nucleic Acids Res., 12, 387 (1984)), and freely available from the University of Wisconsin Genetics Computer Group (UWGCG)). The activity of the variant proteins and/or peptides can be assessed using other methods known to those skilled in the art.

In terms of amino acid residues that are not identical between the variant protein (peptide) and the reference protein (peptide), the variant proteins (peptides) preferably comprise conservative amino acid substitutions, i.e., such that a given amino acid is substituted by another amino acid of similar size, charge density, hydrophobicity/hydrophilicity, and/or configuration (e.g., Val for Phe). The variant site-specific mutations can be introduced by ligating into an expression vector a synthesized oligonucleotide comprising the modified site. Alternately, oligonucleotide-directed site-specific mutagenesis procedures can be used, such as those disclosed in Walder et al., Gene, 42:133 (1986); Bauer et al., Gene, 37:73 (1985); Craik, Biotechniques, January 1995, pp. 12-19; and U.S. Patent Nos. 4,518,584 and 4,737,462.

***Immunomodulatory Molecules***

In the context of the present specification, an "immunomodulatory molecule" is an polypeptide molecule that modulates, i.e. increases or decreases a cellular and/or humoral host immune response directed to a target cell in an antigen-specific fashion, and preferably is one that decreases the host immune response. Generally, in accordance with the teachings of the present invention the immunomodulatory molecule(s) will be associated with the target cell surface membrane, e.g., inserted into the cell surface membrane or covalently or non-covalently bound thereto, after expression from the vectors described herein.

In preferred embodiments, the immunomodulatory molecule comprises all or a functional portion of a CD8 protein, and even more preferably all or a functional portion of the CD8 $\alpha$ chain. For human CD8 coding sequences, see Leahy, Faseb J. 9:17-25 (1995); Leahy et al., Cell 68:1145-62 (1992); Nakayama et al., Immunogenetics 30:393-7 (1989). By "functional portion" with respect to CD8 proteins and polypeptides is meant that portion of the CD8 $\alpha$-chain retaining veto activity as described herein, more particularly that portion retaining the HLA-binding activity of the CD8 $\alpha$-chain, and specifically the Ig-like domain in the extracellular region of the CD8 $\alpha$-chain. Exemplary variant CD8 polypeptides are described in Gao and Jakobsen, Immunology Today 21:630-636 (2000), . In some embodiments, the full length CD8 $\alpha$-chain is used. However, in some embodiments the cytoplasmic domain is deleted. Preferably the transmembrane domain and extracellular domain are retained.

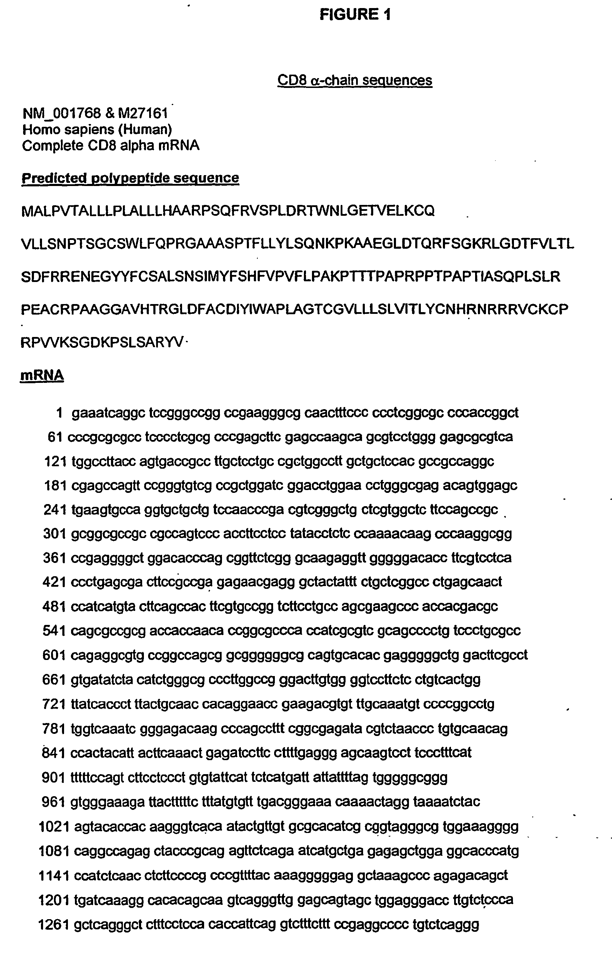

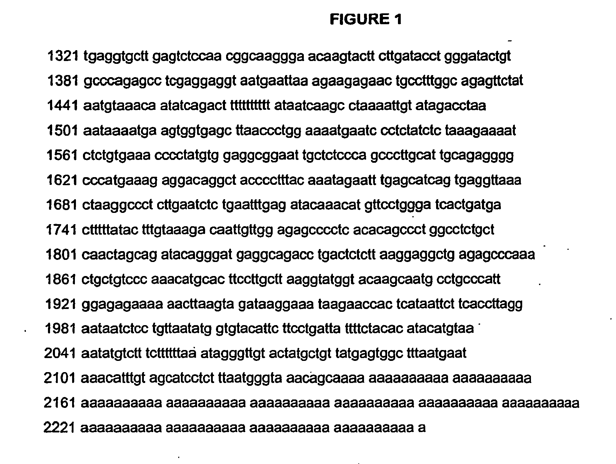

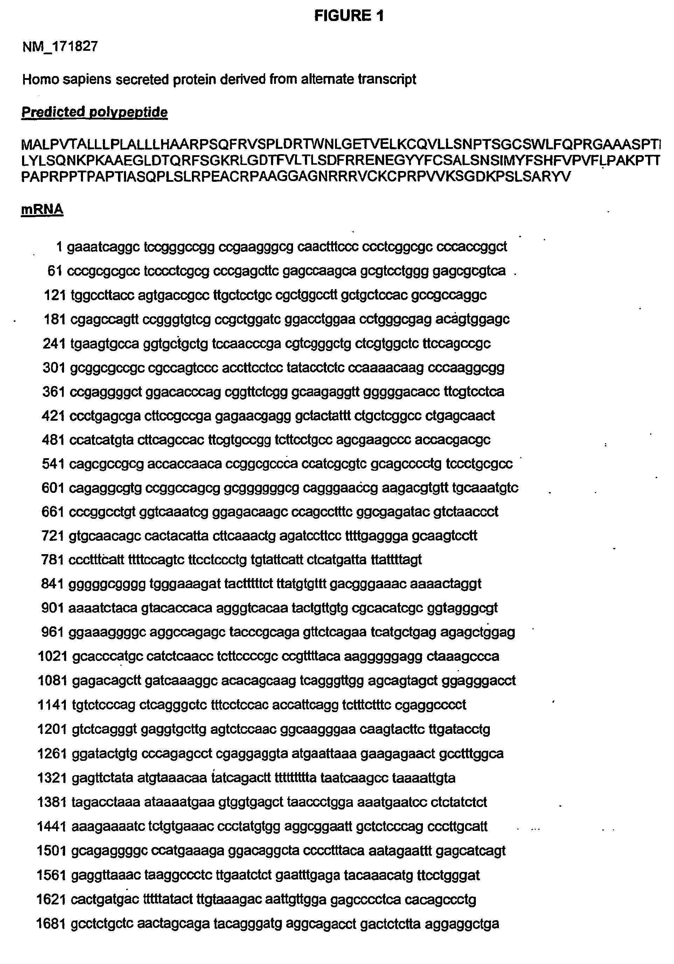

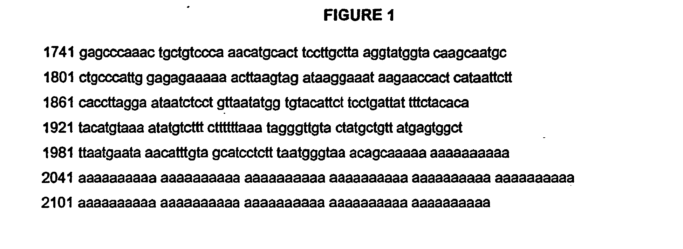

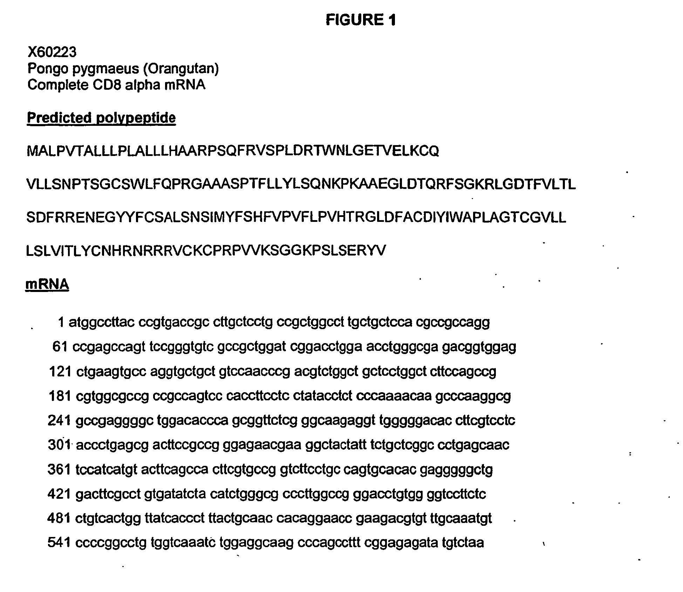

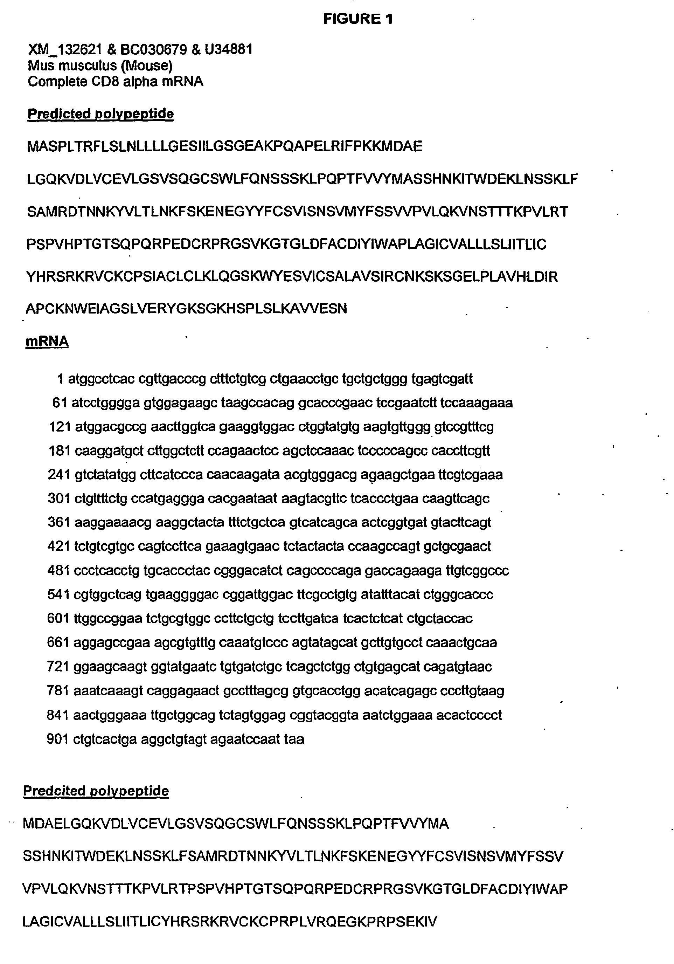

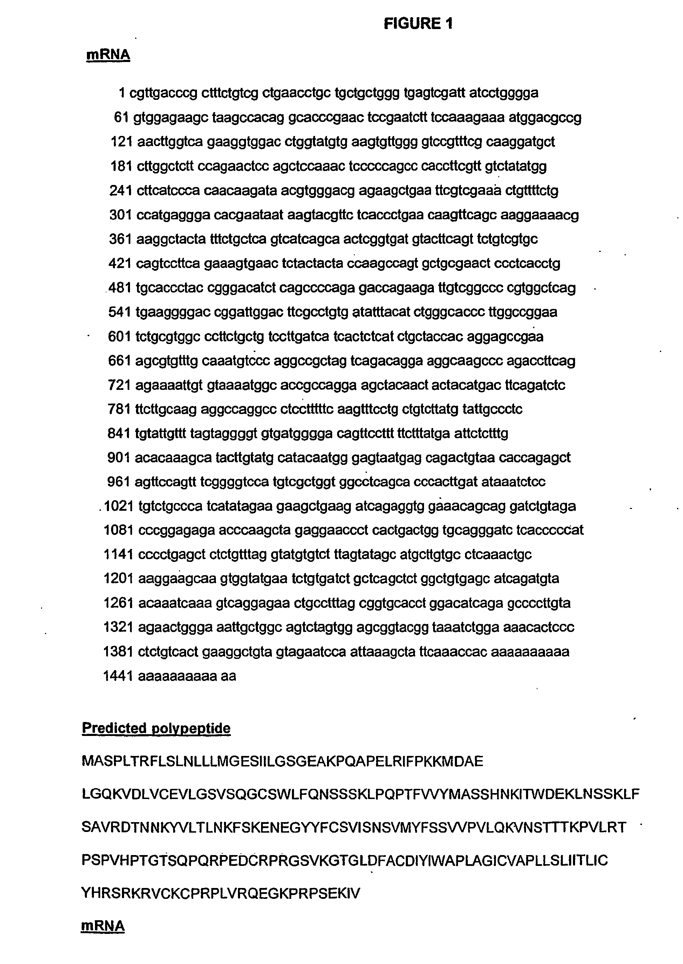

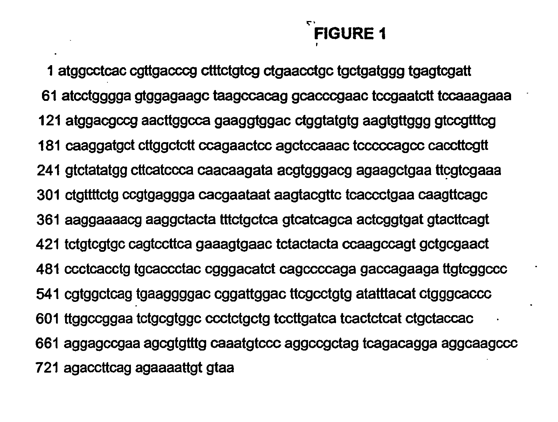

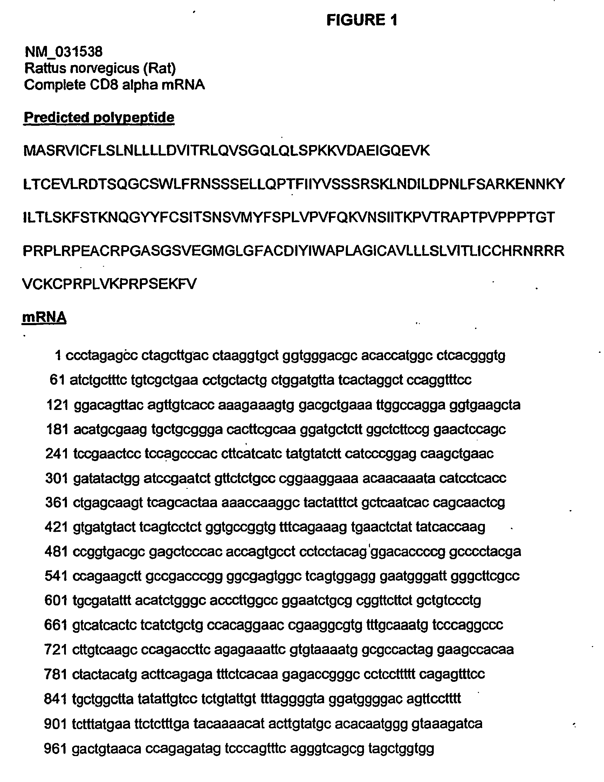

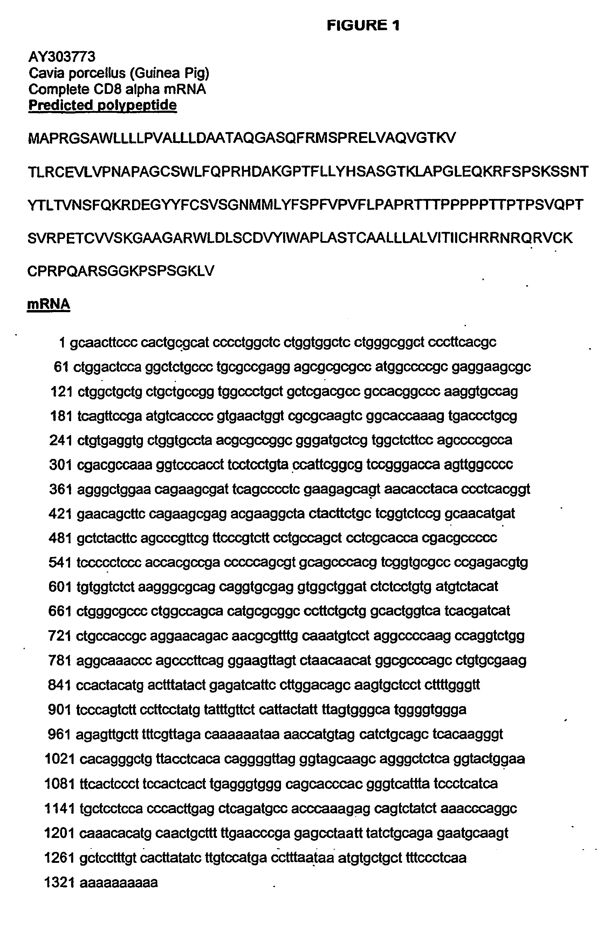

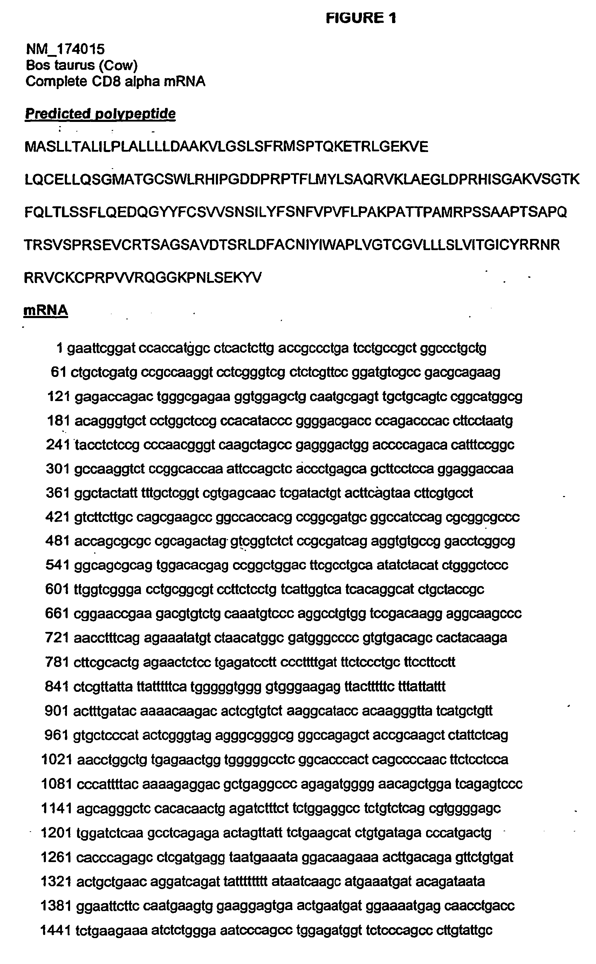

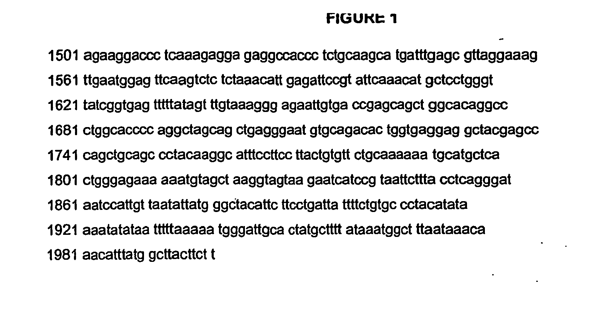

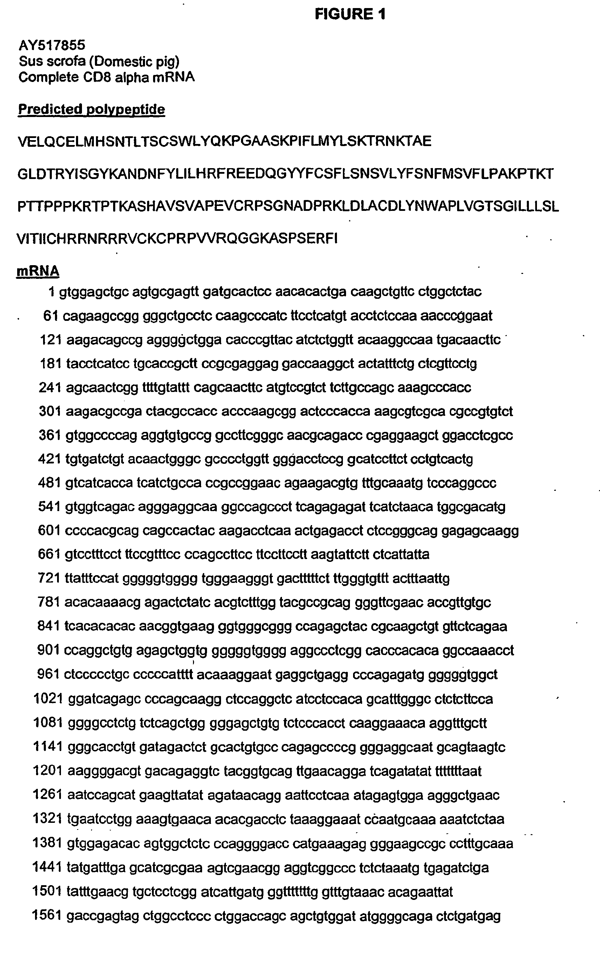

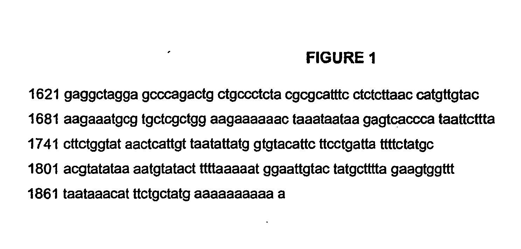

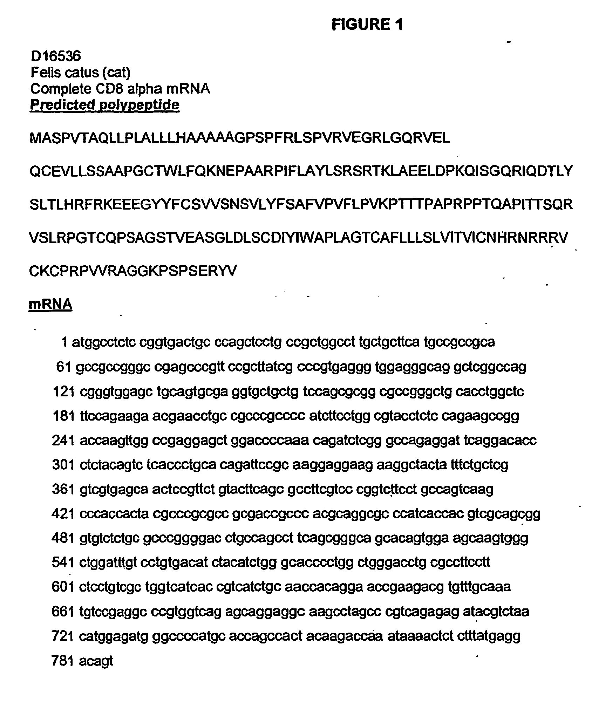

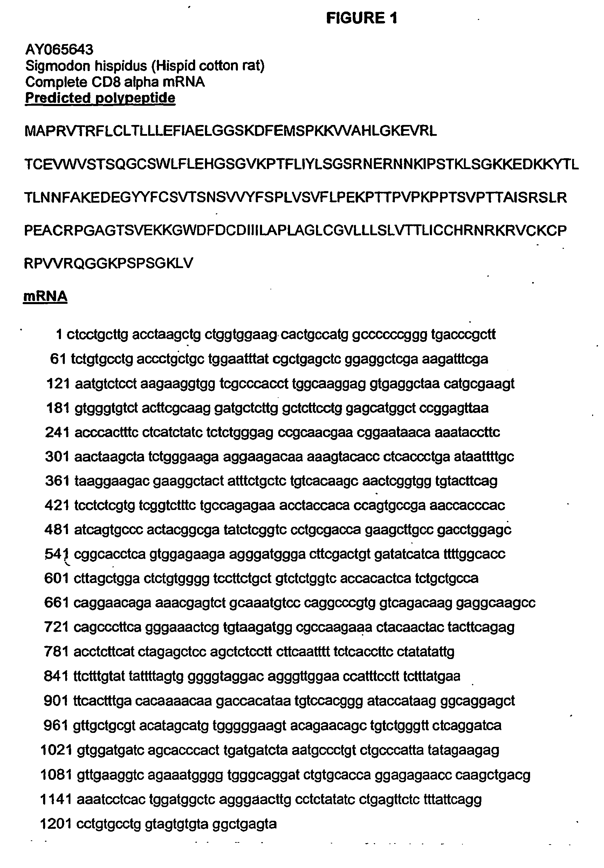

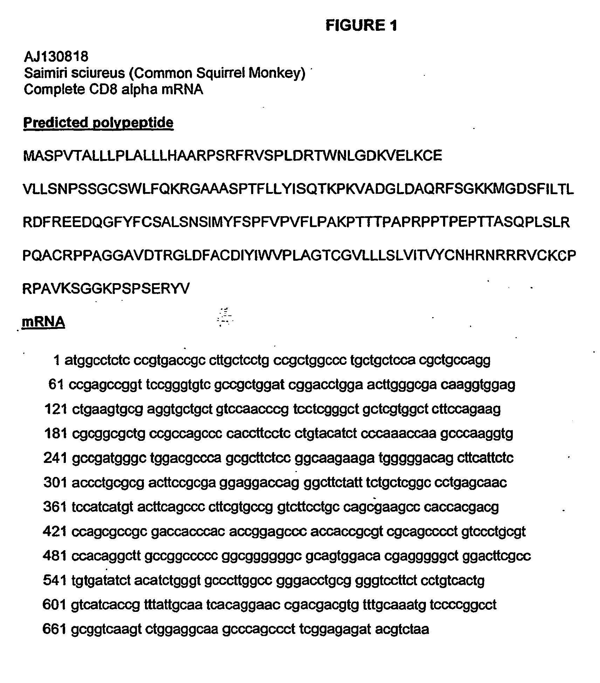

As will be appreciated by those of skill in the art the transmembrane domain of the CD8 $\alpha$-chain can be exchanged with transmembrane domains of other molecules, if necessary, to modify association of the extracellular domain with the target cell surface. In this embodiment the nucleic acid encoding the extracellular domain of CD8 $\alpha$-chain is operably linked to a nucleic acid encoding a transmembrane domain. Transmembrane domains of any transmembrane protein can be used in the invention. Alternatively a transmembrane not known to be found in transmembrane proteins. In this embodiment the "synthetic transmembrane domain" contains from around 20 to 25 hydrophobic amino acids followed by at least one and preferably two charged amino acids. In some embodiments the CD8 extracellular domain is linked to the target cell membrane by conventional techniques in the art. Preferred CD8 $\alpha$-chain sequences are set forth in Figure 1 and include the full length sequences of either the amino acid sequence or nucleic acid sequence encoding a full length CD8 $\alpha$-chain from species including human, mouse, rat, orangutan, spider monkey, guinea pig, cow, Hispid cotton rat, domestic pig and cat.

In a preferred embodiment the CD8 $\alpha$-chain is not a fusion protein, but rather is a truncation protein wherein the intracellular domain is deleted. As depicted in Figure 2, the human CD8 $\alpha$-chain gene expresses a protein of 235 amino acids. The protein can be considered to be divided into the following domains (starting at the amino terminal and ending at the carboxy terminal of the polypeptide): a signal peptide (amino acids 1 to 21); immunoglobulin (Ig)-like domain (approximately amino acids 22-136); membrane proximal stalk region (amino acids 137-181); transmembrane domain (amino acids 183-210) and cytoplasmic domain (amino acids 211-235). The nucleotides of the coding sequence that encode these different domains include 1-63 encoding the signal peptide, 64-546 encoding the extracellular domain, about 547-621 encoding the intracellular domain and about 622-708 encoding the intracellular domain. Likewise, the mouse sequences can be divided into domains as follows. The polypeptide can be divided into a signal sequence including amino acids 1-27, an extracellular domain including about amino acids 28 to 194, a transmembrane domain including about amino acids 195-222 and an intracellular domain including about amino acids 223-310. Similarly, the nucleotides of the coding sequence encoding these domain include nucleic acid 1-81 encoding the signal peptide, about 82-582 encoding the extracellular domain, about 583-666 encoding the transmembrane domain and about 667-923 encoding the extracellular domain.

In some embodiments nucleic acid encoding the full length protein is included in the gene delivery vehicle. In other embodiments, nucleic acids encoding the intracellular domain are not included in the polynucleotide in the gene delivery vehicle resulting in a membrane anchored protein lacking the intracellular domain. Corresponding domains also can be identified in other species, including in preferred embodiments the mouse.

One skilled in the art will also appreciate that immunomodulatory molecules having substantial homology to the afore-mentioned polypeptides may find advantageous use in the invention. Accordingly, for example, also encompassed by "CD8 polypeptides" are homologous polypeptides having at least about 80% sequence identity, usually at least about 85% sequence identity, preferably at least about 90% sequence identity, more preferably at least about 95% sequence identity and most preferably at least about 98% sequence identity with the polypeptide encoded by nucleotides shown in Figure 2.

By "nucleic acid molecules encoding CD8", and grammatical equivalents thereof is meant the nucleotide sequence of human CD8 as shown in Figure 2 as well as nucleotides sequences having at least about 80% sequence identity, usually at least about 85% sequence identity, preferably at least about 90% sequence identity, more preferably at least about 95% sequence identity and most preferably at least about 98% sequence identity with nucleotides shown in Figure 2 and which encode a polypeptide having the sequence shown in Figure 2, and as set forth in Figure 1.

As noted previously, a number of different programs can be used to identify whether a protein or nucleic acid has sequence identity or similarity to a known sequence. Sequence identity and/or similarity is determined using standard techniques known in the art, including, but not limited to, the local sequence identity algorithm of Smith & Waterman, Adv. Appl. Math. 2:482 (1981), by the sequence identity alignment algorithm of Needleman & Wunsch, J. Mol. Biol. 48: 443 (1970), by the search for similarity method of Pearson & Lipman, PNAS USA 85:2444 (1988), by computerized implementations of these algorithms (GAP, BESTFIT, FASTA, and TFASTA in the Wisconsin Genetics Software Package, Genetics Computer Group, 575 Science Drive, Madison, WI), the Best Fit sequence program described by Devereux et al., Nucl. Acid Res. 12:387-395 (1984), preferably using the default settings, or by inspection. Preferably, percent identity is calculated by FastDB based upon the following parameters: mismatch penalty of 1; gap penalty of 1; gap size penalty of 0.33; and joining penalty of 30, "Current Methods in Sequence Comparison and Analysis," Macromolecule Sequencing and Synthesis, Selected Methods and Applications, pp 127-149 (1988), Alan R. Liss, Inc.

An example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, J. Mol. Evol. 35:351-360 (1987); the method is similar to that described by Higgins & Sharp CABIOS 5:151-153 (1989). Useful PILEUP parameters including a default gap weight of 3.00, a default gap length weight of 0.10, and weighted end gaps.

Another example of a useful algorithm is the BLAST algorithm, described in Altschul et al., J. Mol. Biol. 215, 403-410, (1990) and Karlin et al., PNAS USA 90:5873-5787 (1993). A particularly useful BLAST program is the WU-BLAST-2 program which was obtained from Altschul et al., Methods in Enzymology, 266: 460-480 (1996); http://blast.wustl/edu/blast/ README.html]. WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched; however, the values may be adjusted to increase sensitivity.

An additional useful algorithm is gapped BLAST as reported by Altschul et al. Nucleic Acids Res. 25:3389-3402. Gapped BLAST uses BLOSUM-62 substitution scores; threshold T parameter set to 9; the two-hit method to trigger ungapped extensions; charges gap lengths of k a cost of 10+k; Xu set to 16, and Xg set to 40 for database search stage and to 67 for the output stage of the algorithms. Gapped alignments are triggered by a score corresponding to -22 bits.

A % amino acid or nucleic acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

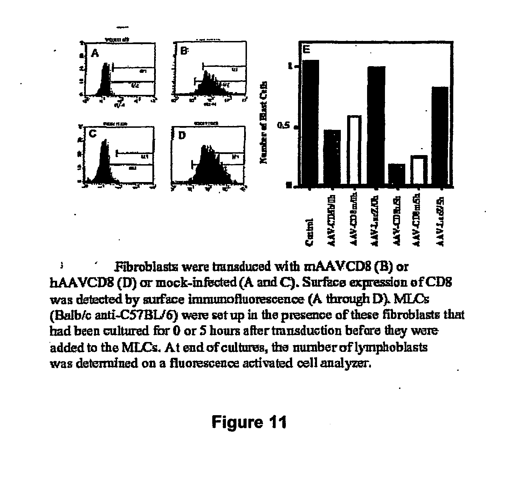

The alignment may include the introduction of gaps in the sequences to be aligned. In addition, for sequences which contain either more or fewer amino acids than the amino acid sequence of the polypeptide encoded by nucleotides shown in Figure 11, it is understood that in one embodiment, the percentage of sequence identity will be determined based on the number of identical amino acids in relation to the total number of amino acids. Thus, for example, sequence identity of sequences shorter than that of the polypeptide encoded by nucleotides in Figure 11, as discussed below, will be determined using the number of amino acids in the shorter sequence, in one embodiment. In percent identity calculations relative weight is not assigned to various manifestations of sequence variation, such as, insertions, deletions, substitutions, etc.

In one embodiment, only identities are scored positively (+1) and all forms of sequence variation including gaps are assigned a value of "0", which obviates the need for a weighted scale or parameters as described below for sequence similarity calculations. Percent sequence identity can be calculated, for example, by dividing the number of matching identical residues by the total number of residues of the "shorter" sequence in the aligned region and multiplying by 100. The "longer" sequence is the one having the most actual residues in the aligned region.

CD8 having less than 100% sequence identity with the polypeptide encoded by nucleotides in Figure 2 will generally be produced from native CD8 nucleotide sequences from species other than human and variants of native CD8 nucleotide sequences from human or non-human sources. In this regard, it is noted that many techniques are well known in the art and may be routinely employed to produce nucleotide sequence variants of native CD8 sequences and assaying the polypeptide products of those variants for the presence of at least one activity that is normally associated with a native CD8 polypeptide. In a preferred embodiment the CD8 $\alpha$-chain is from human but as shown in Figure 1, CD8 $\alpha$-chain from rat, mouse, and primates are known and find use in the invention.

Polypeptides having CD8 activity may be shorter or longer than the polypeptide encoded by nucleotides depicted in Figure 2. Thus, in a preferred embodiment, included within the definition of CD8 polypeptide are portions or fragments of the polypeptide encoded by nucleotides in Figure 2. In one embodiment herein, fragments of the polypeptide encoded by nucleotides in Figure 2 are considered CD8 polypeptides if a) they have at least the indicated sequence identity; and b) preferably have a biological activity of naturally occurring CD8, as described above.

In addition, as is more fully outlined below, CD8 $\alpha$-chain can be made longer than the polypeptide encoded by nucleotides in Figure 2; for example, by the addition of other fusion sequences, or the elucidation of additional coding and non-coding sequences.

The CD8 polypeptides are preferably recombinant. A "recombinant polypeptide" is a polypeptide made using recombinant techniques, i.e. through the expression of a recombinant nucleic acid as described below. In a preferred embodiment, CD8 of the invention is made through the expression of nucleic acid sequence shown in Figure 2, or fragment thereof. A recombinant polypeptide is distinguished from naturally occurring protein by at least one or more characteristics. For example, the polypeptide may be isolated or purified away from some or all of the proteins and compounds with which it is normally associated in its wild type host, and thus may be substantially pure. For example, an isolated polypeptide is unaccompanied by at least some of the material with which it is normally associated in its natural state, preferably constituting at least about 0.5%, more preferably at least about 5% by weight of the total protein in a given sample. A substantially pure polypeptide comprises at least about 75% by weight of the total polypeptide, with at least about 80% being preferred, and at least about 90% being particularly preferred. The definition includes the production of a CD8 polypeptide from one organism in a different organism or host cell.

Alternatively, the polypeptide may be made at a significantly higher concentration than is normally seen, through the use of a inducible promoter or high expression promoter, such that the polypeptide is made at increased concentration levels. Alternatively, the polypeptide may be in a form not normally found in nature, as in the addition of amino acid substitutions, insertions and deletions, as discussed below.

In one embodiment, the present invention provides nucleic acid CD8 variants. These variants fall into one or more of three classes: substitutional, insertional or deletional variants. These variants ordinarily are prepared by site specific mutagenesis of nucleotides in nucleotides of Figure 2, using cassette or PCR mutagenesis or other techniques well known in the art, to produce DNA encoding the variant, including the variant in a gene therapy vector and thereafter expressing the DNA. Amino acid sequence variants are characterized by the predetermined nature of the variation, a feature that sets them apart from naturally occurring allelic or interspecies variation of CD8 amino acid sequence. The variants typically exhibit the same qualitative biological activity as the naturally occurring analogue, although variants can also be selected which have modified characteristics as will be more fully outlined below.

While the site or region for introducing a sequence variation is predetermined, the mutation per se need not be predetermined. For example, in order to optimize the performance of a mutation at a given site, random mutagenesis may be conducted at the target codon or region and the expressed variants screened for the optimal desired activity. Techniques for making substitution mutations at predetermined sites in DNA having a known sequence are well known, for example, M13 primer mutagenesis and PCR mutagenesis. Another example of a technique for making variants is the method of gene shuffling, whereby fragments of similar variants of a nucleotide sequence are allowed to recombine to produce new variant combinations. Examples of such techniques are found in U.S. Patent Nos. 5,605,703; 5,811,238; 5,873,458; 5,830,696; 5,939,250; 5,763,239; 5,965,408; and 5,945,325 .

Amino acid substitutions are typically of single residues; insertions usually will be on the order of from about 1 to 20 amino acids, although considerably larger insertions may be tolerated. Deletions range from about 1 to about 20 residues, although in some cases deletions may be much larger and may include the cytoplasmic domain or fragments thereof.

Substitutions, deletions, insertions or any combination thereof may be used to arrive at a final derivative. Generally these changes are done on a few amino acids to minimize the alteration of the molecule. However, larger changes may be tolerated in certain circumstances. When small alterations in the characteristics of the CD8 are desired, substitutions are generally made in accordance with the following chart:

CHART 1

| Original Residue | Exemplary Substitutions |
|---|---|
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |

(continued)

| Original Residue | Exemplary Substitutions |
|---|---|
| Val | Ile, Leu |

Substantial changes in function or immunological identity are made by selecting substitutions that are less conservative than those shown in Chart 1. For example, substitutions may be made which more significantly affect: the structure of the polypeptide backbone in the area of the alteration, for example the alpha-helical or beta-sheet structure; the charge or hydrophobicity of the molecule at the target site; or the bulk of the side chain. The substitutions which in general are expected to produce the greatest changes in the polypeptide's properties are those in which (a) a hydrophilic residue, e.g. seryl or threonyl, is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g. lysyl, arginyl, or histidyl, is substituted for (or by) an electronegative residue, e.g. glutamyl or aspartyl; or (d) a residue having a bulky side chain, e.g. phenylalanine, is substituted for (or by) one not having a side chain, e.g. glycine.

The variants typically exhibit the same qualitative biological activity and will elicit the same immune response as the naturally-occurring analogue, although variants also are selected to modify the characteristics of the CD8 as needed. Alternatively, the variant may be designed such that the biological activity of the protein is altered.

One type of covalent modification of a polypeptide included within the scope of this invention comprises altering the native glycosylation pattern of the polypeptide. "Altering the native glycosylation pattern" is intended for purposes herein to mean deleting one or more carbohydrate moieties found in native sequence CD8 polypeptide, and/or adding one or more glycosylation sites that are not present in the native sequence polypeptide.

Addition of glycosylation sites to polypeptides may be accomplished by altering the amino acid sequence thereof. The alteration may be made, for example, by the addition of, or substitution by, one or more serine or threonine residues to the native sequence polypeptide (for O-linked glycosylation sites). The amino acid sequence may optionally be altered through changes at the DNA level, particularly by mutating the DNA encoding the polypeptide at preselected bases such that codons are generated that will translate into the desired amino acids.

Removal of carbohydrate moieties present on the polypeptide may be accomplished by mutational substitution of codons encoding for amino acid residues that serve as targets for glycosylation.

Once isolated from its natural source, e.g., contained within a plasmid or other vector or excised therefrom as a linear nucleic acid segment, the recombinant nucleic acid can be further-used as a probe to identify and isolate other nucleic acids. It can also be used as a "precursor" nucleic acid to make modified or variant nucleic acids and proteins. It also can be incorporated into a vector or other delivery vehicle for treating target cells as described herein.

*Gene Therapy Expression Vectors*

In the context of the present invention, any suitable gene therapy expression vector can be used. A "vector" is a vehicle for gene transfer as that term is understood by those of skill in the art. The vectors according to the invention include, but are not limited to, plasmids, phages, viruses, liposomes, and the like. An expression vector according to the invention preferably comprises additional sequences and mutations. In particular, an expression vector according to the invention comprises a nucleic acid comprising a transgene encoding an immunomodulatory molecule, particularly CD8 $\alpha$-chain, as defined herein, and optionally further comprises at least one additional transgene encoding for a therapeutic molecule of interest. The nucleic acid may comprise a wholly or partially synthetically made coding or other genetic sequence or a genomic or complementary DNA (cDNA) sequence, and can be provided in the form of either DNA or RNA.

A transgene and/or a gene encoding for an immunomodulatory and/or therapeutic molecule can be moved to or from a viral vector or into a baculovirus or a suitable prokaryotic or eukaryotic expression vector for expression of mRNA and production of protein, and for evaluation of other biochemical characteristics.

In terms of the production of vectors according to the invention (including recombinant adenoviral vectors and transfer vectors), such vectors can be constructed using standard molecular and genetic techniques, such as those known to those skilled in the art. Vectors comprising virions or viral particles (e.g., recombinant adenoviral vectors) can be produced using viral vectors in the appropriate cell lines. Similarly, particles comprising one or more chimeric coat proteins can be produced in standard cell lines, e.g., those currently used for adenoviral vectors. These resultant particles then can be targeted to specific cells, if desired.

Any appropriate expression vector (e.g., as described in Pouwels et al., Cloning Vectors: A Laboratory Manual (Elsevior, N.Y.: 1985)) and corresponding suitable host cell can be employed for production of a recombinant peptide or protein in a host cell. Expression hosts include, but are not limited to, bacterial species within the genera Escherichia, Bacillus, Pseudomonas, Salmonella, mammalian or insect host cell systems, including baculoviral systems (e.g., as described by Luckow et al., Bio/Technology, 6, 47 (1988)), and established cell lines, such as COS-7, C127, 3T3, CHO, HeLa, BHK, and the like. An especially preferred expression system for preparing chimeric proteins (peptides) according to the invention is the baculoviral expression system wherein Trichoplusia ni, Tn 5B1-4 insect cells, or other appropriate insect cells, are used to produce high levels of recombinant proteins. The ordinary skilled artisan is, of course, aware that the choice of expression host has ramifications for the type of peptide produced. For instance, the glycosylation of peptides produced in yeast or mammalian cells (e.g., COS-7 cells) will differ from that of peptides produced in bacterial cells, such as Escherichia coli.

In a preferred embodiment, the proteins are expressed in mammalian cells. Mammalian expression systems are also known in the art, and include retroviral systems. A mammalian promoter is any DNA sequence capable of binding mammalian RNA polymerase and initiating the downstream (3') transcription of a coding sequence for a protein into mRNA. A promoter will have a transcription initiating region, which is usually placed proximal to the 5' end of the coding sequence, and a TATA box, using a located 25-30 base pairs upstream of the transcription initiation site. The TATA box is thought to direct RNA polymerase II to begin RNA synthesis at the correct site. A mammalian promoter will also contain an upstream promoter element (enhancer element), typically located within 100 to 200 base pairs upstream of the TATA box. An upstream promoter element determines the rate at which transcription is initiated and can act in either orientation. Of particular use as mammalian promoters are the promoters from mammalian viral genes, since the viral genes are often highly expressed and have a broad host range. Examples include the SV40 early promoter, mouse mammary tumor virus LTR promoter, adenovirus major late promoter, herpes simplex virus promoter, and the CMV promoter.

Typically, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-translational cleavage and polyadenylation. Examples of transcription terminator and polyadenlytion signals include those derived form SV40.

The methods of introducing exogenous nucleic acid into mammalian hosts, as well as other hosts, is well known in the art, and will vary with the host cell used. Techniques include dextran-mediated transfection, calcium phosphate precipitation, polybrene mediated transfection, protoplast fusion, electroporation, viral infection, encapsulation of the polynucleotide(s) in liposomes, and direct microinjection of the DNA into nuclei.

The protein may also be made as a fusion protein, using techniques well known in the art. Thus, for example, the protein may be made as a fusion protein to increase expression, or for other reasons. For example, when the protein is a peptide, the nucleic acid encoding the peptide may be linked to other nucleic acid for expression purposes.

To test for CD8, the protein is purified or isolated after expression. Proteins may be isolated or purified in a variety of ways known to those skilled in the art depending on what other components are present in the sample. Standard purification methods include electrophoretic, molecular, immunological and chromatographic techniques, including ion exchange, hydrophobic, affinity, and reverse-phase HPLC chromatography, and chromatofocusing. For example, the CD8 protein may be purified using a standard anti-CD8 antibody column. Ultrafiltration and diafiltration techniques, in conjunction with protein concentration, are also useful. For general guidance in suitable purification techniques, see Scopes, R., Protein Purification, Springer-Verlag, NY (1982). The degree of purification necessary will vary depending on the use of the CD8 protein. In some instances no purification will be necessary in some instances CD8 expression is detected on the cell surface, for example by antibody binding and detection via fluorescence or by Fluorescence Activated Cell Sorting (FACS).

Nucleic acid molecules encoding CD8 as well as any nucleic acid molecule derived from either the coding or non-coding strand of a CD8 nucleic acid molecule may be contacted with cells of an target in a variety of ways that are known and routinely employed in the art, wherein the contacting may be *ex vivo* or *in vivo.*

Viral attachment, entry and gene expression can be evaluated initially by using the adenoviral vector containing the insert of interest to generate a recombinant virus expressing the desired protein or RNA and a marker gene, such as β-galactosidase. β-galactosidase expression in cells infected with adenovirus containing the β-galactosidase gene (Ad-LacZ) can be detected as early as two hours after adding Ad-Gluc to cells. This procedure provides a quick and efficient analysis of cell entry of the recombinant virus and gene expression, and is implemented readily by an artisan of ordinary skill using conventional techniques.

Using the nucleic acids of the present invention which encode a protein, a variety of expression vectors can be made. The expression vectors may be either self-replicating extrachromosomal vectors or vectors which integrate into a host genome. Generally, these expression vectors include transcriptional and translational regulatory nucleic acid operably linked to the nucleic acid encoding the protein. The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. As another example, operably linked refers to DNA sequences linked so as to be contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice. The transcriptional and translational regulatory nucleic acid will generally be appropriate to the host cell used to express the CD8; for example, human transcriptional and translational regulatory nucleic acid sequences are preferably used to express the CD8 in human cells. Numerous types of appropriate expression vectors, and suitable regulatory sequences are known in the art for a variety of host cells.

In general, the transcriptional and translational regulatory sequences may include, but are not limited to, promoter sequences, ribosomal binding sites, transcriptional start and stop sequences, translational start and stop sequences, and enhancer or activator sequences. In a preferred embodiment, the regulatory sequences include a promoter and transcriptional start and stop sequences.

Promoter sequences encode either constitutive or inducible promoters. The promoters may be either naturally occurring promoters or hybrid promoters. Hybrid promoters, which combine elements of more than one promoter, are also known in the art, and are useful in the present invention.

In addition, the expression vector may comprise additional elements. For example, the expression vector may have two replication systems, thus allowing it to be maintained in two organisms, for example in mammalian or insect cells for expression and in a procaryotic host for cloning and amplification. Furthermore, for integrating expression vectors, the expression vector contains at least one sequence homologous to the host cell genome, and preferably two homologous sequences which flank the expression construct. The integrating vector may be directed to a specific locus in the host cell by selecting the appropriate homologous sequence for inclusion in the vector. Constructs for integrating vectors are well known in the art.

In a further embodiment, the expression vector may contain a selectable marker gene to allow the selection of transformed host cells. Selection genes are well known in the art and will vary with the host cell used. ,

Preferably, the vector is a viral vector, such as an adenoviral vector . Most preferably, the viral vector is an adenoviral vector. An adenoviral vector can be derived from any adenovirus. An "adenovirus" is any virus of the family Adenoviridae, and desirably is of the genus Mastadenovirus (e.g., mammalian adenoviruses) or Aviadenovirus (e.g., avian adenoviruses). The adenovirus is of any serotype. Adenoviral stocks that can be employed as a source of adenovirus can be amplified from the adenoviral serotypes 1 through 47, which are currently available from the American Type Culture Collection (ATCC, Rockville, Md.), or from any other serotype of adenovirus available from any other source. For instance, an adenovirus can be of subgroup A (e.g., serotypes 12, 18, and 31), subgroup B (e.g., serotypes 3, 7, 11, 14, 16, 21, 34, and 35), subgroup C (e.g., serotypes 1, 2, 5, and 6), subgroup D (e.g., serotypes 8, 9, 10, 13, 15, 17, 19, 20, 22-30, 32, 33, 36-39, and 42-47), subgroup E (serotype 4), subgroup F (serotypes 40 and 41), or any other adenoviral serotype. Preferably, however, an adenovirus is of serotypes 2, 5 or 9. Desirably, an adenovirus comprises coat proteins (e.g:, penton base, hexon, and/or fiber) of the same serotype. However, also preferably, one or more coat proteins can be chimeric, in the sense, for example, that all or a part of a given coat protein can be from another serotype.

Preferably, the viral vector is replication-deficient or conditionally replication-deficient. For example, the viral vector which is preferably an adenoviral vector, comprises a genome with at least one modification that renders the virus replication-deficient. The modification to the viral genome includes, but is not limited to, deletion of a DNA segment, addition of a DNA segment, rearrangement of a DNA segment, replacement of a DNA segment, or introduction of a DNA lesion. A DNA segment can be as small as one nucleotide or as large as 36 kilobase pairs, i.e., the approximate size of the adenoviral genome, or 38 kilobase pairs, which is the maximum amount that can be packaged into an adenoviral virion.

Preferred modifications to the viral, in particular adenoviral, genome include, in addition to a modification that renders the virus replication-deficient, the insertion of a transgene encoding for an immunomodulatory molecule as defined herein and, additionally and preferably, at least one transgene encoding for a therapeutic molecule of interest. A virus, such as an adenovirus, also preferably can be a cointegrate, i.e., a ligation of viral, such as adenoviral, genomic sequences with other sequences, such as those of a plasmid, phage or other virus.

In terms of an adenoviral vector (particularly a replication-deficient adenoviral vector), such a vector can comprise either complete capsids (i.e., including a viral genome, such as an adenoviral genome) or empty capsids (i.e., in which a viral genome is lacking, or is degraded, e.g., by physical or chemical means). Preferably, the viral vector comprises complete capsids, i.e., as a means of carrying the transgene encoding for the immunomodulatory molecule and, optionally and preferably, at least one transgene encoding an inhibiting means. Alternatively, preferably, the transgenes may be carried into a cell on the outside of the adenoviral capsid.

To the extent that it is preferable or desirable to target a virus, such as an adenovirus, to a particular cell, the virus can be employed essentially as an endosomolytic agent in the transfer into a cell of plasmid DNA, which contains a marker gene and is complexed and condensed with polylysine covalently linked to a cell-binding ligand, such as transferrin (Cotten et al., PNAS (USA), 89, 6094-6098 (1992); and Curiel et al., PNAS (USA), 88, 8850-8854 (1991)). It has been demonstrated that coupling of the transferrin-polylysine/DNA complex and adenovirus (e.g., by means of an adenovirus-directed antibody, with transglutaminase, or via a biotin/streptavidin bridge) substantially enhances gene transfer (Wagner et al., PNAS (USA), 89, 6099-6103 (1992)).

Alternatively, one or more viral coat proteins, such as the adenoviral fiber, can be modified, for example, either by incorporation of sequences for a ligand to a cell-surface receptor or sequences that allow binding to a bispecific antibody (i.e., a molecule with one end having specificity for the fiber, and the other end having specificity for a cell-surface receptor) (PCT international patent application no. WO 95/26412 (the ’412 application) and Watkins et al., Targeting Adenovirus-Mediated Gene Delivery with Recombinant Antibodies," Abst. No. 336). In both cases, the typical fiber/cell-surface receptor interactions are abrogated, and the virus, such as an adenovirus, is redirected to a new cell-surface receptor by means of its fiber.

Alternatively, a targeting element, which is capable of binding specifically to a selected cell type, can be coupled to a first molecule of a high affinity binding pair and administered to a host cell (PCT international patent application no. WO 95/31566). Then, a gene delivery vehicle coupled to a second molecule of the high affinity binding pair can be administered to the host cell, wherein the second molecule is capable of specifically binding to the first molecule, such that the gene delivery vehicle is targeted to the selected cell type.

Along the same lines, since methods (e.g., electroporation, transformation, conjugation of triparental mating, (co-)transfection, (co-) infection, membrane fusion, use of microprojectiles, incubation with calcium phospate-DNA precipitate, direct microinjection; etc.) are available for transferring viruses, plasmids, and phages in the form of their nucleic acid sequences (i.e., RNA or DNA), a vector similarly can comprise RNA or DNA, in the absence of any associated protein, such as capsid protein, and in the absence of any envelope lipid.

Similarly, since liposomes effect cell entry by fusing with cell membranes, a vector can comprise liposomes, with constitutive nucleic acids encoding the coat protein. Such liposomes are commercially available, for instance, from Life Technologies, Bethesda, Md., and can be used according to the recommendation of the manufacturer. Moreover, a liposome can be used to effect gene delivery and liposomes having increased tranfer capacity and/or reduced toxicity *in vivo* can be used. The soluble chimeric coat protein (as produced using methods described herein) can be added to the liposomes either after the liposomes are prepared according to the manufacturer’s instructions, or during the preparation of the liposomes.

The vectors according to the invention are not limited to those that can be employed in the method of the invention, but also include intermediary-type vectors (e.g., "transfer vectors") that can be employed in the construction of gene transfer vectors.

One of the preferred methods for *in vivo* delivery of one or more nucleic acid sequences involves the use of an adenovirus expression vector. "Adenovirus expression vector" is meant to include those constructs containing adenovirus sequences sufficient to (a) support packaging of the construct and (b) to express a polynucleotide that has been cloned therein in a sense or antisense orientation. Of course, in the context of an antisense construct, expression does not require that the gene product be synthesized.

The expression vector comprises a genetically engineered form of an adenovirus. Knowledge of the genetic organization of adenovirus, a 36 kb, linear, double-stranded DNA virus, allows substitution of large pieces of adenoviral DNA with foreign sequences up to 7 kb (Grunhaus and Horwitz, 1992). In contrast to retrovirus, the adenoviral infection of host cells does not result in chromosomal integration because adenoviral DNA can replicate in an episomal manner without potential genotoxicity. Also, adenoviruses are structurally stable, and no genome rearrangement has been detected after extensive amplification. Adenovirus can infect virtually all epithelial cells regardless of their cell cycle stage. So far, adenoviral infection appears to be linked only to mild disease such as acute respiratory disease in humans.

Adenovirus is particularly suitable for use as a gene transfer vector because of its mid-sized genome, ease of manipulation, high titer, wide target-cell range and high infectivity. Both ends of the viral genome contain 100-200 base pair inverted repeats (ITRs), which are cis elements necessary for viral DNA replication and packaging. The early (E) and late (L) regions of the genome contain different transcription units that are divided by the onset of viral DNA replication. The E1 region (E1A and E1B) encodes proteins responsible for the regulation of transcription of the viral genome and a few cellular genes. The expression of the E2 region (E2A and E2B) results in the synthesis of the proteins for viral DNA replication. These proteins are involved in DNA replication, late gene expression and host cell shut-off (Renan, 1990). The products of the late genes, including the majority of the viral capsid proteins, are expressed only after significant processing of a single primary transcript issued by the major late promoter (MLP). The MLP, (located at 16.8 m.u.) is particularly efficient during the late phase of infection, and all the mRNA’s issued from this promoter possess a 5’-tripartite leader (TPL) sequence which makes them preferred mRNA’s for translation.

In a current system, recombinant adenovirus is generated from homologous recombination between shuttle vector and provirus vector. Due to the possible recombination between two proviral vectors, wild-type adenovirus may be generated from this process. Therefore, it is critical to isolate a single clone of virus from an individual plaque and examine its genomic structure.

Generation and propagation of the adenovirus vectors, which are replication deficient, depend one a unique helper cell line. In nature, adenovirus can package approximately 105% of the wild-type genome (Ghosh-Choudhury et al., 1987), providing capacity for about 2 extra kB of DNA. Combined with the approximately 5.5 kB of DNA that is replaceable in the E1 and E3 regions, the maximum capacity of the current adenovirus vector is under 7.5 kB, or about 15% of the total length of the vector. More than 80% of the adenovirus viral genome remains in the vector backbone and is the source of vector-bome cytotoxicity. Also, the replication deficiency of the E1-deleted virus is incomplete. For example, leakage of viral gene expression has been observed with the currently available vectors at high multiplicities of infection (MOI) (Mulligan, 1993).

Helper cell lines may be derived from human cells such as human embryonic kidney cells, muscle cells, hematopoietic cells or other human embryonic mesenchymal or epithelial cells. Alternatively, the helper cells may be derived from the cells of other mammalian species that are permissive for human adenovirus. Such cells include, e.g., Vero cells or other monkey embryonic mesenchymal or epithelial cells. As stated above, the currently preferred helper cell line is 293.

Recently, Racher et al. (1995) disclosed improved methods for culturing 293 cells and propagating adenovirus. In one format, natural cell aggregates are grown by inoculating individual cells into 1 liter siliconized spinner flasks (Techne, Cambridge, UK) containing 100-200 ml of medium. Following stirring at 40 rpm, the cell viability is estimated with trypan blue. In another format, Fibra-Cel microcarriers (Bibby Sterlin, Stone, UK) (5 g/l) is employed as follows. A cell inoculum, resuspended in 5 ml of medium, is added to the carrier (50 ml) in a 250 ml Erlenmeyer flask and left stationary, with occasional agitation, for 1 to 4 h. The medium is then replaced with 50 ml of fresh medium and shaking initiated. For virus production, cells are allowed to grow to about 80% confluence, after which time the medium is replaced (to 25% of the final volume) and adenovirus added at an MOI of 0.05. Cultures are left stationary overnight, following which the volume is increased to 100% and shaking commenced for another 72 h.

In a preferred embodiment the adenovirus is a "gutless" adenovirus as is known in the art. The "gutless" adenovirus vector is a recently developed system for adenoviral gene delivery. The replication of the adenovirus requires a helper virus and a special human 293 cell line expressing both E1a and Cre, a condition that does not exist in natural environment. In the most efficient system to date, an E1-deleted helper virus is used with a packaging signal that is flanked by bacteriophage P1 loxP sites ("floxed"). Infection of the helper cells that express Cre recombinase with the gutless virus together with the helper virus with a floxed packaging signal should only yield gutless rAV, as the packaging signal is deleted from the DNA of the helper virus. However, if 293-based helper cells are used, the helper virus DNA can recombine with the Ad5 DNA that is integrated in the helper cell DNA. As a result, a wild-type packaging signal, as well as the E1 region, is regained. Thus, also production of gutless rAV on 293- (or 911-) based helper cells can result in the generation of RCA, if an E1-deleted helper virus is used.

The vector is deprived of all viral genes. Thus the vector is non-immunogenic and may be used repeatedly, if necessary. The "gutless" adenovirus vector also contains 36 kb space for accommodating transgenes, thus allowing co-delivery of a large number of genes into cells. Specific sequence motifs such as the RGD motif may be inserted into the H-I loop of an adenovirus vector to enhance its infectivity. An adenovirus recombinant is constructed by cloning specific transgenes or fragments of transgenes into any of the adenovirus vectors such as those described herein and known in the art. The adenovirus recombinant can be used to transduce epidermal cells of a vertebrate in a non-invasive mode for use as an immunizing agent.

Use of the "gutless" adenoviruses is particularly advantageous for insertion of large inserts of heterologous DNA (for a review, see Yeh. and Perricaudet, FASEB J. 11:615 (1997)). In addition, gutless adenoviral vectors and methods of making and using them are described in more detail in U.S. Patent No. 6,156,497 and 6,228,646.

Other than the requirement that the adenovirus vector be replication defective, or at least conditionally defective, the nature of the adenovirus vector is not believed to be crucial to the successful practice of the invention. The adenovirus, may be of any of the 42 different known serotypes or subgroups A-F. Adenovirus type 5 of subgroup C is the preferred starting material in order to obtain a conditional replication-defective adenovirus vector for use in the present invention, since Adenovirus type 5 is a human adenovirus about which a great deal of biochemical and genetic information is known, and it has historically been used for most constructions employing adenovirus as a vector.

As stated above, the typical vector according to the present invention is replication defective and will not have an adenovirus E1 region. Thus, it will be most convenient to introduce the transgene encoding the immunomodulatory molecule and/or additional therapeutic protein of interest at the position from which the E1-coding sequences have been removed. However, the position of insertion of the expression construct within the adenovirus sequences is not critical to the invention. The transgene(s) of interest may also be inserted in lieu of the deleted E3 region in E3 replacement vectors as described by Karlsson et al. (1986) or in the E4 region where a helper cell line or helper virus complements the E4 defect.

Adenovirus is easy to grow and manipulate and exhibits broad host range *in vitro* and *in vivo.* This group of viruses can be obtained in high titers, e.g., 109 - 1011 plaque-forming units per ml, and they are highly infective. The life cycle of adenovirus does not require integration into the host cell genome. The foreign genes delivered by adenovirus vectors are episomal and, therefore, have low genotoxicity to host cells. No side effects have been reported in studies of vaccination with wild-type adenovirus (Couch et al., 1963; Top et al., 1971), demonstrating their safety and therapeutic potential as *in vivo* gene transfer vectors.

Adenovirus vectors have been used in eukaryotic gene expression (Levrero et al., 1991; Gomez-Foix et al., 1992) and vaccine development (Grunhaus and Horwitz, 1992; Graham and Prevec, 1992). Recently, animal studies suggested that recombinant adenovirus could be used for gene therapy (Stratford-Perricaudet and Perricaudet, 1991; Stratford-Perricaudet et al., 1990; Rich et al., 1993). Studies in administering recombinant adenovirus to different tissues include trachea instillation (Rosenfeld et al., 1991; Rosenfeld et al., 1992), muscle injection. (Ragot et al., 1993), peripheral intravenous injections (Herz and Gerard, 1993) and stereotactic inoculation into the brain (Le Gal La Salle et al., 1993).

Accordingly, in a preferred embodiment, the expression vectors used herein are adenoviral vectors. Suitable adenoviral vectors include modifications of human adenoviruses such as Ad2 or Ad5, wherein genetic elements necessary for the virus to replicate *in vivo* have been removed; e.g. the E1 region, and an expression cassette coding for the exogenous gene of interest inserted into the adenoviral genome.

In addition, as described above, a preferred expression vector system is a retroviral vector system such as is generally described in PCT/US97/01019 and PCT/US97/01048.

The retroviruses are a group of single-stranded RNA viruses characterized by an ability to convert their RNA to double-stranded DNA in infected cells by a process of reverse-transcription (Coffin, 1990). The resulting DNA then stably integrates into cellular chromosomes as a provirus and directs synthesis of viral proteins. The integration results in the retention of the viral gene sequences in the recipient cell and its descendants. The retroviral genome contains three genes, gag, pol, and env that code for capsid proteins, polymerase enzyme, and envelope components, respectively. A sequence found upstream from the gag gene contains a signal for packaging of the genome into virions. Two long terminal repeat (LTR) sequences are present at the 5' and 3' ends of the viral genome. These contain strong promoter and enhancer sequences and are also required for integration in the host cell genome (Coffin, 1990).

In order to construct a retroviral vector, a nucleic acid encoding one or more oligonucleotide or polynucleotide sequences of interest is inserted into the viral genome in the place of certain viral sequences to produce a virus that is replication-defective. In order to produce virions, a packaging cell line containing the gag, pol, and env genes but without the LTR and packaging components is constructed (Mann et al., 1983). When a recombinant plasmid containing a cDNA, together with the retroviral LTR and packaging sequences is introduced into this cell line (by calcium phosphate precipitation for example), the packaging sequence allows the RNA transcript of the recombinant plasmid to be packaged into viral particles, which are then secreted into the culture media (Nicolas and Rubenstein, 1988; Temin, 1986; Mann et al., 1983). The media containing the recombinant retroviruses is then collected, optionally concentrated, and used for gene transfer. Retroviral vectors are able to infect a broad variety of cell types. However, integration and stable expression require the division of host cells (Paskind et al., 1975).

A novel approach designed to allow specific targeting of retrovirus vectors was recently developed based on the chemical modification of a retrovirus by the chemical addition of lactose residues to the viral envelope. This modification could permit the specific infection of hepatocytes via sialoglycoprotein receptors.

A different approach to targeting of recombinant retroviruses was designed in which biotinylated antibodies against a retroviral envelope protein and against a specific cell receptor were used. The antibodies were coupled via the biotin components by using streptavidin (Roux et al., 1989). Using antibodies against major histocompatibility complex class I and class II antigens, they demonstrated the infection of a variety of human cells that bore those surface antigens with an ecotropic virus *in vitro* (Roux et al., 1989). Suitable retroviral vectors include LNL6, LXSN, and LNCX (see Byun et al., Gene Ther. 3(9):780-8 (1996 for review).

AAV (Ridgeway, 1988; Hermonat and Muzycska, 1984) is a parvovirus, discovered as a contamination of adenoviral stocks. It is a ubiquitous virus (antibodies are present in 85% of the US human population) that has not been linked to any disease. It is also classified as a dependovirus, because its replication is dependent on the presence of a helper virus, such as adenovirus. Five serotypes have been isolated, of which AAV-2 is the best characterized. AAV has a single-stranded linear DNA that is encapsidated into capsid proteins VP1, VP2 and VP3 to form an icosahedral virion of 20 to 24 nm in diameter (Muzyczka and McLaughlin, 1988).

The AAV DNA is approximately 4.7 kilobases long. It contains two open reading frames and is flanked by two ITRs. There are two major genes in the AAV genome: rep and cap. The rep gene codes for proteins responsible for viral replications, whereas cap codes for capsid protein VP1-3. Each ITR forms a T-shaped hairpin structure. These terminal repeats are the only essential cis components of the AAV for chromosomal integration. Therefore, the AAV can be used as a vector with all viral coding sequences removed and replaced by the cassette of genes for delivery. Three viral promoters have been identified and named p5, p19, and p40, according to their map position. Transcription from p5 and p19 results in production of rep proteins, and transcription from p40 produces the capsid proteins (Hermonat and Muzyczka, 1984).

AAV is also a good choice of delivery vehicles due to its safety. There is a relatively complicated rescue mechanism: not only wild type adenovirus but also AAV genes are required to mobilize rAAV. Likewise, AAV is not pathogenic and not associated with any disease. The removal of viral coding sequences minimizes immune reactions to viral gene expression, and therefore, rAAV does not evoke an inflammatory response. Other disclosure related to AAV is set forth in U.S. Patent No. 6.531,456.

Other viral vectors may be employed as expression vectors in the present invention for the delivery of immunomodulatory molecules to a host cell. Vectors derived from viruses such as vaccinia virus (Ridgeway, 1988; Coupar et al., 1988), lentiviruses, polio viruses and herpes viruses may be employed. They offer several attractive features for various mammalian cells (Friedmann, 1989; Ridgeway, 1988; Coupar et al., 1988; Horwich et al., 1990).

***Delivery of Expression Vectors***

In order to effect expression of the immunomodulatory molecule (e.g. CD8 $\alpha$-chain) and/or additional therapeutic protein the expression vectors must be delivered into a cell. This delivery may be accomplished *in vitro,* as in laboratory procedures for transforming cells lines, or *in vivo* or *ex vivo,* as in the treatment of certain disease states. As described above, one preferred mechanism for delivery is via infection where the nucleic acid is encapsulated in a recombinant viral particle.

Once the expression vector has been delivered into the cell the nucleic acid encoding the desired oligonucleotide or polynucleotide sequences may be positioned and expressed at different sites. In certain embodiments, the nucleic acid encoding the construct may be stably integrated into the genome of the cell. This integration may be in the specific location and orientation via homologous recombination (gene replacement) or it may be integrated in a random, non-specific location (gene augmentation). In further and preferred embodiments, the nucleic acid may be stably maintained in the cell as a separate, episomal segment of DNA. Such nucleic acid segments or "episomes" encode sequences sufficient to permit maintenance and replication independent of or in synchronization with the host cell cycle. How the expression construct is delivered to a cell and where in the cell the nucleic acid remains is dependent on the type of expression vector employed.

In certain embodiments of the invention, the expression vector may simply consist of naked recombinant DNA or plasmids. Transfer of the vector may be performed by any of the methods mentioned above which physically or chemically permeabilize the cell membrane. This is particularly applicable for transfer *in vitro* but it may be applied to *in vivo* use as well. Dubensky et al. (1984) successfully injected polyomavirus DNA in the form of calcium phosphate precipitates into liver and spleen of adult and newborn mice demonstrating active viral replication and acute infection. Benvenisty and Reshef (1986) also demonstrated that direct intraperitoneal injection of calcium phosphate-precipitated plasmids results in expression of the transfected genes. It is envisioned that DNA encoding a gene of interest may also be transferred in a similar manner *in vivo* and express the gene product.

Another embodiment of the invention for transferring a naked DNA expression construct into cells may involve particle bombardment. This method depends on the ability to accelerate DNA-coated microprojectiles to a high velocity allowing them to pierce cell membranes and enter cells without killing them (Klein et al., 1987). Several devices for accelerating small particles have been developed. One such device relies on a high voltage discharge to generate an electrical current, which in turn provides the motive force (Yang et al., 1990). The microprojectiles used have generally consisted of biologically inert substances such as tungsten or gold beads.

Selected organs including the liver, skin, and muscle tissue of rats and mice have been bombarded *in vivo* (Yang et al., 1990; Zelenin et al., 1991). This may require surgical exposure of the tissue or cells, .to eliminate any intervening tissue between the gun and the target organ, i.e. *ex vivo* treatment. Again, DNA encoding a particular gene may be delivered via this method and still be incorporated by the present invention.

In one embodiment of the present invention, the nucleic acid molecule is introduced into target cells, by liposome-mediated nucleic acid transfer. In this regard, many liposome-based reagents are well known in the art, are commercially available and may be routinely employed for introducing a nucleic acid molecule into cells of the target. Certain embodiments of the present invention will employ cationic lipid transfer vehicles such as Lipofectamine or Lipofectin (Life Technologies), dioleoylphosphatidylethanolamine (DOPE) together with a cationic cholesterol derivative (DC cholesterol), N[1-(2,3-dioleyloxy)propyl]-N,N,N-trimethylammonium chloride (DOTMA) (Sioud et al., J. Mol. Biol. 242:831-835 (1991)), DOSPA:DOPE, DOTAP, DMRIE:cholesterol, DDAB:DOPE, and the like. Production of liposome-encapsulated nucleic acid is well known in the art and typically involves the combination of lipid and nucleic acid in a ratio of about 1:1.

***Uses of the Present Invention***

As detailed above, the methods and compositions described and enabled herein find general utility in preventing a host immune response directed against an expression vector for use, e.g., in gene therapy protocols. That is, a common problem encountered by most gene therapy protocols is the host immune response against vector-associated antigens. According to the present invention, however, this difficult problem has been overcome by the inclusion of nucleic acids encoding the subject CD8 polypeptides in the gene therapy vector. That is, a chimeric vector is used that includes a nucleic acid sequence encoding for the therapeutic molecule(s) of interest together with CD8 polypeptides. The resulting expression of CD8 polypeptide on the cell surface in conjunction with vector-associated antigens results in effective and specific inhibition of the host immune response directed to the vector-associated antigens, such as viral coat proteins present in adenoviral vectors. That is, when the viral proteins and CD8 are expressed in the same cell, CD8 allows the infected cell to inhibit the host immune response thereby prolonging the therapeutic treatment with the gene therapy vector.

Without being bound by theory, it is thought that expression of CD8 on target cells confers on the target cells the ability to induce the "veto effect" on the host immune system. That is, as described above, when cells expressing CD8 are contacted with host T cells, the T cells are downregulated or killed. Accordingly, by "veto effect" or "classical veto" is meant the ability of a target cell to downregulate the immune response against the target cell. It is thought that the CD8 molecule is necessary for induction or transfer of the veto effect. By "transfer of the veto effect" is meant that the veto effect is transferred to a cell that normally would not induce the veto effect. That is, the ability to reduce or down regulate the T cell response to a target cell is conferred upon the target cell by induced or increased expression of CD8.

Accordingly, the invention finds use in reducing the immune response to gene therapy delivery vehicles and/or target cells by inducing the veto effect. This results in the down regulation and deletion of T cells that would otherwise recognize the target cell. Likewise, this results in reduced humoral immune response.

An expression vector of the present invention additionally has utility *in vitro*. Such a vector can be used as a research tool in the study of viral clearance and persistence and in a method of assessing the efficacy of means of circumventing an immune response. Similarly, an expression vector, preferably a recombinant expression vector, specifically a viral or adenoviral vector, which comprises a transgene and at least one gene encoding for an immunomodulatory molecule, can be employed *in vivo*.

*In vivo* delivery includes, but is not limited to direct injection into the organ, via catheter, or by other means of perfusion. The nucleic acid may be administered intravascularly at a proximal location to the target organ or administered systemically. One of ordinary skill in the art will recognized the advantages and disadvantages of each mode of delivery. For instance, direct injection may produce the greatest titer of nucleic acid, but distribution of the nucleic acid will likely be uneven throughout the target. Introduction of the nucleic acid proximal to the target will generally result in greater contact with the cells of the organ, but systemic administration is generally much simpler.

In particular, expression vectors, such as recombinant adenoviral vectors, of the present invention can be used to treat any one of a number of diseases by delivering to cells corrective DNA, e.g., DNA encoding a function that is either absent or impaired. Diseases that are candidates for such treatment include, for example, cancer, e.g., melanoma or glioma, cystic fibrosis, genetic disorders, and pathogenic infections, including HIV infection.

Use of the subject compositions and methods to specifically inhibit alloimmune and autoimmune responses is described in co-pending U.S. Patent Application. Other applications of the method and compositions of the present invention will be apparent to those skilled in the art.

### *Compositions and Methods for Administering Expression Vectors*

One skilled in the art will appreciate that many suitable methods of administering an expression vector (particularly an adenoviral vector) and means of inhibiting an immune response of the present invention to an animal (see, for example, Rosenfeld et al., Science, 252, 431-434 (1991); Jaffe et al., Clin. Res., 39(2), 302A (1991); Rosenfeld et al., Clin. Res., 39(2), 311A (1991); Berkner, BioTechniques, 6, 616-629 (1988)) are available, and, although more than one route can be used for administration, a particular route can provide a more immediate and more effective reaction than another route. Pharmaceutically acceptable excipients for use in administering the expression vector and/or means of inhibiting an immune response also are well-known to those who are skilled in the art, and are readily available. The choice of excipient will be determined in part by the particular method used to administer the expression vector and for means of inhibiting an immune response. Accordingly, the present invention provides a composition comprising an expression vector encoding an immunomodulatory protein (e.g. CD8 $\alpha$-chain), alone or in further combination with a transgene, in a suitable carrier, and there are a wide variety of suitable formulations for use in the context of the present invention. In particular, the present invention provides a composition comprising an expression vector comprising a gene encoding an alpha chain of CD8 (or a functional fragment thereof) and a carrier therefor. In preferred embodiments, the expression vector further comprises a transgene encoding a therapeutic molecule or protein of interest. Such compositions can further comprise other active agents, such as therapeutic or prophylactic agents and/or immunosuppressive agents as are known in the art. The following methods and excipients are merely exemplary and are in no way limiting.

Formulations suitable for oral administration can consist of (a) liquid solutions, such as an effective amount of the compound dissolved in diluents, such as water, saline, or orange juice; (b) capsules, sachets or tablets, each containing a predetermined amount of the active ingredient, as solids or granules; (c) suspensions in an appropriate liquid; and (d) suitable emulsions. Tablet forms can include one or more of lactose, mannitol, corn starch, potato starch, microcrystalline cellulose, acacia, gelatin, colloidal silicon dioxide, croscarmellose sodium, talc, magnesium stearate, stearic acid, and other excipients, colorants, diluents, buffering agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, emulsions, gels, and the like containing, in addition to the active ingredient, such excipients as are known in the art.

Aerosol formulations can be made for administration via inhalation These aerosol formulations can be placed into pressurized acceptable propellants, such as dichlorodifluoromethane, propane, nitrogen, and the like. They also can be formulated as pharmaceuticals for non-pressurized preparations, such as in a nebulizer or an atomizer.

Formulations suitable for parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The formulations can be presented in unit-dose or multi-dose sealed containers, such as ampules and vials, and can be stored in a freeze-dried (lyophilized) condition requiring only the addition of the sterile liquid excipient, for example, water, for injections, immediately prior to use. Extemporaneous injection solutions and suspensions can be prepared from sterile powders, granules, and tablets of the kind previously described. Additionally, suppositories can be made with the use of a variety of bases, such as emulsifying bases or water-soluble bases. Formulations suitable for vaginal administration can be presented as pessaries, tampons, creams, gels, pastes, foams, or spray formulas containing, in addition to the active ingredient, such carriers as are known in the art to be appropriate.

The dose administered to an animal, particularly a human, in the context of the present invention will vary with the therapeutic transgene of interest, source of vector and/or the nature of the immunomodulatory molecule, the composition employed, the method of administration, and the particular site and organism being treated. However, preferably, a dose corresponding to an effective amount of a vector (e.g., an adenoviral vector according to the invention) is employed. An "effective amount" is one that is sufficient to produce the desired effect in a host, which can be monitored using several end-points known to those skilled in the art. For instance, one desired effect is nucleic acid transfer to a host cell. Such transfer can be monitored by a variety of means, including, but not limited to, a therapeutic effect (e.g., alleviation of some symptom associated with the disease, condition, disorder or syndrome being treated), or by evidence of the transferred gene or coding sequence or its expression within the host (e.g., using the polymerase chain reaction, Northern or Southern hybridizations, or transcription assays to detect the nucleic acid in host cells, or using immunoblot analysis, antibody-mediated detection, or particularized assays to detect protein or polypeptide encoded by the transferred nucleic acid, or impacted in level or function due to such transfer). These methods described are by no means all-inclusive, and further methods to suit the specific application will be apparent to the ordinary skilled artisan. In this regard, it should be noted that the response of a host to the introduction of a vector, such as a viral vector, in particular an adenoviral vector, as well as a vector encoding a means of inhibiting an immune response, can vary depending on the dose of virus administered, the site of delivery, and the genetic makeup of the vector as well as the transgene and the means of inhibiting an immune response.

Generally, to ensure effective transfer of the vectors of the present invention, it is preferable that about 1 to about 5,000 copies of the vector according to the invention be employed per cell to be contacted, based on an approximate number of cells to be contacted in view of the given route of administration, and it is even more preferable that about 3 to about 300 pfu enter each cell. However, this is merely a general guideline, which by no means precludes use of a higher or lower amount, as might be warranted in a particular application, either *in vitro* or *in vivo.* Similarly, the amount of a means of inhibiting an immune response, if in the form of a composition comprising a protein, should be sufficient to inhibit an immune response to the recombinant vector comprising the transgene. For example, the actual dose and schedule can vary depending on whether the composition is administered in combination with other pharmaceutical compositions, or depending on interindividual differences in pharmacokinetics, drug disposition, and metabolism. Similarly, amounts can vary in *in vitro* applications, depending on the particular cell type targeted or the means by which the vector is transferred. One skilled in the art easily can make any necessary adjustments in accordance with the necessities of the particular situation.

Although the present invention has been described with reference to preferred embodiments, persons skilled in the art will recognize that changes may be made in form and detail without departing from the scope of the invention.

**Example 1 The Veto Effect - STUDIES WITH VECTORS**

a. The Use of Plasmid Expression Vectors to Engineer Fibroblasts as Veto Cells

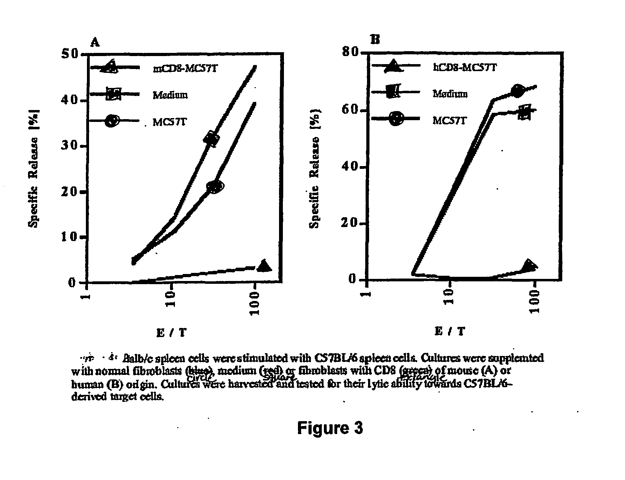

Fibroblasts were engineered to express either human or mouse CD8 - chain on their surface. Fibroblasts were transfected with the pCMVhCD8 plasmid or pCMVmCDB plasmid in which expression of the CD8 $\alpha$-chain is driven by the CMV immediate early promotor/enhancer (Invitrogen). When the CD8 -chain transfected fibroblasts (H-$2^b$) were added to mixed lymphocyte cultures (BALB/c; H-$2^d$ anti-C57BL/6; H-$2^b$), only the CD8 -chain expressing line suppressed CTL responses. As depicted in Figures 3A and B, the addition of MC57T fibroblasts expressing either the mouse or human CD8 -chain completely suppressed the induction of CTLs. In contrast, the addition of non-transfected fibroblasts did not affect T-lymphocyte activation. In addition to establishing the inhibitory function of a CD8 α-chain, these experiments also demonstrated that mouse T-lymphocytes could be veto-ed with the human CD8 α-chain. Therefore, the mouse model will be useful in examining veto designed for clinical use.

*In vivo* Function of Engineered Veto Cells

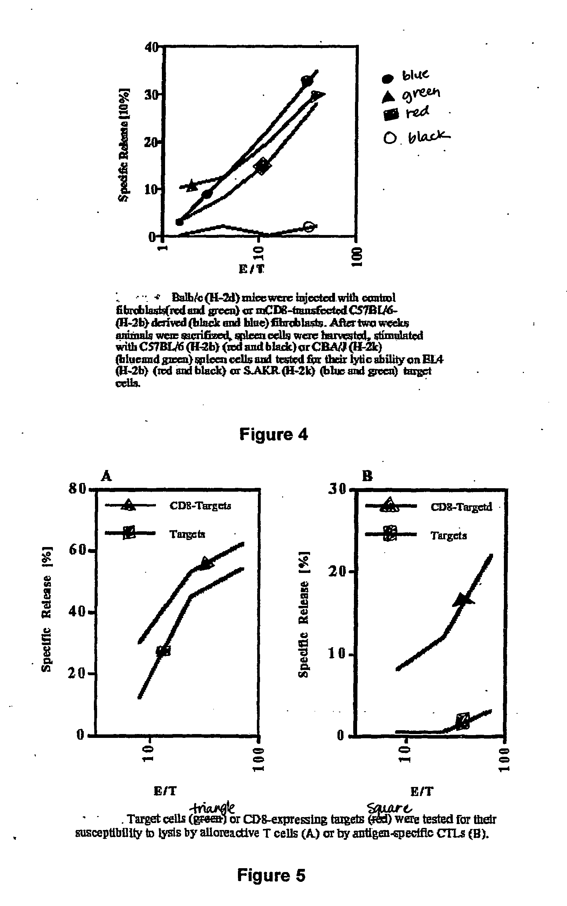

It was determined whether engineered veto functioned in the animal. C57BU6 (H-2 $^{b}$)-derived fibroblasts transfected to express the CD8 α-chain were injected into Balb/c (H-$2^d$) mice. Control animals were injected with non-transfected fibroblasts. Spleen cells were harvested after 8 to 40 days and introduced into MLCs cultures with C57BU6 (H-$2^b$) spleen cells as stimulator cells. After 5 days, cultures were harvested and tested for their ability to lyse EL4 (C57BL/6, H-$2^b$) target cells. Induction of anti-H-$2^b$ CTL responses was completely suppressed in animals that had bee n injected with CD8 - chain expressing fibroblasts (Figure 4). Inhibition of anti-H-$2^b$ T cells was highly specific. T cells from these mice still mounted responses to third party H-$2^k$ allo-MHC molecules. These experiments confirmed that engineered veto cells specifically suppressed immune responses *in vivo* similar to conventional veto cells and that non-classical veto cells could be engineered to become veto cells. In other words, engineered cells negatively immunized animals to antigens carried on these cells.

It was tested whether expression of the CD8 -chain interfered with the function of fully activated T cells. For this purpose, target cells expressing CD8 α-chains were tested for their susceptibility to lysis by fully activated CTLs. Two different T cell populations were chose for these studies, allo-reactive CTLs stimulated in a MLCs and activated peptide-specific CTLs. As depicted in Figure 5, targets expressing the CD8 α-chain were lysed efficiently by populations of alloreactive T cells, but not by antigen-specific T cells. These results suggested that engineered veto was able to interfere even with on-going antigen specific immune responses, such as those found in autoimmune responses.

b. Viral Transfer Vectors to Engineer Fibroblasts as Veto Cells

**Veto function of the Adenoviral Transfer Vector m-CD8:** A replication-deficient vector Adenoviral Transfer Vector (mAdCD8α) was developed that carried the mouse CD8 α-chain. Mouse fibroblasts (MC57) that had been infected with the mAdCDB veto transfer vector expressed high levels of the mouse CD8 α-chain on day 2. In these fast proliferating cells, expression of the mouse CD8 α-chain is significantly reduced by day 5. mAdCD8 also infected other mouse cell lines, such as EL4, albeit with lower efficiency (data not shown).

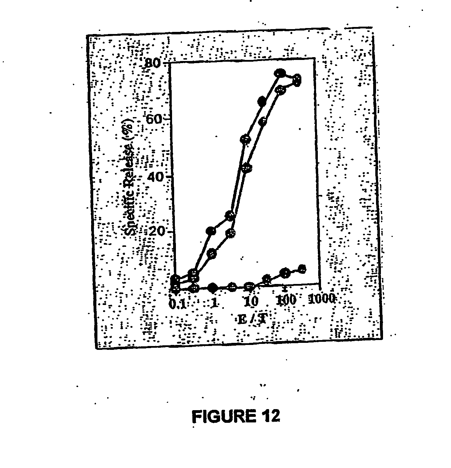

In subsequent experiments, mAdCD8 α-infected MC57 fibroblasts (H-$2^b$) were added to Balb/C (H-$2^d$) anti-C57B1/6 (H-$2^b$) MLCs. After 5 days, the cultures were harvested and tested for the presence of anti-H-$2^b$ CTLs. MLCs to which infected fibroblasts had been added, no longer contained anti- H-$2^b$ CTLs (Figure 12). These experiments established the ability of a veto transfer vector to mediate immune suppression.

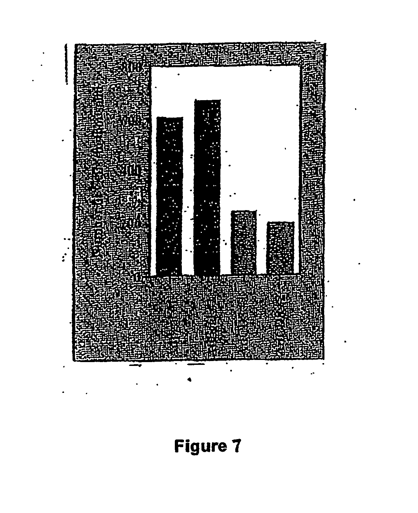

In addition, the human CD8-version of the Adenoviral vectors have been produced. Also, Adenoviral Associated Viruses that expressed mouse CD8 α-chain have been produced. It has been demonstrated that these viruses induce expression of the respective CD8 chains. Adenoviral veto vectors expressing either the mouse or the human CD8 α-chain mediated the complete inhibition of the induction of killer T cells (see Figure 7).

**Negative immunization with the mAdCD8 Veto Transfer Vector:** Two different experiments were set up to determine whether mAdCD8 suppressed immune responses *in vivo.* In the first experiment, C57Bl/6 mice were infected with equivalent doses of either the mAdCD8 veto transfer vector or a similar adenoviral control vector coding for β-galactosidase, instead of the mouse CD8 α-chain (Adβgal). Seven days after immunization, these animals were sacrificed. Single cell suspensions of their spleen cells were cultured in the presence of Adβgal viruses for 5 days. Then the cultures were harvested and their ability to proliferate was evaluated. As depicted in Figure 7, T cells proliferated vigorously to Adβgal harvested from mice immunized with Adβgal indicative of the presence of the highly proliferative $CD4^+$ T cells. In contrast, T cells harvested from mAdCD8-injected animals failed to expand.

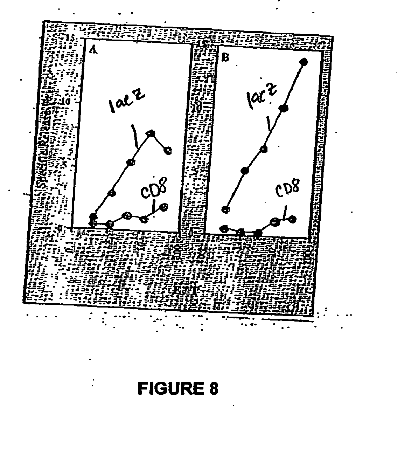

In a second step, we tested whether these cultures contained functional $CD8^+$ CTLs testing them for their ability to lyse Adβgal-infected target cells (EL4, H-$2^b$). CTLs could only be revealed in cultures established form mice injected with Adβgal (Figure 8). This first experiment suggested that AdCD8α did not induce responses to the adenoviral antigens possibly due to the expression of the CD8 α-chain. However, it was possible that AdCD8 failed to induce immune responses for different reasons. AdCD8 was non-functional in some undefined way, or the mice could only react with the β-galactosidase protein not found in mAdCD8.

To test the validity of the different conclusions, C57Bl/6 mice were injected once with either mAdCD8 or Adβgal followed by a second infusion with Adβgal after 7 days. Seven days later, mice were sacrificed, and 5-day spleen cell cultures were established in the presence of Adβgal. The responding T cells were tested for their lytic ability towards Adβgal-infected target cells (Figure 8). Indeed, two exposures to Adβgal led to improved immunization. These studies also showed that after an AdCD8 injection, mice no longer responded to Adβgal and that Adβgal primarily, if not exclusively induced CTL responses towards the adenoviral proteins common to both vectors. This set of experiments strongly suggests that it will be possible to produce a gene therapy viral vector able to negatively immunize against responses towards genes carried on these vectors.

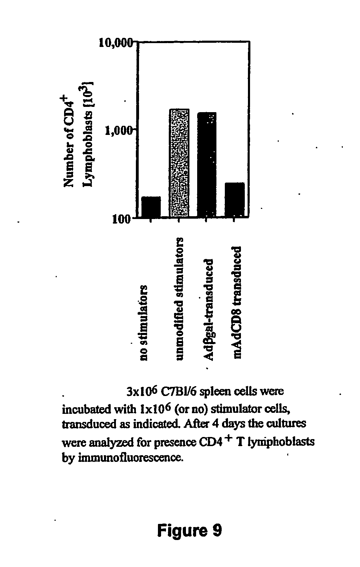

**Inhibition of CD4+ T lymphocytes by veto**: To examine whether veto transfer vectors can be used to inhibit the induction of CD4+ T lymphocytes, the following experimental system was established. C57Bl/6-derived fibroblast stimulator were transformed to express an allogeneic MHC class II molecule (H-2E$^k$) and the immune stimulatory CD80. These slow-proliferating fibroblasts non-irradiated to preserve their full stimulatory capacity, were transduced with either the mAdCD8 or the Adβgal transfer vectors and added to unselected C57B1/6 spleen cells. After 4 days, these cultures were harvested and analyzed by surface immunofluorescence for the presence of activated, i.e. blasting, CD4+ T lymphocytes (Figure 9). It was found that unselected C57Bl/6 spleen cells cultured with normal or Adβgal-transduced stimulator cells had high numbers of CD4+ T lymphoblasts. In contrast, cultures to which mAdCD8-infected stimulators had been added, only few CD4+T lymphoblasts were detected. These studies confirmed that veto inhibited CD4+ T lymphocytes and in addition that a viral veto transfer vector could be used for this purpose.

Surface Expression of the mouse and human CD8 α-chains after infection with the different virus constructs

Staining Protocols:

mAdCD8:

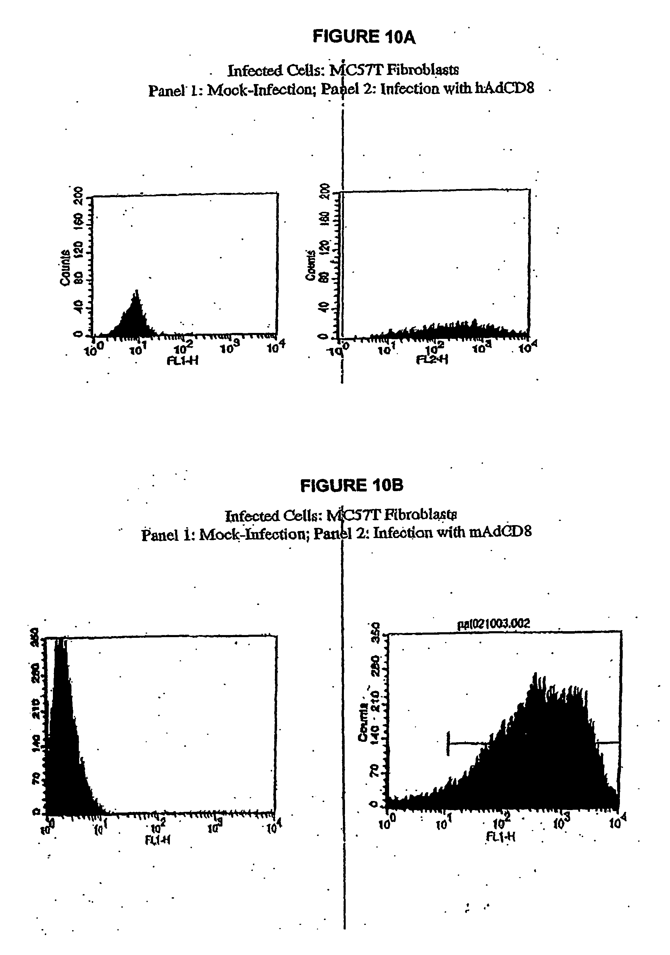

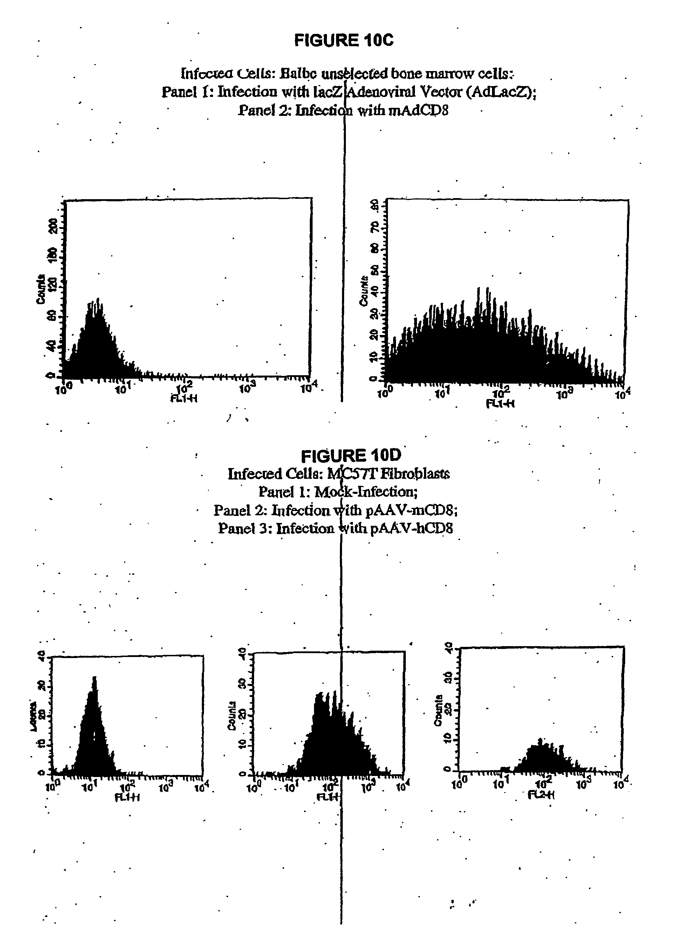

MC57T were mock-infected or infected with mAdCD8 at a multiplicity of infection of approximately $10^4$ for 3 days in modified IMDM. The infected cells were harvested and stained for the surface expression of the CD8 α-chain with the anti-mouse CD8 α-chain antibody directly labeled with FITC (Pharmingen). The extent of surface fluorescence was measured on a fluorescent activated cell analyzer (FACScan, Beckton-Dickinson) (Figure 10).

Bone marrow cells were harvested from the cavity of femoral bones of Balb/c mice. The cells were infected with a ß-galactosidase expressing Adenoviral control vector (AdLacZ) or with mAdCD8 at a multiplicity of infection of $10^4$ for 3 days cultures in modified IMDM. The infected cells were harvested and stained for the surface expression of the CD8 α-chain with the anti-mouse CD8 α-chain antibody directly labeled with FITC. The extent of surface fluorescence was measured (Figure 10C). In addition, it was determined that several cell types including CD34+ bone marrow cells, i.e. cells within the stem cell pool, were transduced efficiently (Table 2)

**Table 2**

| Marker | Cell Type | Positive Staining | |
|---|---|---|---|
| CD11a | Leucocytes | 29.3% | 31.5% |
| CD34 | Hematopoietic Lineages | 13.8% | 10.5% |
| CD19 | B Lymphocytes | 0.6% | 7.7% |
| CD3 | T Lymphocytes | 0.6% | nd |

hAdCD8:

MC57T were mock-infected. The viral titer of the hAdCD8 is not known. 100μl of its stock solution was used to infect 3x10$^5$ cells for 3 days. The infected cells were harvested and stained for the surface expression of the CD8 α-chain with the antihuman CD8 α-chain antibody directly labeled with FITC (Pharmingen). The extent of surface fluorescence was measured on a fluorescent activated cell analyzer (Figure 10).

AAV-Based Veto Vectors

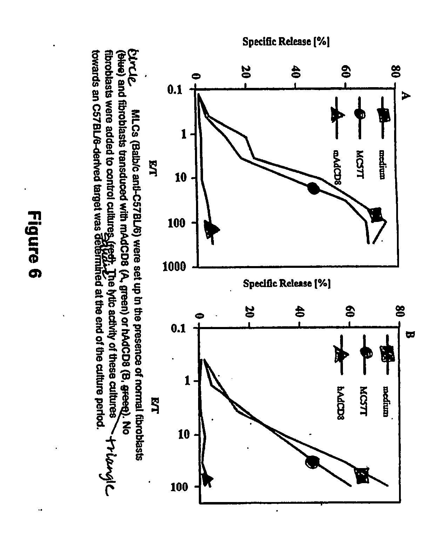

AAV-based veto vectors were produced in parallel using a Strategene/Avigen system. In these constructs, the human and mouse CD8 α-chains were driven from the same CMV intermediate early promotor/enhancer. The two viruses, mAAVCD8 and hAAVCD8 were packaged in the HEK 293 packaging cell line. The system employed is free of helper virus. mAAVCD8 and hAAVCD8 efficiently infected mouse fibroblasts (MC57T) and drove high levels of expression of the mouse or human CD8 α-chains, respectively. The extent of fluorescence was measured on a fluorescent activated cell analyzer (Figure 10D). It is interesting to note that high levels of CD8 α-chain expression was seen within 36 hours after transduction. This finding was in contrast to observation by others. They had found that AAV-driven gene expression took several days to reach significant levels (PH Schmelck, PrimeBiotech). Additional studies with AAV veto vectors reiterated our previous findings that they could be used to suppress immune responses. Here, the standard MLC protocol was used (Figure 6)

Example 2: *In vitro* Inhibition Studies - Mixed Lymphocyte Cultures

Spleen cells were harvested from Balb/c (H-$2^d$) and C57BU6 (H-$2^b$) mice. Single cell suspensions were prepared. The C57BU6 spleen cells were irradiated with 3,000 rad (Mark 1 Cesium Irradiator). 4x$10^6$ Balb/c spleen cells (responder/effector cells) were cultured together with 4x$10^6$ irradiated C57BU6 spleen cells (stimulator cells) per well in 24-well plates (TPP, Midwest Scientific, Inc.) in IMDM (Sigma) that contained 10% fetal calf serum (FCS) (Sigma), HEPES, penicillin G, streptomycin sulfate, gentamycine sulfate, L-glutamine, 2-mercaptoethanol, non-essential amino acids (Sigma), sodium pyruvate and sodium bicarbonate (modified IMDM). After 5 days of culture in a $CO_2$ incubator (Forma Scientific), the cultures were harvested in their entirety and tested for the ability to lyse C57BU6-derived target cells (H-$2^b$).

To some of these cultures 4x$10^5$ MC57T fibroblasts (H-$2^d$) were added that had been irradiated with 12,000 rad. In inhibition cultures, 4x$10^5$ MC57T cells were included that had been infected with mAdCD8 at a multiplicity of infection of approximately $10^4$ to 1 for 2 days.

Cytotoxic T Lymphocyte Killer Assays

Cells harvested from the mixed lymphocyte cultures were counted for the number of blast cells, as an indicator of activated T lymphocytes. These effector cells were added to a single well in a U-bottomed 96-well plate. The number of effectors per well was titrated in 3-fold titration steps starting from 3x$10^8$ or 1x$10^5$ effectors per well. To these effector cells 1x$10^4$ target cells EL4 (H-$2^b$), MC57T (H-$2^b$) or P815 (H-$2^d$) per well were added. The target cells had previously been labeled with $^{51}$Cr (Na-Chromate, Perkin-Elmer). 1x$10^6$ target cells had been incubated with 100 μCi in a modified IMDM in a volume of approximately 500μl for 90 min. Thereafter, the non-incorporated $^{51}$Cr was removed my multiple washes with modified IMDM.

The effector and target cells were incubated in a total volume of 200 μl for 4 hrs in a $CO_2$ incubator. Thereafter, the plates were spun in centrifuge (Centra CJ35R, International Equipment Company) at 1,500 rpm for 3 min. 100 ml of medium was removed from each well and the amount of $^{51}$Cr released from the target cells was counted in a Model 4000 Gamma counter (Beckman Instruments). Control cultures were set, in which effector cells were omitted to determine the background release. Total $^{51}$Cr incorporation into target cells was determined in wells, in which a 1% solution (w/v) of Triton X100 (Sigma) was substituted for the effector cells.

The amount of specific lysis was determined as:

$$\text{in \%} = (\text{specific release} - \text{background release}) / (\text{total release} - \text{background release}) \times 100$$

The Activity of mAdCD8 *in vitro*

Mixed lymphocyte cultures were set up (Balb/c anti-C57BL/6). To these cultures MC57T fibroblasts were added (as indicated) that had been irradiated with 12,000 rad and had been infected with mAdCD8. After 5 days of culture, the cultures were harvested and tested for their ability to lyse EL4 (H-$2^b$) target cells at different effector-to-target (E/T) ratios (see Figure 4).

As can be seen, even in the mixed lymphocyte culture, the cells expressing CD8 inhibited the induction of lytic T lymphocytes.

Production of mAdCD8 and hAdCD8

Both Adenoviral vectors were produced with the help of the AdEasy™ system from Biogene. Here the mouse and human CD8 α-chain cDNA is incorporated into the Transfer Vector (Step 1). Recombination with the Ad5ΔE1/ΔE3 vector is achieved in BJ5183 EC bacteria (Step 2). The recombinant vector is then transferred into the QBI-HEK 293A cells that contain the E1A and E1B Adenovirus 5 viral genes, which complement the deletion of this essential region in the recombinant adenovirus. The hAdCD8 and mAdCD8 produced in these cells are thus replication deficient.

As control vector expressing the bacterial LacZ gene (β-galactosidase) the Qbiogene provided QBI-Infect+ Viral Particle (Ad5.CMVLacZΔE1/ΔE3). Mouse CD8 α-chain sequence used. This sequence is similar to the published mouse sequence:

Protein-Sequence:

ACTUAL SEQUENCE: MASPLTRFLS LNLLLMGESI

ILGSGEAKPQAPELRIFPKK MDAELGQKVD LVCEVLGSVS QGCSWLFQNS

SSKLPQPTFVVYMASSHNKI TWDEKLNSSK LFSAVRDTNN KYVLTLNKFS

KENEGYYFCSVISNSVMYFS SVVPVLQKVN STTTKPVLRT PSPVHPTGTS

QPQRPEDCRPRGSVKGTGLD FACDIYIWAP LAGICVAPLL SLIITLICYH

RSRKRVCKCPRPLVRQEGKP RPSEKIV

Human CD8 α-chain sequence used. This sequence has a silent mutation compared to the published human sequence as indicated.

ACTUAL SEQUENCE: MALPVTALLL PLALLLHAAR

PSQFRVSPLDRTWNLGWTVE LKCQVLLSNP TSGCSWLFQP RGAAASPTFL

LYLSQNKPKAAEGLDTQRFS GKRLGDTFVL TLSDFRRENE GYYFCSALSN

SIMYFSHFVPVFLPAKPTTT PAPRPPTPAP TIASQPLSLR PEACRPAAGG

AGNRRRVCKCPRPVVKSGDK PSLARYV

Production of pAAV-mCD8 and pAAV-hCD8

These vectors were produced with the help of the AAV Helper-Free System from Stratagene. The system works by inserting the mouse and human sequences into the pAAV-MCS cloning vector. This plasmid is then co-transfected into HEK 293 cells together with a helper plasmid (containing the necessary Adenoviral proteins) and the pAAV-RC vector (containing the capsid genes) to produce the recombinant AAV particles.

Example 3: Engineered Veto in Animal Models

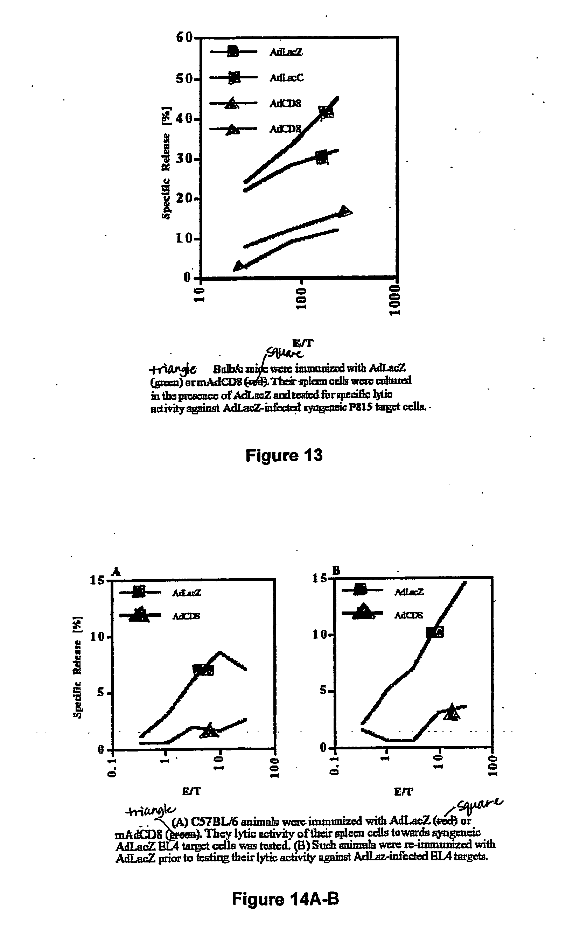

We investigated how animals responded to the injection of large doses of the mAdCD8. In the first set of experiments, Balb/c mice (two mice in each group) were injected i.v. with equivalent doses of mAdCD8 or an Adenoviral control vector coding for β-galactosidase (AdLacZ). After seven days the animals were sacrificed. Their spleen cells were cultured in the presence of AdLacZ for five days. They were then tested for their ability to lyse AdLacZ-infected target cells (P815, Balb/c-derived). As depicted in Figure 13, CTLs with specific lytic ability could be expanded from Balb/c mice that had been immunized with AdLacZ, but not from mice that had received the mAdCD8. This result suggested that AdCD8 did not induce immune responses to Adenoviral antigens due to the expression of the CD8 α-chain.

In a second set-up, C57Bl/6 mice were immunized with equivalent doses of mAdCD8 (2 mice) or AdLacZ (2 mice). Seven days after immunization, one animal of each group was sacrificed. Their spleen cells were cultured in cell suspension in the presence of AdLacZ for five days. They were then tested for their ability to specifically lyse AdLacZ-infected target cells (EL-4, C57Bl/6-derived). Again, injection of AdLacZ had induced the development of specific killer cells albeit at a low frequency, whereas mAdCDB had failed to do so (Figure 14).

In the second phase of this experiments, the remaining C57BL/6 mice that had received either mAdCD8 or AdLacZ received a second dose of AdLacZ seven days after their first viral injection. Seven days later, mice were sacrificed, and five-day spleen cell cultures were established in the presence of AdLacZ. The responding T cells were again tested for their lytic ability towards AdLacZ-infected EL4-target cells (Figure 8). Indeed, two exposures to AdLacZ led to a somewhat improved immunization. However, the animal that had previously received mAdCD8 still failed to mount a response. These experiments suggest that AdCD8 not only failed to induce immune responses, but prevented the induction immune responses directed against itself. Thus, mAdCD8 evaded the immune system.

## Claims

**1.** A replication-deficient recombinant adenovirus vector comprising:

i) a first nucleic acid encoding an extracellular domain of a CD8 α-chain operably linked to a nucleic acid encoding a transmembrane polypeptide; and
ii) a second nucleic acid comprising a therapeutic transgene encoding a therapeutic molecule of interest;

for use in gene therapy for increasing the persistence of the therapeutic transgene in a host thereby improving the expression of the therapeutic transgene, wherein the extracellular domain of the CD8 α-chain is expressed on the surface of a host cell and wherein a host immune response against vector-associated antigens including said therapeutic molecule of interest is inhibited.

**2.** Use of a replication-deficient recombinant adenovirus vector comprising:

i) a first nucleic acid encoding an extracellular domain of a CD8 α-chain operably linked to a nucleic acid encoding a transmembrane polypeptide; and
ii) a second nucleic acid comprising a therapeutic transgene encoding a therapeutic molecule of interest;

in the preparation of a medicament for use in increasing the persistence of the therapeutic transgene in a host thereby improving the expression of the therapeutic transgene, wherein the extracellular domain of the CD8 α-chain is expressed on the surface of a host cell and wherein a host immune response against vector-associated antigens including said therapeutic molecule of interest is inhibited.

**3.** The replication-deficient recombinant adenovirus vector for use in gene therapy according to claim 1 or the use of claim 2, wherein said therapeutic molecule of interest is selected from the group consisting of hemoglobin-β, GATA-binding protein, d-aminoevulinate synthase, glucose-6-phosphate-dehydrogenase, Coagulation Factor VIII, Coagulation Factor XI, cystic fibrosis transmembrane conductance regulator, ornithine carbamoyl transferase, α-L-iduronidase, iduronate-2-sulfatase, β-glucosidase, α-galactosidase, galactosylceramidase, acid α-glucosidase, hexamidase A, phenylalanine hydroxylase, collagen type IV, α5, Bloom Syndrome Gene Product, and low density lipoprotein receptor.

## Patentansprüche

**1.** Replikationsdefizienter rekombinanter Adenovirusvektor, der Folgendes umfasst:

i) eine erste Nukleinsäure, die für eine extrazelluläre Domäne einer CD8-α-Kette codiert, in operativer Verknüpfung mit einer Nukleinsäure, die für ein Transmembranpolypeptid codiert; und
ii)eine zweite Nukleinsäure, die ein therapeutisches Transgen, das für ein interessierendes therapeutisches Molekül codiert, umfasst;

für die Verwendung in der Gentherapie zum Erhöhen der Persistenz des therapeutischen Transgens in einen Wirt, wodurch die Expression des therapeutischen Transgens verbessert wird, wobei die extrazelluläre Domäne der CD8-α-Kette an der Oberfläche einer Wirtszelle exprimiert wird und wobei eine Immunantwort des Wirts gegen mit dem Vektor assoziierte Antigene, einschließlich des interessierenden therapeutischen Moleküls, inhibiert wird.

**2.** Verwendung eines replikationsdefizienten rekombinanten Adenovirusvektors, der Folgendes umfasst:

i) eine erste Nukleinsäure, die für eine extrazelluläre Domäne einer CD8-α-Kette codiert, in operativer Verknüpfung mit einer Nukleinsäure, die für ein Transmembranpolypeptid codiert; und
ii) eine zweite Nukleinsäure, die ein therapeutisches Transgen, das für ein interessierendes therapeutisches Molekül codiert, umfasst;

in der Herstellung eines Arzneimittels für die Verwendung zum Erhöhen der Persistenz des therapeutischen Transgens in einen Wirt, wodurch die Expression des therapeutischen Transgens verbessert wird, wobei die extrazelluläre Domäne der CD8-α-Kette an der Oberfläche einer Wirtszelle exprimiert wird und wobei eine Immunantwort des Wirts gegen mit dem Vektor assoziierte Antigene, einschließlich des interessierenden therapeutischen Moleküls, inhibiert wird.

3. Replikationsdefizienter rekombinanter Adenovirusvektor für die Verwendung in der Gentherapie nach Anspruch 1 oder die Verwendung nach Anspruch 2, wobei das interessierende therapeutische Molekül aus der Gruppe bestehend aus Hämoglobin-β, GATA-Binding-Protein, d-Aminoevulinatsynthase, Glucose-6-phosphatdehydrogenase, Coagulationsfaktor VIII, Coagulationsfaktor XI, CFTR (Cystic Fibrosis Transmembrane Conductance Regulator), Ornithincarbamoyltransferase, α-L-Iduronidase, Iduronat-2-sulfatase, β-Glucosidase, α-Galactosidase, Galactosylceramidase, saure α-Glucosidase, Hexamidase A, Phenylalaninhydroxylase, Collagen Typ IV, α5, Bloom Syndrome Gene Product sowie Low-Density-Lipoproteinrezeptor ausgewählt ist.

## Revendications

1. Vecteur adénoviral recombinant déficient pour la réplication, comprenant :

   i) un premier acide nucléique codant pour un domaine extracellulaire de la chaîne α de CD8 lié de manière opérationnelle à un acide nucléique codant pour un polypeptide transmembranaire ; et
   ii) un deuxième acide nucléique, comprenant un transgène thérapeutique codant pour une molécule thérapeutique d'intérêt ;

   pour utilisation en génothérapie, pour augmenter la persistance du transgène thérapeutique dans un hôte, en améliorant de ce fait l'expression du transgène thérapeutique, dans lequel le domaine extracellulaire de la chaîne α de CD8 est exprimé sur la surface d'une cellule hôte, et une réponse immune de l'hôte contre des antigènes associés au vecteur, comprenant ladite molécule thérapeutique d'intérêt, est inhibée.

2. Utilisation d'un vecteur adénoviral recombinant déficient pour la réplication, comprenant :

   i) un premier acide nucléique codant pour un domaine extracellulaire d'une chaîne α de CD8 lié de manière opérationnelle à un acide nucléique codant pour un polypeptide transmembranaire ; et
   ii) un deuxième acide nucléique, comprenant un transgène thérapeutique codant pour une molécule thérapeutique d'intérêt ;

   pour la préparation d'un médicament destiné à une utilisation pour augmenter la persistance du transgène thérapeutique dans un hôte, en améliorant de ce fait l'expression du transgène thérapeutique, le domaine extracellulaire de la chaîne α de CD8 étant exprimé sur la surface d'une cellule hôte, et une réponse immune de l'hôte contre des antigènes associés au vecteur, comprenant ladite molécule thérapeutique d'intérêt, étant inhibée.

3. Vecteur adénoviral recombinant déficient pour la réplication, pour utilisation en génothérapie selon la revendication 1, ou utilisation de la revendication 2, la molécule thérapeutique d'intérêt étant choisie dans le groupe consistant en l'hémoglobine β, la protéine de liaison GATA, la d-aminoévulinate synthase, la glucose-6-phosphate-déshydrogénase, le facteur de coagulation VIII, le facteur de coagulation XI, le régulateur de la conductance transmembranaire de la mucoviscidose, l'ornithine carbamoyl transférase, l'α-L-iduronidase, l'iduronate-2-sulfatase, la β-glucosidase, l'a-galactosidase, la galactosylcéramidase, l'α-glucosidase acide, l'hexamidase A, la phénylalanine hydroxylase, la chaîne α5 du collagène de type IV, le produit génique du syndrome de Bloom et le récepteur des lipoprotéines basse densité.

# FIGURE 1

## CD8 α-chain sequences

NM_001768 & M27161
Homo sapiens (Human)
Complete CD8 alpha mRNA

**Predicted polypeptide sequence**

MALPVTALLLPLALLLHAARPSQFRVSPLDRTWNLGETVELKCQ

VLLSNPTSGCSWLFQPRGAAASPTFLLYLSQNKPKAAEGLDTQRFSGKRLGDTFVLTL

SDFRRENEGYYFCSALSNSIMYFSHFVPVFLPAKPTTTPAPRPPTPAPTIASQPLSLR

PEACRPAAGGAVHTRGLDFACDIYIWAPLAGTCGVLLLSLVITLYCNHRNRRRVCKCP

RPVVKSGDKPSLSARYV

**mRNA**

1 gaaatcaggc tccgggccgg ccgaagggcg caactttccc ccctcggcgc cccaccggct
61 cccgcgcgcc tcccctcgcg cccgagcttc gagccaagca gcgtcctggg gagcgcgtca
121 tggccttacc agtgaccgcc ttgctcctgc cgctggcctt gctgctccac gccgccaggc
181 cgagccagtt ccgggtgtcg ccgctggatc ggacctggaa cctgggcgag acagtggagc
241 tgaagtgcca ggtgctgctg tccaacccga cgtcgggctg ctcgtggctc ttccagccgc
301 gcggcgccgc cgccagtccc accttcctcc tatacctctc ccaaaacaag cccaaggcgg
361 ccgaggggct ggacacccag cggttctcgg gcaagaggtt gggggacacc ttcgtcctca
421 ccctgagcga cttccgccga gagaacgagg gctactattt ctgctcggcc ctgagcaact
481 ccatcatgta cttcagccac ttcgtgccgg tcttcctgcc agcgaagccc accacgacgc
541 cagcgccgcg accaccaaca ccggcgccca ccatcgcgtc gcagcccctg tccctgcgcc
601 cagaggcgtg ccggccagcg gcggggggcg cagtgcacac gagggggctg gacttcgcct
661 gtgatatcta catctgggcg cccttggccg ggacttgtgg ggtccttctc ctgtcactgg
721 ttatcaccct ttactgcaac cacaggaacc gaagacgtgt ttgcaaatgt ccccggcctg
781 tggtcaaatc gggagacaag cccagccttt cggcgagata cgtctaaccc tgtgcaacag
841 ccactacatt acttcaaact gagatccttc cttttgaggg agcaagtcct tccctttcat
901 tttttccagt cttcctccct gtgtattcat tctcatgatt attattttag tgggggcggg
961 gtgggaaaga ttactttttc tttatgtgtt tgacgggaaa caaaactagg taaaatctac
1021 agtacaccac aagggtcaca atactgttgt gcgcacatcg cggtagggcg tggaaagggg
1081 caggccagag ctacccgcag agttctcaga atcatgctga gagagctgga ggcacccatg
1141 ccatctcaac ctcttccccg cccgttttac aaagggggag gctaaagccc agagacagct
1201 tgatcaaagg cacacagcaa gtcagggttg gagcagtagc tggagggacc ttgtctccca
1261 gctcagggct ctttcctcca caccattcag gtctttcttt ccgaggcccc tgtctcaggg

## FIGURE 1

1321 tgaggtgctt gagtctccaa cggcaaggga acaagtactt cttgatacct gggatactgt
1381 gcccagagcc tcgaggaggt aatgaattaa agaagagaac tgcctttggc agagttctat
1441 aatgtaaaca atatcagact ttttttttt ataatcaagc ctaaaattgt atagacctaa
1501 aataaaatga agtggtgagc ttaaccctgg aaaatgaatc cctctatctc taaagaaaat
1561 ctctgtgaaa cccctatgtg gaggcggaat tgctctccca gcccttgcat tgcagagggg
1621 cccatgaaag aggacaggct accccttac aaatagaatt tgagcatcag tgaggttaaa
1681 ctaaggccct cttgaatctc tgaatttgag atacaaacat gttcctggga tcactgatga
1741 ctttttatac tttgtaaaga caattgttgg agagcccctc acacagccct ggcctctgct
1801 caactagcag atacagggat gaggcagacc tgactctctt aaggaggctg agagcccaaa
1861 ctgctgtccc aaacatgcac ttccttgctt aaggtatggt acaagcaatg cctgcccatt
1921 ggagagaaaa aacttaagta gataaggaaa taagaaccac tcataattct tcaccttagg
1981 aataatctcc tgttaatatg gtgtacattc ttcctgatta ttttctacac atacatgtaa
2041 aatatgtctt tcttttttaa atagggttgt actatgctgt tatgagtggc tttaatgaat
2101 aaacatttgt agcatcctct ttaatgggta aacagcaaaa aaaaaaaaaa aaaaaaaaaa
2161 aaaaaaaaaa aaaaaaaaaa aaaaaaaaaa aaaaaaaaaa aaaaaaaaaa aaaaaaaaaa
2221 aaaaaaaaaa aaaaaaaaaa aaaaaaaaaa aaaaaaaaaa a

## FIGURE 1

NM_171827

Homo sapiens secreted protein derived from alternate transcript

**Predicted polypeptide**

MALPVTALLLPLALLLHAARPSQFRVSPLDRTWNLGETVELKCQVLLSNPTSGCSWLFQPRGAAASPTI
LYLSQNKPKAAEGLDTQRFSGKRLGDTFVLTLSDFRRENEGYYFCSALSNSIMYFSHFVPVFLPAKPTT
PAPRPPTPAPTIASQPLSLRPEACRPAAGGAGNRRRVCKCPRPVVKSGDKPSLSARYV

**mRNA**

1 gaaatcaggc tccgggccgg ccgaagggcg caactttccc ccctcggcgc cccaccggct
61 cccgcgcgcc tcccctcgcg cccgagcttc gagccaagca gcgtcctggg gagcgcgtca
121 tggccttacc agtgaccgcc ttgctcctgc cgctggcctt gctgctccac gccgccaggc
181 cgagccagtt ccgggtgtcg ccgctggatc ggacctggaa cctgggcgag acagtggagc
241 tgaagtgcca ggtgctgctg tccaacccga cgtcgggctg ctcgtggctc ttccagccgc
301 gcggcgccgc cgccagtccc accttcctcc tatacctctc ccaaaacaag cccaaggcgg
361 ccgaggggct ggacacccag cggttctcgg gcaagaggtt gggggacacc ttcgtcctca
421 ccctgagcga cttccgccga gagaacgagg gctactattt ctgctcggcc ctgagcaact
481 ccatcatgta cttcagccac ttcgtgccgg tcttcctgcc agcgaagccc accacgacgc
541 cagcgccgcg accaccaaca ccggcgccca ccatcgcgtc gcagcccctg tccctgcgcc
601 cagaggcgtg ccggccagcg gcgggggggcg cagggaaccg aagacgtgtt tgcaaatgtc
661 cccggcctgt ggtcaaatcg ggagacaagc ccagcctttc ggcgagatac gtctaaccct
721 gtgcaacagc cactacatta cttcaaactg agatccttcc ttttgaggga gcaagtcctt
781 ccctttcatt ttttccagtc ttcctccctg tgtattcatt ctcatgatta ttattttagt
841 gggggcgggg tgggaaagat tactttttct ttatgtgttt gacgggaaac aaaactaggt
901 aaaatctaca gtacaccaca agggtcacaa tactgttgtg cgcacatcgc ggtagggcgt
961 ggaaaggggc aggccagagc tacccgcaga gttctcagaa tcatgctgag agagctggag
1021 gcacccatgc catctcaacc tcttccccgc ccgttttaca aagggggagg ctaaagccca
1081 gagacagctt gatcaaaggc acacagcaag tcagggttgg agcagtagct ggagggacct
1141 tgtctcccag ctcagggctc tttcctccac accattcagg tctttctttc cgaggcccct
1201 gtctcagggt gaggtgcttg agtctccaac ggcaagggaa caagtacttc ttgatacctg
1261 ggatactgtg cccagagcct cgaggaggta atgaattaaa gaagagaact gcctttggca
1321 gagttctata atgtaaacaa tatcagactt ttttttttta taatcaagcc taaaattgta
1381 tagacctaaa ataaaatgaa gtggtgagct taaccctgga aaatgaatcc ctctatctct
1441 aaagaaaatc tctgtgaaac ccctatgtgg aggcggaatt gctctcccag cccttgcatt
1501 gcagaggggc ccatgaaaga ggacaggcta cccctttaca aatagaattt gagcatcagt
1561 gaggttaaac taaggccctc ttgaatctct gaatttgaga tacaaacatg ttcctgggat
1621 cactgatgac tttttatact ttgtaaagac aattgttgga gagcccctca cacagccctg
1681 gcctctgctc aactagcaga tacagggatg aggcagacct gactctctta aggaggctga

## FIGURE 1

1741 gagcccaaac tgctgtccca aacatgcact tccttgctta aggtatggta caagcaatgc
1801 ctgcccattg gagagaaaaa acttaagtag ataaggaaat aagaaccact cataattctt
1861 caccttagga ataatctcct gttaatatgg tgtacattct tcctgattat tttctacaca
1921 tacatgtaaa atatgtcttt cttttttaaa tagggttgta ctatgctgtt atgagtggct
1981 ttaatgaata aacatttgta gcatcctctt taatgggtaa acagcaaaaa aaaaaaaaaa
2041 aaaaaaaaaa aaaaaaaaaa aaaaaaaaaa aaaaaaaaaa aaaaaaaaaa aaaaaaaaaa
2101 aaaaaaaaaa aaaaaaaaaa aaaaaaaaaa aaaaaaaaaa aaaaaaaaaa

## FIGURE 1

X60223
Pongo pygmaeus (Orangutan)
Complete CD8 alpha mRNA

**Predicted polypeptide**

MALPVTALLLPLALLLHAARPSQFRVSPLDRTWNLGETVELKCQ

VLLSNPTSGCSWLFQPRGAAASPTFLLYLSQNKPKAAEGLDTQRFSGKRLGDTFVLTL

SDFRRENEGYYFCSALSNSIMYFSHFVPVFLPVHTRGLDFACDIYIWAPLAGTCGVLL

LSLVITLYCNHRNRRRVCKCPRPVVKSGGKPSLSERYV

**mRNA**

1 atggccttac ccgtgaccgc cttgctcctg ccgctggcct tgctgctcca cgccgccagg
61 ccgagccagt tccgggtgtc gccgctggat cggacctgga acctgggcga gacggtggag
121 ctgaagtgcc aggtgctgct gtccaacccg acgtctggct gctcctggct cttccagccg
181 cgtggcgccg ccgccagtcc caccttcctc ctatacctct cccaaaacaa gcccaaggcg
241 gccgaggggc tggacaccca gcggttctcg ggcaagaggt tgggggacac cttcgtcctc
301 accctgagcg acttccgccg ggagaacgaa ggctactatt tctgctcggc cctgagcaac
361 tccatcatgt acttcagcca cttcgtgccg gtcttcctgc cagtgcacac gagggggctg
421 gacttcgcct gtgatatcta catctgggcg cccttggccg ggacctgtgg ggtccttctc
481 ctgtcactgg ttatcaccct ttactgcaac cacaggaacc gaagacgtgt ttgcaaatgt
541 ccccggcctg tggtcaaatc tggaggcaag cccagccttt cggagagata tgtctaa

## FIGURE 1

XM_132621 & BC030679 & U34881
Mus musculus (Mouse)
Complete CD8 alpha mRNA

**Predicted polypeptide**

MASPLTRFLSLNLLLLGESIILGSGEAKPQAPELRIFPKKMDAE

LGQKVDLVCEVLGSVSQGCSWLFQNSSSKLPQPTFVVYMASSHNKITWDEKLNSSKLF

SAMRDTNNKYVLTLNKFSKENEGYYFCSVISNSVMYFSSVVPVLQKVNSTTTKPVLRT

PSPVHPTGTSQPQRPEDCRPRGSVKGTGLDFACDIYIWAPLAGICVALLLSLIITLIC

YHRSRKRVCKCPSIACLCLKLQGSKWYESVICSALAVSIRCNKSKSGELPLAVHLDIR

APCKNWEIAGSLVERYGKSGKHSPLSLKAVVESN

**mRNA**

1 atggcctcac cgttgacccg ctttctgtcg ctgaacctgc tgctgctggg tgagtcgatt
61 atcctgggga gtggagaagc taagccacag gcacccgaac tccgaatctt tccaaagaaa
121 atggacgccg aacttggtca gaaggtggac ctggtatgtg aagtgttggg gtccgtttcg
181 caaggatgct cttggctctt ccagaactcc agctccaaac tcccccagcc caccttcgtt
241 gtctatatgg cttcatccca caacaagata acgtgggacg agaagctgaa ttcgtcgaaa
301 ctgttttctg ccatgaggga cacgaataat aagtacgttc tcaccctgaa caagttcagc
361 aaggaaaacg aaggctacta tttctgctca gtcatcagca actcggtgat gtacttcagt
421 tctgtcgtgc cagtccttca gaaagtgaac tctactacta ccaagccagt gctgcgaact
481 ccctcacctg tgcaccctac cgggacatct cagccccaga gaccagaaga ttgtcggccc
541 cgtggctcag tgaaggggac cggattggac ttcgcctgtg atatttacat ctgggcaccc
601 ttggccggaa tctgcgtggc ccttctgctg tccttgatca tcactctcat ctgctaccac
661 aggagccgaa agcgtgtttg caaatgtccc agtatagcat gcttgtgcct caaactgcaa
721 ggaagcaagt ggtatgaatc tgtgatctgc tcagctctgg ctgtgagcat cagatgtaac
781 aaatcaaagt caggagaact gcctttagcg gtgcacctgg acatcagagc cccttgtaag
841 aactgggaaa ttgctggcag tctagtggag cggtacggta aatctggaaa acactcccct
901 ctgtcactga aggctgtagt agaatccaat taa

**Predcited polypeptide**

MDAELGQKVDLVCEVLGSVSQGCSWLFQNSSSKLPQPTFVVYMA

SSHNKITWDEKLNSSKLFSAMRDTNNKYVLTLNKFSKENEGYYFCSVISNSVMYFSSV

VPVLQKVNSTTTKPVLRTPSPVHPTGTSQPQRPEDCRPRGSVKGTGLDFACDIYIWAP

LAGICVALLLSLIITLICYHRSRKRVCKCPRPLVRQEGKPRPSEKIV

## FIGURE 1

**mRNA**

1 cgttgacccg ctttctgtcg ctgaacctgc tgctgctggg tgagtcgatt atcctgggga
61 gtggagaagc taagccacag gcacccgaac tccgaatctt tccaaagaaa atggacgccg
121 aacttggtca gaaggtggac ctggtatgtg aagtgttggg gtccgtttcg caaggatgct
181 cttggctctt ccagaactcc agctccaaac tcccccagcc caccttcgtt gtctatatgg
241 cttcatccca caacaagata acgtgggacg agaagctgaa ttcgtcgaaa ctgttttctg
301 ccatgaggga cacgaataat aagtacgttc tcaccctgaa caagttcagc aaggaaaacg
361 aaggctacta tttctgctca gtcatcagca actcggtgat gtacttcagt tctgtcgtgc
421 cagtccttca gaaagtgaac tctactacta ccaagccagt gctgcgaact ccctcacctg
481 tgcaccctac cgggacatct cagccccaga gaccagaaga ttgtcggccc cgtggctcag
541 tgaaggggac cggattggac ttcgcctgtg atatttacat ctgggcaccc ttggccggaa
601 tctgcgtggc ccttctgctg tccttgatca tcactctcat ctgctaccac aggagccgaa
661 agcgtgtttg caaatgtccc aggccgctag tcagacagga aggcaagccc agaccttcag
721 agaaaattgt gtaaaatggc accgccagga agctacaact actacatgac ttcagatctc
781 ttcttgcaag aggccaggcc ctcctttttc aagtttcctg ctgtcttatg tattgccctc
841 tgtattgttt tagtaggggt gtgatgggga cagttccttt ttctttatga attctctttg
901 acacaaagca tacttgtatg catacaatgg gagtaatgag cagactgtaa caccagagct
961 agttccagtt tcggggtcca tgtcgctggt ggcctcagca cccacttgat ataaatctcc
1021 tgtctgccca tcatatagaa gaagctgaag atcagaggtg gaaacagcag gatctgtaga
1081 cccggagaga acccaagcta gaggaaccct cactgactgg tgcagggatc tcaccccccat
1141 cccctgagct ctctgtttag gtatgtgtct ttagtatagc atgcttgtgc ctcaaactgc
1201 aaggaagcaa gtggtatgaa tctgtgatct gctcagctct ggctgtgagc atcagatgta
1261 acaaatcaaa gtcaggagaa ctgcctttag cggtgcacct ggacatcaga gccccttgta
1321 agaactggga aattgctggc agtctagtgg agcggtacgg taaatctgga aaacactccc
1381 ctctgtcact gaaggctgta gtagaatcca attaaagcta ttcaaaccac aaaaaaaaaa
1441 aaaaaaaaaa aa

**Predicted polypeptide**

MASPLTRFLSLNLLLMGESIILGSGEAKPQAPELRIFPKKMDAE

LGQKVDLVCEVLGSVSQGCSWLFQNSSSKLPQPTFVVYMASSHNKITWDEKLNSSKLF

SAVRDTNNKYVLTLNKFSKENEGYYFCSVISNSVMYFSSVVPVLQKVNSTTTKPVLRT

PSPVHPTGTSQPQRPEDCRPRGSVKGTGLDFACDIYIWAPLAGICVAPLLSLIITLIC

YHRSRKRVCKCPRPLVRQEGKPRPSEKIV

**mRNA**

# FIGURE 1

1 atggcctcac cgttgacccg ctttctgtcg ctgaacctgc tgctgatggg tgagtcgatt
61 atcctgggga gtggagaagc taagccacag gcacccgaac tccgaatctt tccaaagaaa
121 atggacgccg aacttggcca gaaggtggac ctggtatgtg aagtgttggg gtccgtttcg
181 caaggatgct cttggctctt ccagaactcc agctccaaac tcccccagcc caccttcgtt
241 gtctatatgg cttcatccca caacaagata acgtgggacg agaagctgaa ttcgtcgaaa
301 ctgttttctg ccgtgaggga cacgaataat aagtacgttc tcaccctgaa caagttcagc
361 aaggaaaacg aaggctacta tttctgctca gtcatcagca actcggtgat gtacttcagt
421 tctgtcgtgc cagtccttca gaaagtgaac tctactacta ccaagccagt gctgcgaact
481 ccctcacctg tgcaccctac cgggacatct cagccccaga gaccagaaga ttgtcggccc
541 cgtggctcag tgaaggggac cggattggac ttcgcctgtg atatttacat ctgggcaccc
601 ttggccggaa tctgcgtggc ccctctgctg tccttgatca tcactctcat ctgctaccac
661 aggagccgaa agcgtgtttg caaatgtccc aggccgctag tcagacagga aggcaagccc
721 agaccttcag agaaaattgt gtaa

## FIGURE 1

NM_031538
Rattus norvegicus (Rat)
Complete CD8 alpha mRNA

**Predicted polypeptide**

MASRVICFLSLNLLLLDVITRLQVSGQLQLSPKKVDAEIGQEVK

LTCEVLRDTSQGCSWLFRNSSSELLQPTFIIYVSSSRSKLNDILDPNLFSARKENNKY

ILTLSKFSTKNQGYYFCSITSNSVMYFSPLVPVFQKVNSIITKPVTRAPTPVPPPTGT

PRPLRPEACRPGASGSVEGMGLGFACDIYIWAPLAGICAVLLLSLVITLICCHRNRRR

VCKCPRPLVKPRPSEKFV

**mRNA**

1 ccctagagcc ctagcttgac ctaaggtgct ggtgggacgc acaccatggc ctcacgggtg
61 atctgctttc tgtcgctgaa cctgctactg ctggatgtta tcactaggct ccaggtttcc
121 ggacagttac agttgtcacc aaagaaagtg gacgctgaaa ttggccagga ggtgaagcta
181 acatgcgaag tgctgcggga cacttcgcaa ggatgctctt ggctcttccg gaactccagc
241 tccgaactcc tccagcccac cttcatcatc tatgtatctt catcccggag caagctgaac
301 gatatactgg atccgaatct gttctctgcc cggaaggaaa acaacaaata catcctcacc
361 ctgagcaagt tcagcactaa aaaccaaggc tactatttct gctcaatcac cagcaactcg
421 gtgatgtact tcagtcctct ggtgccggtg tttcagaaag tgaactctat tatcaccaag
481 ccggtgacgc gagctcccac accagtgcct cctcctacag ggacaccccg gcccctacga
541 ccagaagctt gccgacccgg ggcgagtggc tcagtggagg gaatgggatt gggcttcgcc
601 tgcgatattt acatctgggc acccttggcc ggaatctgcg cggttcttct gctgtccctg
661 gtcatcactc tcatctgctg ccacaggaac cgaaggcgtg tttgcaaatg tcccaggccc
721 cttgtcaagc ccagaccttc agagaaattc gtgtaaaatg gcgccactag gaagccacaa
781 ctactacatg acttcagaga tttctcacaa gagaccgggc cctccttttt cagagtttcc
841 tgctggctta tatattgtcc tctgtattgt tttaggggta ggatggggac agttcctttt
901 tctttatgaa ttctctttga tacaaaacat acttgtatgc acacaatggg gtaaagatca
961 gactgtaaca ccagagatag tcccagtttc agggtcagcg tagctggtgg

## FIGURE 1

AY303773
Cavia porcellus (Guinea Pig)
Complete CD8 alpha mRNA
**Predicted polypeptide**

MAPRGSAWLLLLPVALLLDAATAQGASQFRMSPRELVAQVGTKV

TLRCEVLVPNAPAGCSWLFQPRHDAKGPTFLLYHSASGTKLAPGLEQKRFSPSKSSNT

YTLTVNSFQKRDEGYYFCSVSGNMMLYFSPFVPVFLPAPRTTTPPPPPTTPTPSVQPT

SVRPETCVVSKGAAGARWLDLSCDVYIWAPLASTCAALLLALVITIICHRRNRQRVCK

CPRPQARSGGKPSPSGKLV

**mRNA**

1 gcaacttccc cactgcgcat cccctggctc ctggtggctc ctgggcggct cccttcacgc
61 ctggactcca ggctctgccc tgcgccgagg agcgcgcgcc atggccccgc gaggaagcgc
121 ctggctgctg ctgctgccgg tggccctgct gctcgacgcc gccacggccc aaggtgccag
181 tcagttccga atgtcacccc gtgaactggt cgcgcaagtc ggcaccaaag tgaccctgcg
241 ctgtgaggtg ctggtgccta acgcgccggc gggatgctcg tggctcttcc agccccgcca
301 cgacgccaaa ggtcccacct tcctcctgta ccattcggcg tccgggacca agttggcccc
361 agggctggaa cagaagcgat tcagcccctc gaagagcagt aacacctaca ccctcacggt
421 gaacagcttc cagaagcgag acgaaggcta ctacttctgc tcggtctccg gcaacatgat
481 gctctacttc agcccgttcg ttcccgtctt cctgccagct cctcgcacca cgacgccccc
541 tccccctccc accacgccga cccccagcgt gcagcccacg tcggtgcgcc ccgagacgtg
601 tgtggtctct aagggcgcag caggtgcgag gtggctggat ctctcctgtg atgtctacat
661 ctgggcgccc ctggccagca catgcgcggc ccttctgctg gcactggtca tcacgatcat
721 ctgccaccgc aggaacagac aacgcgtttg caaatgtcct aggccccaag ccaggtctgg
781 aggcaaaccc agcccttcag ggaagttagt ctaacaacat ggcgcccagc ctgtgcgaag
841 ccactacatg actttatact gagatcattc cttggacagc aagtgctcct cttttgggtt
901 tcccagtctt ccttcctatg tatttgttct cattactatt ttagtgggca tggggtggga
961 agagttgctt tttcgttaga caaaaaataa aaccatgtag catctgcagc tcacaagggt
1021 cacagggctg ttacctcaca caggggttag ggtagcaagc agggctctca ggtactggaa
1081 ttcactccct tccactcact tgagggtggg cagcacccac gggtcattta tccctcatca
1141 tgctcctcca cccacttgag ctcagatgcc acccaaagag cagtctatct aaacccaggc
1201 caaacacatg caactgcttt ttgaacccga gagcctaatt tatctgcaga gaatgcaagt
1261 gctcctttgt cacttatatc ttgtccatga cctttaataa atgtgctgct tttccctcaa
1321 aaaaaaaaa

## FIGURE 1

NM_174015
Bos taurus (Cow)
Complete CD8 alpha mRNA

**Predicted polypeptide**

MASLLTALILPLALLLLDAAKVLGSLSFRMSPTQKETRLGEKVE

LQCELLQSGMATGCSWLRHIPGDDPRPTFLMYLSAQRVKLAEGLDPRHISGAKVSGTK

FQLTLSSFLQEDQGYYFCSVVSNSILYFSNFVPVFLPAKPATTPAMRPSSAAPTSAPQ

TRSVSPRSEVCRTSAGSAVDTSRLDFACNIYIWAPLVGTCGVLLLSLVITGICYRRNR

RRVCKCPRPVVRQGGKPNLSEKYV

**mRNA**

1 gaattcggat ccaccatggc ctcactcttg accgccctga tcctgccgct ggccctgctg
61 ctgctcgatg ccgccaaggt cctcgggtcg ctctcgttcc ggatgtcgcc gacgcagaag
121 gagaccagac tgggcgagaa ggtggagctg caatgcgagt tgctgcagtc cggcatggcg
181 acagggtgct cctggctccg ccacataccc ggggacgacc ccagacccac cttcctaatg
241 tacctctccg cccaacgggt caagctagcc gagggactgg accccagaca catttccggc
301 gccaaggtct ccggcaccaa attccagctc accctgagca gcttcctcca ggaggaccaa
361 ggctactatt tttgctcggt cgtgagcaac tcgatactgt acttcagtaa cttcgtgcct
421 gtcttcttgc cagcgaagcc ggccaccacg ccggcgatgc ggccatccag cgcggcgccc
481 accagcgcgc cgcagactag gtcggtctct ccgcgatcag aggtgtgccg gacctcggcg
541 ggcagcgcag tggacacgag ccggctggac ttcgcctgca atatctacat ctgggctccc
601 ttggtcggga cctgcggcgt ccttctcctg tcattggtca tcacaggcat ctgctaccgc
661 cggaaccgaa gacgtgtctg caaatgtccc aggcctgtgg tccgacaagg aggcaagccc
721 aacctttcag agaaatatgt ctaacatggc gatgggcccc gtgtgacagc cactacaaga
781 cttcgcactg agaactctcc tgagatcctt ccctttgat ttctccctgc ttccttcctt
841 ctcgttatta ttatttttca tgggggtggg gtgggaagag ttactttttc tttattattt
901 actttgatac aaaacaagac actcgtgtct aaggcatacc acaagggtta tcatgctgtt
961 gtgctcccat actcgggtag agggcgggcg ggccagagct accgcaagct ctattctcag
1021 aacctggctg tgagaactgg tgggggcctc ggcacccact cagccccaac ttctcctcca
1081 cccattttac aaaagaggac gctgaggccc agagatgggg aacagctgga tcagagtccc
1141 agcagggctc cacacaactg agatctttct tctggaggcc tctgtctcag cgtggggagc
1201 tggatctcaa gcctcagaga actagttatt tctgaagcat ctgtgataga cccatgactg
1261 cacccagagc ctcgatgagg taatgaaata ggacaagaaa acttgacaga gttctgtgat
1321 actgctgaac aggatcagat tattttttt ataatcaagc atgaaatgat acagataata
1381 ggaattcttc caatgaagtg gaaggagtga actgaatgat ggaaaatgag caacctgacc
1441 tctgaagaaa atctctggga aatcccagcc tggagatggt tctcccagcc cttgtattgc

FIGURE 1

1501 agaaggaccc tcaaagagga gaggccaccc tctgcaagca tgatttgagc gttaggaaag
1561 ttgaatggag ttcaagtctc tctaaacatt gagattccgt attcaaacat gctcctgggt
1621 tatcggtgag tttttatagt ttgtaaaggg agaattgtga ccgagcagct ggcacaggcc
1681 ctggcacccc aggctagcag ctgagggaat gtgcagacac tggtgaggag gctacgagcc
1741 cagctgcagc cctacaaggc atttccttcc ttactgtgtt ctgcaaaaaa tgcatgctca
1801 ctgggagaaa aaatgtagct aaggtagtaa gaatcatccg taattcttta cctcagggat
1861 aatccattgt taatattatg ggctacattc ttcctgatta ttttctgtgc cctacatata
1921 aaatatataa tttttaaaaa tgggattgca ctatgctttt ataaatggct ttaataaaca
1981 aacatttatg gcttacttct t

## FIGURE 1

AY517855
Sus scrofa (Domestic pig)
Complete CD8 alpha mRNA

**Predicted polypeptide**

VELQCELMHSNTLTSCSWLYQKPGAASKPIFLMYLSKTRNKTAE

GLDTRYISGYKANDNFYLILHRFREEDQGYYFCSFLSNSVLYFSNFMSVFLPAKPTKT

PTTPPPKRTPTKASHAVSVAPEVCRPSGNADPRKLDLACDLYNWAPLVGTSGILLLSL

VITIICHRRNRRRVCKCPRPVVRQGGKASPSERFI

**mRNA**

1 gtggagctgc agtgcgagtt gatgcactcc aacacactga caagctgttc ctggctctac
61 cagaagccgg gggctgcctc caagcccatc ttcctcatgt acctctccaa aacccggaat
121 aagacagccg aggggctgga cacccgttac atctctggtt acaaggccaa tgacaacttc
181 tacctcatcc tgcaccgctt ccgcgaggag gaccaaggct actatttctg ctcgttcctg
241 agcaactcgg ttttgtattt cagcaacttc atgtccgtct tcttgccagc aaagcccacc
301 aagacgccga ctacgccacc acccaagcgg actcccacca aagcgtcgca cgccgtgtct
361 gtggccccag aggtgtgccg gccttcgggc aacgcagacc cgaggaagct ggacctcgcc
421 tgtgatctgt acaactgggc gcccctggtt gggacctccg gcatccttct cctgtcactg
481 gtcatcacca tcatctgcca ccgccggaac agaagacgtg tttgcaaatg tcccaggccc
541 gtggtcagac agggaggcaa ggccagccct tcagagagat tcatctaaca tggcgacatg
601 ccccacgcag cagccactac aagacctcaa actgagacct ctccgggcag gagagcaagg
661 gtcctttcct ttccgtttcc ccagccttcc ttccttcctt aagtattctt ctcattatta
721 ttatttccat gggggtgggg tgggaagggt gactttttct ttgggtgttt actttaattg
781 acacaaaacg agactctatc acgtctttgg tacgccgcag gggttcgaac accgttgtgc
841 tcacacacac aacggtgaag ggtgggcggg ccagagctac cgcaagctgt gttctcagaa
901 ccaggctgtg agagctggtg gggggtgggg aggccctcgg cacccacaca ggccaaacct
961 ctccccctgc cccccatttt acaaaggaat gaggctgagg cccagagatg gggggtggct
1021 ggatcagagc cccagcaagg ctccaggctc atcctccaca gcatttgggc ctctcttcca
1081 ggggcctctg tctcagctgg gggagctgtg tctcccacct caaggaaaca aggtttgctt
1141 gggcacctgt gatagactct gcactgtgcc cagagccccg gggaggcaat gcagtaagtc
1201 aaggggacgt gacagaggtc tacggtgcag ttgaacagga tcagatatat ttttttaat
1261 aatccagcat gaagttatat agataacagg aattcctcaa atagagtgga agggctgaac
1321 tgaatcctgg aaagtgaaca acacgacctc taaaggaaat ccaatgcaaa aaatctctaa
1381 gtggagacac agtggctctc ccaggggacc catgaaagag gggaagccgc cctttgcaaa
1441 tatgatttga gcatcgcgaa agtcgaacgg aggtcggccc tctctaaatg tgagatctga
1501 tatttgaacg tgctcctcgg atcattgatg ggttttttg gtttgtaaac acagaattat
1561 gaccgagtag ctggcctccc ctggaccagc agctgtggat atggggcaga ctctgatgag

**FIGURE 1**

1621 gaggctagga gcccagactg ctgccctcta cgcgcatttc ctctcttaac catgttgtac

1681 aagaaatgcg tgctcgctgg aagaaaaaac taaataataa gagtcaccca taattcttta

1741 cttctggtat aactcattgt taatattatg gtgtacattc ttcctgatta ttttctatgc

1801 acgtatataa aatgtatact ttttaaaaat ggaattgtac tatgctttta gaagtggttt

1861 taataaacat ttctgctatg aaaaaaaaaa a

## FIGURE 1

D16536
Felis catus (cat)
Complete CD8 alpha mRNA
***Predicted polypeptide***

MASPVTAQLLPLALLLHAAAAAGPSPFRLSPVRVEGRLGQRVEL

QCEVLLSSAAPGCTWLFQKNEPAARPIFLAYLSRSRTKLAEELDPKQISGQRIQDTLY

SLTLHRFRKEEEGYYFCSVVSNSVLYFSAFVPVFLPVKPTTTPAPRPPTQAPITTSQR

VSLRPGTCQPSAGSTVEASGLDLSCDIYIWAPLAGTCAFLLLSLVITVICNHRNRRRV

CKCPRPVVRAGGKPSPSERYV

**mRNA**

1 atggcctctc cggtgactgc ccagctcctg ccgctggcct tgctgcttca tgccgccgca
61 gccgccgggc cgagcccgtt ccgcttatcg cccgtgaggg tggagggcag gctcggccag
121 cgggtggagc tgcagtgcga ggtgctgctg tccagcgcgg cgccgggctg cacctggctc
181 ttccagaaga acgaacctgc cgcccgcccc atcttcctgg cgtacctctc cagaagccgg
241 accaagttgg ccgaggagct ggaccccaaa cagatctcgg gccagaggat tcaggacacc
301 ctctacagtc tcaccctgca cagattccgc aaggaggaag aaggctacta tttctgctcg
361 gtcgtgagca actccgttct gtacttcagc gccttcgtcc cggtcttcct gccagtcaag
421 cccaccacta cgcccgcgcc gcgaccgccc acgcaggcgc ccatcaccac gtcgcagcgg
481 gtgtctctgc gcccggggac ctgccagcct tcagcgggca gcacagtgga agcaagtggg
541 ctggatttgt cctgtgacat ctacatctgg gcacccctgg ctgggacctg cgccttcctt
601 ctcctgtcgc tggtcatcac cgtcatctgc aaccacagga accgaagacg tgtttgcaaa
661 tgtccgaggc ccgtggtcag agcaggaggc aagcctagcc cgtcagagag atacgtctaa
721 catggagatg ggccccatgc accagccact acaagaccaa ataaaactct ctttatgagg
781 acagt

# FIGURE 1

AY065643
Sigmodon hispidus (Hispid cotton rat)
Complete CD8 alpha mRNA
**Predicted polypeptide**

MAPRVTRFLCLTLLLEFIAELGGSKDFEMSPKKVVAHLGKEVRL

TCEVWVSTSQGCSWLFLEHGSGVKPTFLIYLSGSRNERNNKIPSTKLSGKKEDKKYTL

TLNNFAKEDEGYYFCSVTSNSVVYFSPLVSVFLPEKPTTPVPKPPTSVPTTAISRSLR

PEACRPGAGTSVEKKGWDFDCDIIILAPLAGLCGVLLLSLVTTLICCHRNRKRVCKCP

RPVVRQGGKPSPSGKLV

**mRNA**

1 ctcctgcttg acctaagctg ctggtggaag cactgccatg gccccccggg tgacccgctt
61 tctgtgcctg accctgctgc tggaatttat cgctgagctc ggaggctcga aagatttcga
121 aatgtctcct aagaaggtgg tcgcccacct tggcaaggag gtgaggctaa catgcgaagt
181 gtgggtgtct acttcgcaag gatgctcttg gctcttcctg gagcatggct ccggagttaa
241 acccactttc ctcatctatc tctctgggag ccgcaacgaa cggaataaca aaataccttc
301 aactaagcta tctgggaaga aggaagacaa aaagtacacc ctcaccctga ataattttgc
361 taaggaagac gaaggctact atttctgctc tgtcacaagc aactcggtgg tgtacttcag
421 tcctctcgtg tcggtctttc tgccagagaa acctaccaca ccagtgccga aaccacccac
481 atcagtgccc actacggcga tatctcggtc cctgcgacca gaagcttgcc gacctggagc
541 cggcacctca gtggagaaga agggatggga cttcgactgt gatatcatca ttttggcacc
601 cttagctgga ctctgtgggg tccttctgct gtctctggtc accacactca tctgctgcca
661 caggaacaga aaacgagtct gcaaatgtcc caggcccgtg gtcagacaag gaggcaagcc
721 cagcccttca gggaaactcg tgtaagatgg cgccaagaaa ctacaactac tacttcagag
781 acctcttcat ctagagctcc agctctcctt cttcaatttt tctcaccttc ctatatattg
841 ttctttgtat tattttagtg ggggtaggac agggttggaa ccatttcctt tctttatgaa
901 ttcactttga cacaaaacaa gaccacataa tgtccacggg ataccataag ggcaggagct
961 gttgctgcgt acatagcatg tgggggaagt acagaacagc tgtctgggtt ctcaggatca
1021 gtggatgatc agcacccact tgatgatcta aatgccctgt ctgcccatta tatagaagag
1081 gttgaaggtc agaaatgggg tgggcaggat ctgtgcacca ggagagaacc caagctgacg
1141 aaatcctcac tggatggctc agggaacttg cctctatatc ctgagttctc tttattcagg
1201 cctgtgcctg gtagtgtgta ggctgagta

## FIGURE 1

AJ130818
Saimiri sciureus (Common Squirrel Monkey)
Complete CD8 alpha mRNA

**Predicted polypeptide**

MASPVTALLLPLALLLHAARPSRFRVSPLDRTWNLGDKVELKCE

VLLSNPSSGCSWLFQKRGAAASPTFLLYISQTKPKVADGLDAQRFSGKKMGDSFILTL

RDFREEDQGFYFCSALSNSIMYFSPFVPVFLPAKPTTTPAPRPPTPEPTTASQPLSLR

PQACRPPAGGAVDTRGLDFACDIYIWVPLAGTCGVLLLSLVITVYCNHRNRRRVCKCP

RPAVKSGGKPSPSERYV

**mRNA**

1 atggcctctc ccgtgaccgc cttgctcctg ccgctggccc tgctgctcca cgctgccagg
61 ccgagccggt tccgggtgtc gccgctggat cggacctgga acttgggcga caaggtggag
121 ctgaagtgcg aggtgctgct gtccaacccg tcctcgggct gctcgtggct cttccagaag
181 cgcggcgctg ccgccagccc caccttcctc ctgtacatct cccaaaccaa gcccaaggtg
241 gccgatgggc tggacgccca gcgcttctcc ggcaagaaga tgggggacag cttcattctc
301 accctgcgcg acttccgcga ggaggaccag ggcttctatt tctgctcggc cctgagcaac
361 tccatcatgt acttcagccc cttcgtgccg gtcttcctgc cagcgaagcc caccacgacg
421 ccagcgccgc gaccacccac accggagccc accaccgcgt cgcagcccct gtccctgcgt
481 ccacaggctt gccggccccc ggcgggggc gcagtggaca cgaggggct ggacttcgcc
541 tgtgatatct acatctgggt gcccttggcc gggacctgcg gggtccttct cctgtcactg
601 gtcatcaccg tttattgcaa tcacaggaac cgacgacgtg tttgcaaatg tccccggcct
661 gcggtcaagt ctggaggcaa gcccagccct tcggagagat acgtctaa

**Domains of the CD8 α-Chains**

**Leader**
Transmembrane

**Human CD8 α-Chain**

Protein:

**MALPVTALLL PLALLLHAAR P**SQFRVSPLD RTWNLGETVE LKCQVLLSNP
TSGCSWLFQP RGAAASPTFL LYLSQNKPKA AEGLDTQRFS GKRLGDTFVL
TLSDFRRENE GYYFCSALSN SIMYFSHFVP VFLPAKPTTT PAPRPPTPAP
TIASQPLSLR PEACRPAAGG AVHTRGLDFA CDIYIWAPLA GTCGVLLLSL
VITLYCNHRN RRRVCKCPRP VVKSGDKPSL SARYV

mRNA - coding

**atggccttac cagtgaccgc cttgctcctg ccgctggcct tgctgctcca**
**cgccgccagg ccg**agccagt tccgggtgtc gccgctggat cggacctgga
acctgggcga gacagtggag ctgaagtgcc aggtgctgct gtccaacccg
acgtcgggct gctcgtggct cttccagccg cgcggcgccg ccgccagtcc
caccttcctc ctatacctct cccaaaacaa gcccaaggcg gccgaggggc
tggacaccca gcggttctcg ggcaagaggt tgggggacac cttcgtcctc
accctgagcg acttccgccg agagaacgag ggctactatt tctgctcggc
cctgagcaac tccatcatgt acttcagcca cttcgtgccg gtcttcctgc
cagcgaagcc caccacgacg ccagcgccgc gaccaccaac accggcgccc
accatcgcgt cgcagcccct gtccctgcgc ccagaggcgt gccggccagc
ggcgggggc gcagtgcaca cgagggggct ggacttcgcc tgtgatatct
acatctgggc gcccttggcc gggacttgtg gggtccttct cctgtcactg
gttatcaccc tttactgcaa ccacaggaac cgaagacgtg tttgcaaatg
tccccggcct gtggtcaaat cgggagacaa gcccagcctt tcggcgagat
acgtctaa

**Figure 2A**

**mouse CD8 α-Chain**

Protein:

**MASPLTRFLS LNLLLLGESI ILGSGEA**KPQ APELRIFPKK MDAELGQKVD
LVCEVLGSVS QGCSWLFQNS SSKLPQPTFV VYMASSHNKI TWDEKLNSSK
LFSAMRDTNN KYVLTLNKFS KENEGYYFCS VISNSVMYFS SVVPVLQKVN
STTTKPVLRT PSPVHPTGTS QPQRPEDCRP RGSVKGTGLD FACDIYIWAP
LAGICVALLL SLIITLICYH RSRKRVCKCP SIACLCLKLQ GSKWYESVIC
SALAVSIRCN KSKSGELPLA VHLDIRAPCK NWEIAGSLVE RYGKSGKHSP
LSLKAVVESN

mRNA - Coding

**atggcctcac cgttgacccg ctttctgtcg ctgaacctgc tgctgctggg**
**tgagtcgatt atcctgggga gtggagaagc t**aagccacag gcacccgaac
tccgaatctt tccaaagaaa atggacgccg aacttggtca gaaggtggac
ctggtatgtg aagtgttggg gtccgtttcg caaggatgct cttggctctt
ccagaactcc agctccaaac tcccccagcc caccttcgtt gtctatatgg
cttcatccca caacaagata acgtgggacg agaagctgaa ttcgtcgaaa
ctgttttctg ccatgaggga cacgaataat aagtacgttc tcaccctgaa
caagttcagc aaggaaaacg aaggctacta tttctgctca gtcatcagca
actcggtgat gtacttcagt tctgtcgtgc cagtccttca gaaagtgaac
tctactacta ccaagccagt gctgcgaact ccctcacctg tgcaccctac
cgggacatct cagccccaga gaccagaaga ttgtcggccc cgtggctcag
tgaaggggac cggattggac ttcgcctgtg atatttacat ctgggcaccc
ttggccggaa tctgcgtggc ccttctgctg tccttgatca tcactctcat
ctgctaccac aggagccgaa agcgtgtttg caaatgtccc agtatagcat
gcttgtgcct caaactgcaa ggaagcaagt ggtatgaatc tgtgatctgc
tcagctctgg ctgtgagcat cagatgtaac aaatcaaagt caggagaact
gcctttagcg gtgcacctgg acatcagagc cccttgtaag aactgggaaa
ttgctggcag tctagtggag cggtacggta aatctggaaa acactcccct
ctgtcactga aggctgtagt agaatccaat taa

**Figure 2B**

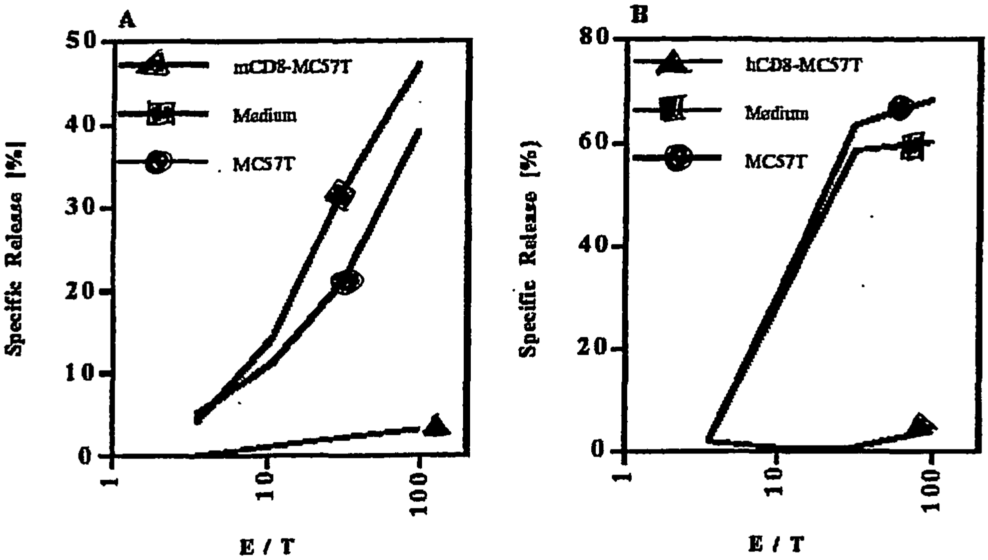


Balb/c spleen cells were stimulated with C57BL/6 spleen cells. Cultures were supplemented with normal fibroblasts ~~(blue)~~ circle, medium ~~(red)~~ square or fibroblasts with CD8 ~~(green)~~ triangle of mouse (A) or human (B) origin. Cultures were harvested and tested for their lytic ability towards C57BL/6-derived target cells.

**Figure 3**

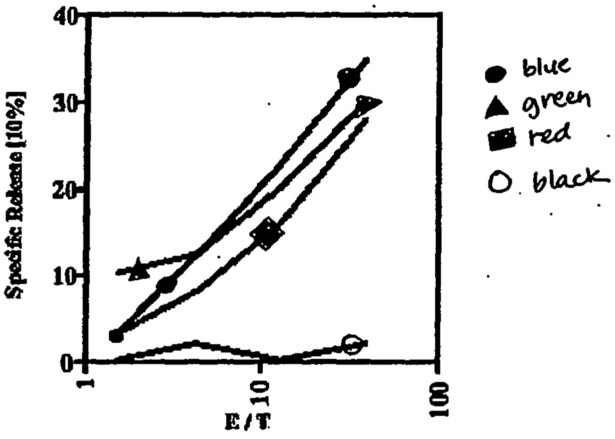


Balb/c (H-2d) mice were injected with control fibroblasts(red and green) or mCD8-transfected C57Bl/6-(H-2b) derived (black and blue) fibroblasts. After two weeks animals were sacrifized, spleen cells were harvested, stimulated with C57BL/6 (H-2b) (red and black) or CBA/J (H-2k) (blueand green) spleen cells and tested for their lytic ability on EL4 (H-2b) (red and black) or S.AKR (H-2k) (blue and green) target cells.

**Figure 4**

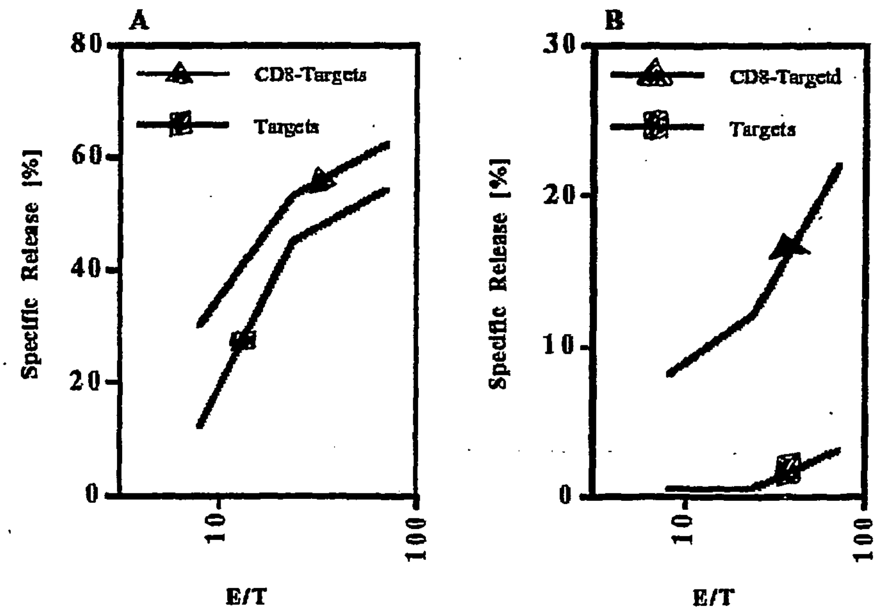


. Target cells (~~green~~ triangle) or CD8-expressing targets (~~red~~ square) were tested for their susceptibility to lysis by alloreactive T cells (A) or by antigen-specific CTLs (B).

**Figure 5**

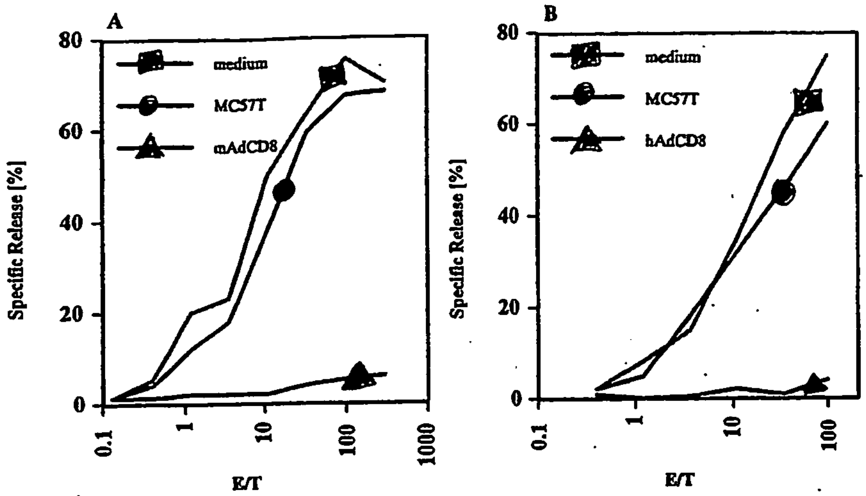


circle MLCs (Balb/c anti-C57BL/6) were set up in the presence of normal fibroblasts (blue) and fibroblasts transduced with mAdCD8 (A, green) or hAdCD8 (B, green). No fibroblasts were added to control cultures (red). square The lytic activity of these cultures triangle towards an C57BL/6-derived target was determined at the end of the culture period.

**Figure 6**

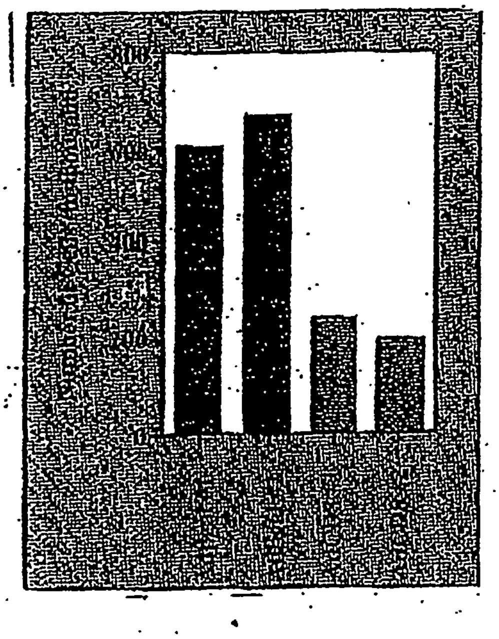

Figure 7

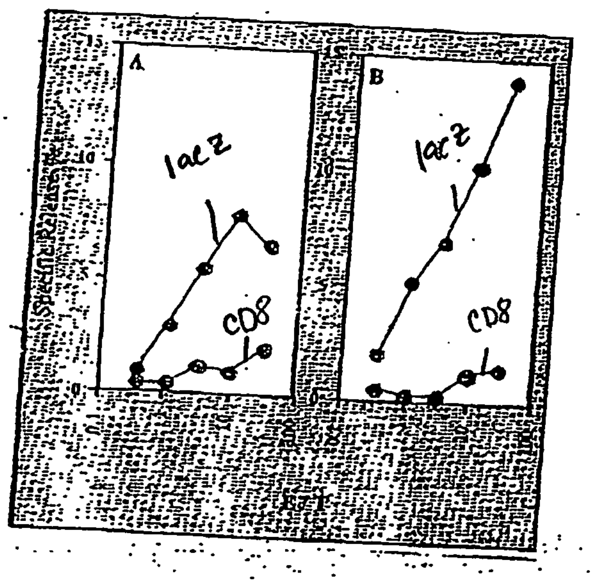


FIGURE 8

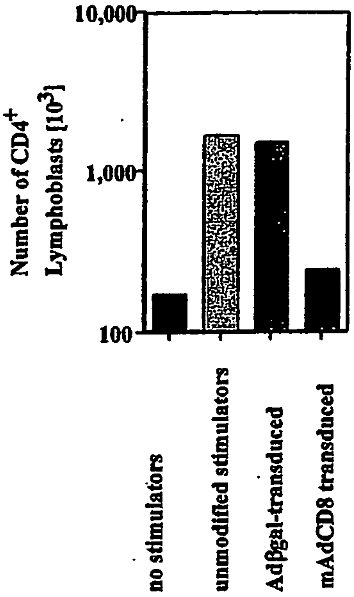


3x10⁶ C7Bl/6 spleen cells were incubated with 1x10⁶ (or no) stimulator cells, transduced as indicated. After 4 days the cultures were analyzed for presence CD4⁺ T lymphoblasts by immunofluorescence.

**Figure 9**

## FIGURE 10A

Infected Cells: MC57T Fibroblasts
Panel 1: Mock-Infection; Panel 2: Infection with hAdCD8

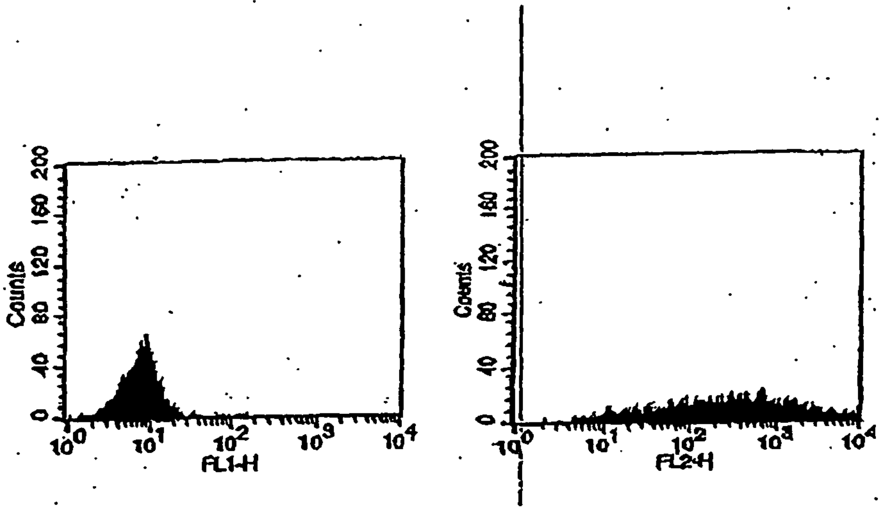


## FIGURE 10B

Infected Cells: MC57T Fibroblasts
Panel 1: Mock-Infection; Panel 2: Infection with mAdCD8

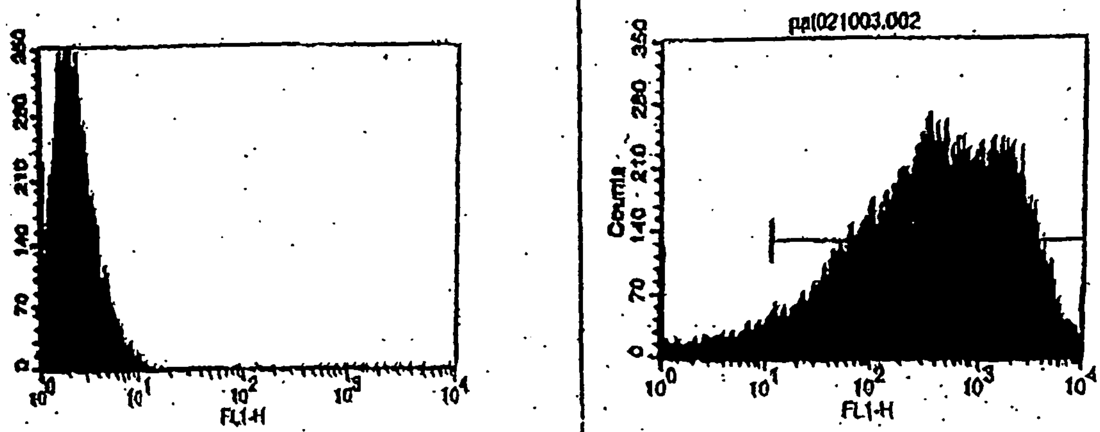

## FIGURE 10C

Infected Cells: Balbc unselected bone marrow cells:
Panel 1: Infection with lacZ Adenoviral Vector (AdLacZ);
Panel 2: Infection with mAdCD8

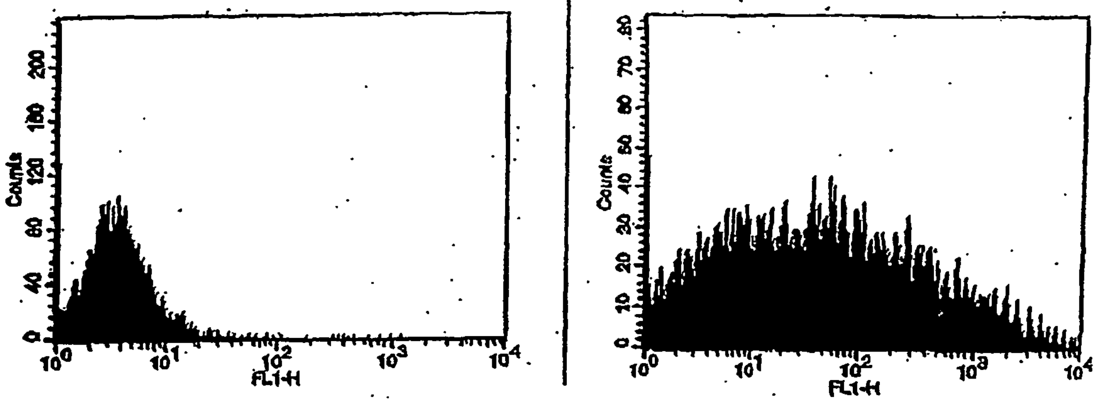


## FIGURE 10D

Infected Cells: MC57T Fibroblasts
Panel 1: Mock-Infection;
Panel 2: Infection with pAAV-mCD8;
Panel 3: Infection with pAAV-hCD8

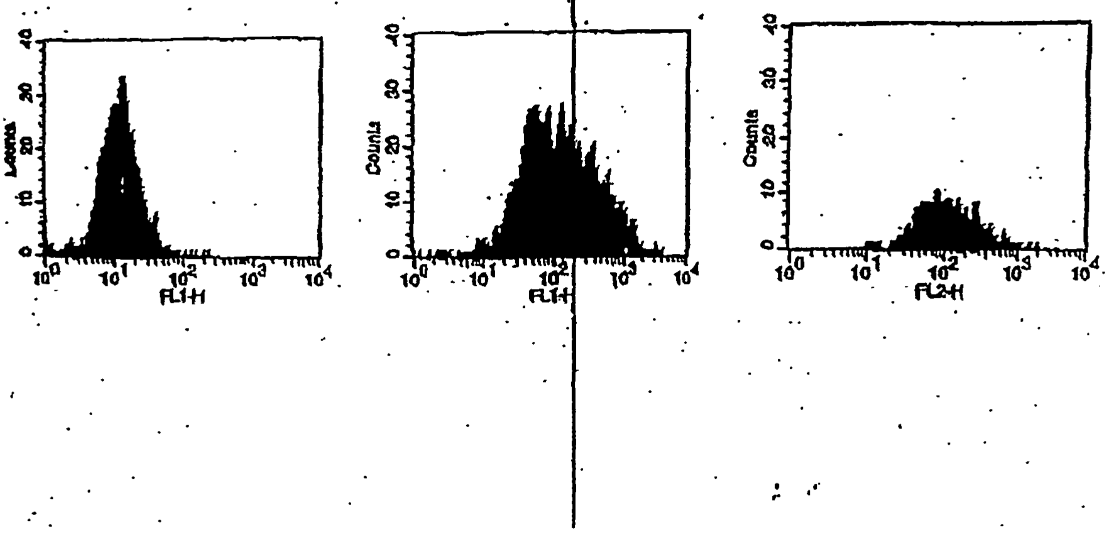

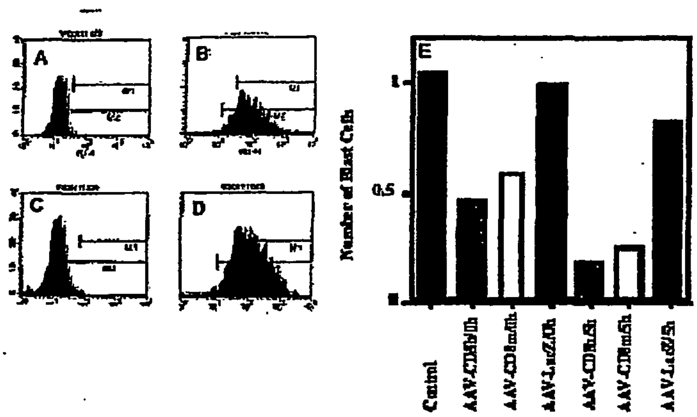


Fibroblasts were transduced with mAAVCD8 (B) or hAAVCD8 (D) or mock-infected (A and C). Surface expression of CD8 was detected by surface immunofluorescence (A through D). MLCs (Balb/c anti-C57BL/6) were set up in the presence of these fibroblasts that had been cultured for 0 or 5 hours after transduction before they were added to the MLCs. At end of cultures, the number of lymphoblasts was determined on a fluorescence activated cell analyzer.

**Figure 11**

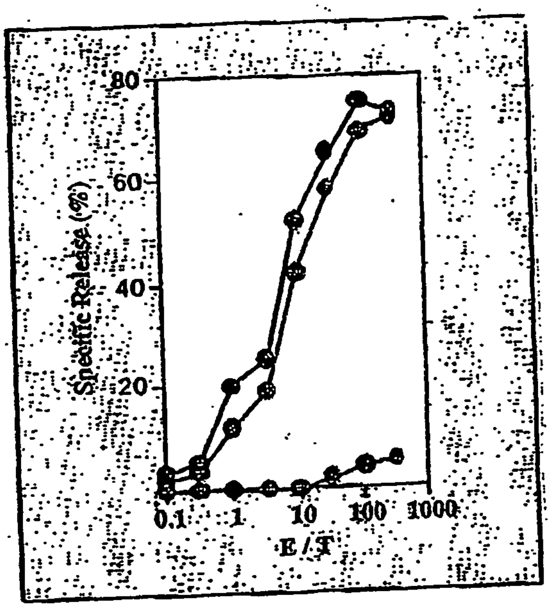


FIGURE 12

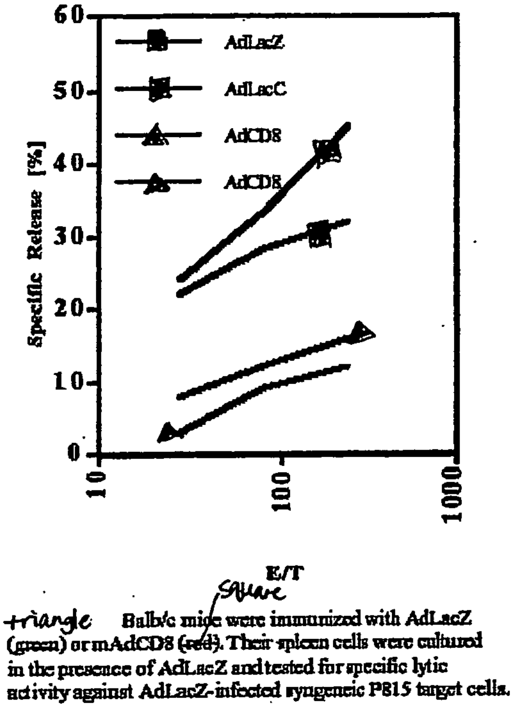


square
triangle Balb/c mice were immunized with AdLacZ (green) or mAdCD8 (red). Their spleen cells were cultured in the presence of AdLacZ and tested for specific lytic activity against AdLacZ-infected syngeneic P815 target cells.

**Figure 13**

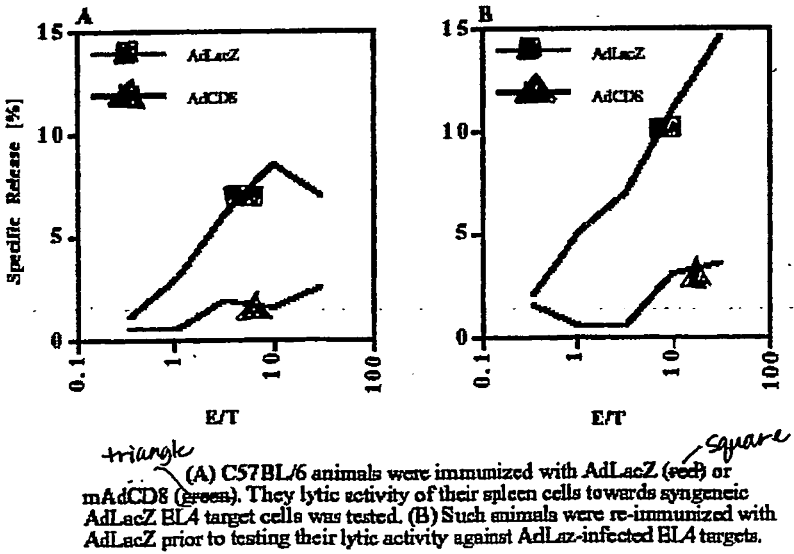


triangle square
(A) C57BL/6 animals were immunized with AdLacZ (red) or mAdCD8 (green). They lytic activity of their spleen cells towards syngeneic AdLacZ EL4 target cells was tested. (B) Such animals were re-immunized with AdLacZ prior to testing their lytic activity against AdLaz-infected EL4 targets.

**Figure 14A-B**

**Figure 15**

**Hemoglobin β**

mRNA

1 acatttgctt ctgacacaac tgtgttcact agcaacctca aacagacacc atggtgcatc

61 tgactcctga ggagaagtct gccgttactg ccctgtgggg caaggtgaac gtggatgaag

121 ttggtggtga ggccctgggc aggctgctgg tggtctaccc ttggacccag aggttctttg

181 agtcctttgg ggatctgtcc actcctgatg ctgttatggg caaccctaag gtgaaggctc

241 atggcaagaa agtgctcggt gcctttagtg atggcctggc tcacctggac aacctcaagg

301 gcacctttgc cacactgagt gagctgcact gtgacaagct gcacgtggat cctgagaact

361 tcaggctcct gggcaacgtg ctggtctgtg tgctggccca tcactttggc aaagaattca

421 ccccaccagt gcaggctgcc tatcagaaag tggtggctgg tgtggctaat gccctggccc

481 acaagtatca ctaagctcgc tttcttgctg tccaatttct attaaaggtt cctttgttcc

541 ctaagtccaa ctactaaact gggggatatt atgaagggcc ttgagcatct ggattctgcc

601 taataaaaaa catttatttt cattgc

## Figure 16

### GATA-binding protein

mRNA

1 gcaaaggcca aggccagcca ggacaccccc tgggatcaca ctgagcttgc cacatcccca

61 aggcggccga accctccgca accaccagcc caggttaatc cccagaggct ccatggagtt

121 ccctggcctg gggtccctgg ggacctcaga gcccctcccc cagtttgtgg atcctgctct

181 ggtgtcctcc acaccagaat caggggtttt cttcccctct gggcctgagg gcttggatgc

241 agcagcttcc tccactgccc cgagcacagc caccgctgca gctgcggcac tggcctacta

301 cagggacgct gaggcctaca gacactcccc agtctttcag gtgtacccat tgctcaactg

361 tatggagggg atcccagggg gctcaccata tgccggctgg gcctacggca agacggggct

421 ctaccctgcc tcaactgtgt gtcccacccg cgaggactct cctccccagg ccgtggaaga

481 tctggatgga aaaggcagca ccagcttcct ggagactttg aagacagagc ggctgagccc

541 agacctcctg accctgggac ctgcactgcc ttcatcactc cctgtcccca atagtgctta

601 tgggggccct gacttttcca gtaccttctt ttctcccacc gggagccccc tcaattcagc

661 agcctattcc tctcccaagc ttcgtggaac tctcccccctg cctccctgtg aggccaggga

721 gtgtgtgaac tgcggagcaa cagccactcc actgtggcgg agggacagga caggccacta

781 cctatgcaac gcctgcggcc tctatcacaa gatgaatggg cagaacaggc ccctcatccg

841 gcccaagaag cgcctgattg tcagtaaacg ggcaggtact cagtgcacca actgccagac

901 gaccaccacg acactgtggc ggagaaatgc cagtggggat cccgtgtgca atgcctgcgg

961 cctctactac aagctacacc aggtgaaccg gccactgacc atgcggaagg atggtattca

1021 gactcgaaac cgcaaggcat ctggaaaagg gaaaaagaaa cggggctcca gtctgggagg

1081 cacaggagca gccgaaggac cagctggtgg ctttatggtg gtggctgggg gcagcggtag

1141 cgggaattgt ggggaggtgg cttcaggcct gacactgggc cccccaggta ctgcccatct

1201 ctaccaaggc ctgggccctg tggtgctgtc agggcctgtt agccacctca tgcctttccc

1261 tggaccccta ctgggctcac ccacgggctc cttccccaca ggccccatgc cccccaccac

1321 cagcactact gtggtggctc cgctcagctc atgagggcac agagcatggc ctccagagga

1381 ggggtggtgt ccttctcctc ttgtagccag aattctggac aacccaagtc tctgggcccc

1441 aggcaccccc tggcttgaac cttcaaagct tttgtaaaat aaaaccacca aagtcctgaa

1501 aaaaaaaaaa aaaaaaaaaa aa

## FIGURE 17

**d-aminoevulinate synthase**

mRNA

1 cacctgtcat tcgttcgtcc tcagtgcagg gcaacaggac tttaggttca agatggtgac

61 tgcagccatg ctgctacagt gctgcccagt gcttgcccgg ggccccacaa gcctcctagg

121 caaggtggtt aagactcacc agttcctgtt tggtattgga cgctgtccca tcctggctac

181 ccaaggacca aactgttctc aaatccacct taaggcaaca aaggctggag gagattctcc

241 atcttgggcg aagggccact gtcccttcat gctgtcggaa ctccaggatg ggaagagcaa

301 gattgtgcag aaggcagccc cagaagtcca ggaagatgtg aaggctttca agacagatct

361 gcctagctcc ctggtctcag tcagcctaag gaagccattt tccggtcccc aggagcagga

421 gcagatctct gggaaggtca cacacctgat tcagaacaat atgcctggaa actatgtctt

481 cagttatgac cagtttttca gggacaagat catggagaag aaacaggatc acacctaccg

541 tgtgttcaag actgtgaacc gctgggctga tgcatatccc tttgcccaac atttctttga

601 ggcatctgtg gcctcaaagg atgtgtccgt ctggtgtagt aatgattacc tgggcatgag

661 ccgacaccct caggtcttgc aagccacaca ggagaccctg cagcgtcatg gtgctggagc

721 tggtggcacc cgcaacatct caggcaccag taagtttcat gtggagcttg agcaggagct

781 ggctgagctg caccagaagg actcagccct gctcttctcc tcctgctttg ttgccaatga

841 ctctactctc ttcaccttgg ccaagatcct gccagggtgc gagatttact cagacgcagg

901 caaccatgct tccatgatcc aaggtatccg taacagtgga gcagccaagt ttgtcttcag

961 gcacaatgac cctgaccacc taaagaaact tctagagaag tctaacccta agatacccaa

1021 aattgtggcc tttgagactg tccactccat ggatggtgcc atctgtcccc tcgaggagtt

1081 gtgtgatgtg tcccaccagt atggggccct gaccttcgtg gatgaggtcc atgctgtagg

1141 actgtatggg tcccggggcg ctgggattgg ggagcgtgat ggaattatgc ataagattga

1201 catcatctct ggaactcttg gcaaggcctt tggctgtgtg ggcggctaca ttgccagcac

1261 ccgtgacttg gtggacatgg tgcgctccta tgctgcaggc ttcatcttta ccacttctct

1321 gcccccccatg gtgctctctg gagctctaga atctgtgcgg ctgctcaagg gagaggaggg

1381 ccaagccctg aggcgagccc accagcgcaa tgtcaagcac atgcgccagc tactcatgga

1441 caggggcctt cctgtcatcc cctgccccag ccacatcatc cccatccggg tgggcaatgc

1501 agcactcaac agcaagctct gtgatctcct gctctccaag catggcatct atgtgcaggc

1561 catcaactac ccaactgtcc cccggggtga agagctcctg cgcttggcac cctcccccca

1621 ccacagccct cagatgatgg aagattttgt ggagaagctg ctgctggctt ggactgcggt

1681 ggggctgccc ctccaggatg tgtctgtggc tgcctgcaat ttctgtcgcc gtcctgtaca

1741 ctttgagctc atgagtgagt gggaacgttc ctacttcggg aacatggggc cccagtatgt

1801 caccacctat gcctgagaag ccagctgcct aggattcaca ccccacctgc gcttcacttg

1861 ggtccaggcc tactcctgtc ttctgctttg ttgtgtgcct ctagctgaat tgagcctaaa

1921 aataaagcac aaaccac

## Figure 18

### Glucose-6-phosphate-dehydrogenase

mRNA

1 agggacagcc cagaggaggc gtggccacgc tgccggcgga agtggagccc tccgcgagcg

61 cgcgaggccg ccggggcagg cggggaaacc ggacagtagg ggcggggccg ggccggcgat

121 ggggatgcgg gagcactacg cggagctgca cccgtgcccg ccggaattgg ggatgcagag

181 cagcggcagc gggtatggca ggcagccggc gggccggcct ccagcgcagg tgcccgagag

241 gcaggggctg gcctgggatg cgcgcgcacc tgccctcgcc ccgccccgcc cgcacgaggg

301 gtggtggccg aggccccgcc ccgcacgcct cgcctgaggc gggtccgctc agcccaggcg

361 cccgccccg cccccgccga ttaaatgggc cggcggggct cagcccccgg aaacggtcgt

421 aacttcgggg ctgcgagcgc ggagggcgac gacgacgaag cgcagacagc gtcatggcag

481 agcaggtggc cctgagccgg acccaggtgt gcgggatcct gcgggaagag cttttccagg

541 gcgatgcctt ccatcagtcg gatacacaca tattcatcat catgggtgca tcgggtgacc

601 tggccaagaa gaagatctac cccaccatct ggtggctgtt ccgggatggc cttctgcccg

661 aaaacacctt catcgtgggc tatgcccgtt cccgcctcac agtggctgac atccgcaaac

721 agagtgagcc cttcttcaag gccaccccag aggagaagct caagctggag gacttctttg

781 cccgcaactc ctatgtggct ggccagtacg atgatgcagc ctcctaccag cgcctcaaca

841 gccacatgga tgccctccac ctggggtcac aggccaaccg cctcttctac ctggccttgc

901 ccccgaccgt ctacgaggcc gtcaccaaga acattcacga gtcctgcatg agccagatag

961 gctggaaccg catcatcgtg gagaagccct tcgggaggga cctgcagagc tctgaccggc

1021 tgtccaacca catctcctcc ctgttccgtg aggaccagat ctaccgcatc gaccactacc

1081 tgggcaagga gatggtgcag aacctcatgg tgctgagatt tgccaacagg atcttcggcc

1141 ccatctggaa ccgggacaac atcgcctgcg ttatcctcac cttcaaggag ccctttggca

1201 ctgagggtcg cgggggctat ttcgatgaat ttgggatcat ccgggacgtg atgcagaacc

1261 acctactgca gatgctgtgt ctggtggcca tggagaagcc cgcctccacc aactcagatg

1321 acgtccgtga tgagaaggtc aaggtgttga aatgcatctc agaggtgcag gccaacaatg

1381 tggtcctggg ccagtacgtg gggaaccccg atggagaggg cgaggccacc aaagggtacc

1441 tggacgaccc cacggtgccc cgcgggtcca ccaccgccac ttttgcagcc gtcgtcctct

1501 atgtggagaa tgagaggtgg gatggggtgc ccttcatcct gcgctgcggc aaggccctga

1561 acgagcgcaa ggccgaggtg aggctgcagt tccatgatgt ggccggcgac atcttccacc

1621 agcagtgcaa gcgcaacgag ctggtgatcc gcgtgcagcc caacgaggcc gtgtacacca

1681 agatgatgac caagaagccg ggcatgttct tcaaccccga ggagtcggag ctggacctga

1741 cctacggcaa cagatacaag aacgtgaagc tccctgacgc ctacgagcgc ctcatcctgg

1801 acgtcttctg cgggagccag atgcacttcg tgcgcagcga cgagctccgt gaggcctggc

1861 gtattttcac cccactgctg caccagattg agctggagaa gcccaagccc atcccctata

1921 tttatggcag ccgaggcccc acggaggcag acgagctgat gaagagagtg ggtttccagt

1981 atgagggcac ctacaagtgg gtgaaccccc acaagctctg agccctgggc acccacctcc

2041 acccccgcca cggccaccct ccttcccgcc gcccgacccc gagtcgggag gactccggga

2101 ccattgacct cagctgcaca ttcctggccc cgggctctgg ccaccctggc ccgcccctcg

2161 ctgctgctac tacccgagcc cagctacatt cctcagctgc caagcactcg agaccatcct

2221 ggcccctcca gaccctgcct gagcccagga gctgagtcac ctcctccact cactccagcc

2281 caacagaagg aaggaggagg gcgcccattc gtctgtccca gagcttattg gccactgggt

2341 ctcactcctg agtggggcca gggtgggagg gagggacaag gggaggaaa ggggcgagca

2401 cccacgtgag agaatctgcc tgtggccttg cccgccagcc tcagtgccac ttgacattcc

2461 ttgtcaccag caacatctcg agccccctgg atgtcccctg tcccaccaac tctgcactcc

2521 atggccaccc cgtgccaccc gtaggcagcc tctctgctat aagaaaagca gacgcagcag

2581 ctgggacccc tcccaacctc aatgccctgc cattaaatcc gcaaacagcc aaaaaaaaa

2641 aaaaaaaaa

## Figure 19

**Ornithine carbamoyl transferase**

mRNA

1 gagccccagg actgagatat ttttactata ccttctctat catcttgcac ccccaaaata

61 gcttccaggg cacttctatt tgtttttgtg gaaagactgg caattagagg tagaaaagtg

121 aaataaatgg aaatagtact actcagggct gtcacatcta catctgtgtt tttgcagtgc

181 caatttgcat tttctgagtg agttacttct actcaccttc acagcagcca gtaccgcagt

241 gccttgcata tattatatcc tcaatgagta cttgtcaatt gattttgtac atgcgtgtga

301 cagtataaat atattatgaa aaatgaggag gccaggcaat aaaagagtca ggatttcttc

361 caaaaaaaat acacagcggt ggagcttggc ataaagttca aatgctccta caccctgccc

421 tgcagtatct ctaaccaggg gactttgata aggaagctga agggtgatat tacctttgct

481 ccctcactgc aactgaacac atttcttagt ttttaggtgg cccccgctgg ctaacttgct

541 gtggagtttt caagggcata gaatcgtcct ttacacaatt aaaagaagat gctgtttaat

601 ctgaggatcc tgttaaacaa tgcagctttt agaaatggtc acaacttcat ggttcgaaat

661 tttcggtgtg gacaaccact acaaaataaa gtgcagctga agggccgtga ccttctcact

721 ctaaaaaact ttaccggaga agaaattaaa tatatgctat ggctatcagc agatctgaaa

781 tttaggataa aacagaaagg agagtatttg cctttattgc aagggaagtc cttaggcatg

841 atttttgaga aaagaagtac tcgaacaaga ttgtctacag aaacaggctt tgcacttctg

901 ggaggacatc cttgttttct taccacacaa gatattcatt tgggtgtgaa tgaaagtctc

961 acggacacgg cccgtgtatt gtctagcatg gcagatgcag tattggctcg agtgtataaa

1021 caatcagatt tggacaccct tgctaaagaa gcatccatcc caattatcaa tgggctgtca

1081 gatttgtacc atcctatcca gatcctggct gattacctca cgctccagga acactatagc

1141 tctctgaaag gtcttaccct cagctggatc ggggatggga acaatatcct gcactccatc

1201 atgatgagcg cagcgaaatt cggaatgcac cttcaggcag ctactccaaa gggttatgag

1261 ccggatgcta gtgtaaccaa gttggcagag cagtatgcca aagagaatgg taccaagctg

1321 ttgctgacaa atgatccatt ggaagcagcg catggaggca atgtattaat tacagacact

1381 tggataagca tgggacaaga agaggagaag aaaaagcggc tccaggcttt ccaaggttac

1441 caggttacaa tgaagactgc taaagttgct gcctctgact ggacattttt acactgcttg

1501 cccagaaagc cagaagaagt ggatgatgaa gtcttttatt ctcctcgatc actagtgttc

1561 ccagaggcag aaaacagaaa gtggacaatc atggctgtca tggtgtccct gctgacagat

1621 tactcacctc agctccagaa gcctaaattt tgatgttgtg ttacttgtca agaaagaagc

1681 aatgttcttc agtaacagaa tgagttggtt tatggggaaa agagaagaga atctaaaaaa

1741 taaacaaatc cctaacacgt ggtatgggtg aaccgtatga tatgctttgc cattgtgaaa

1801 ctttccttaa gcctttaatt taagtgctga tgcactgtaa tacgtgctta actttgctta

1861 aactctctaa ttcccaattt ctgagttaca tttagatatc atattaatta tcatatacat

1921 ttacttc

## Figure 20

### α-L-iduronidase

mRNA

1 gtcacatggg gtgcgcgccc agactccgac ccggaggcgg aaccggcagt gcagcccgaa

61 gccccgcagt ccccgagcac gcgtggccat gcgtcccctg cgcccccgcg ccgcgctgct

121 ggcgctcctg gcctcgctcc tggccgcgcc cccggtggcc ccggccgagg ccccgcacct

181 ggtgcaggtg gacgcggccc gcgcgctgtg gcccctgcgg cgcttctgga ggagcacagg

241 cttctgcccc ccgctgccac acagccaggc tgaccagtac gtcctcagct gggaccagca

301 gctcaacctc gcctatgtgg gcgccgtccc tcaccgcggc atcaagcagg tccggaccca

361 ctggctgctg gagcttgtca ccaccagggg gtccactgga cggggcctga gctacaactt

421 cacccacctg gacgggtact tggaccttct cagggagaac cagctcctcc cagggtttga

481 gctgatgggc agcgcctcgg gccacttcac tgactttgag gacaagcagc aggtgtttga

541 gtggaaggac ttggtctcca gcctggccag gagatacatc ggtaggtacg gactggcgca

601 tgtttccaag tggaacttcg agacgtggaa tgagccagac caccacgact ttgacaacgt

661 ctccatgacc atgcaaggct tcctgaacta ctacgatgcc tgctcggagg gtctgcgcgc

721 cgccagcccc gccctgcggc tgggaggccc cggcgactcc ttccacaccc caccgcgatc

781 cccgctgagc tggggcctcc tgcgccactg ccacgacggt accaacttct tcactgggga

841 ggcgggcgtg cggctggact acatctccct ccacaggaag ggtgcgcgca gctccatctc

901 catcctggag caggagaagg tcgtcgcgca gcagatccgg cagctcttcc ccaagttcgc

961 ggacaccccc atttacaacg acgaggcgga cccgctggtg ggctggtccc tgccacagcc

1021 gtggagggcg gacgtgacct acgcggccat ggtggtgaag gtcatcgcgc agcatcagaa

1081 cctgctactg gccaacacca cctccgcctt cccctacgcg ctcctgagca acgacaatgc

1141 cttcctgagc taccacccgc acccettcgc gcagcgcacg ctcaccgcgc gcttccaggt

1201 caacaacacc cgcccgccgc acgtgcagct gttgcgcaag ccggtgctca cggccatggg

1261 gctgctggcg ctgctggatg aggagcagct ctgggccgaa gtgtcgcagg ccgggaccgt

1321 cctggacagc aaccacacgg tgggcgtcct ggccagcgcc caccgccccc agggcccggc

1381 cgacgcctgg cgcgccgcgg tgctgatcta cgcgagcgac gacacccgcg cccaccccaa

1441 ccgcagcgtc gcggtgaccc tgcggctgcg cggggtgccc cccggcccgg gcctggtcta

1501 cgtcacgcgc tacctggaca acgggctctg cagccccgac ggcgagtggc ggcgcctggg

1561 ccggcccgtc ttccccacgg cagagcagtt ccggcgcatg cgcgcggctg aggacccggt

1621 ggccgcggcg ccccgcccct tacccgccgg cggccgcctg accctgcgcc ccgcgctgcg

1681 gctgccgtcg cttttgctgg tgcacgtgtg tgcgcgcccc gagaagccgc ccgggcaggt

1741 cacgcggctc cgcgccctgc ccctgaccca agggcagctg gttctggtct ggtcggatga

1801 acacgtgggc tccaagtgcc tgtggacata cgagatccag ttctctcagg acggtaaggc

1861 gtacaccccg gtcagcagga agccatcgac cttcaacctc tttgtgttca gcccagacac

1921 aggtgctgtc tctggctcct accgagttcg agccctggac tactgggccc gaccaggccc

1981 cttctcggac cctgtgccgt acctggaggt ccctgtgcca agagggcccc catccccggg

2041 caatccatga gcctgtgctg agccccagtg ggttgcacct ccaccggcag tcagcgagct

2101 ggggctgcac tgtgcccatg ctgccctccc atcacccccct ttgcaatata tttttatatt

2161 ttattatttt cttttatatc ttggtaaaaa aaaaaaa

## Figure 21

**β-glucosidase**

mRNA

1 gctaacctag tgcctatagc taaggcaggt acctgcatcc ttgtttttgt ttagtggatc

61 ctctatcctt cagagactct ggaacccctg tggtcttctc ttcatctaat gaccctgagg

121 ggatggagtt ttcaagtcct tccagagagg aatgtcccaa gcctttgagt agggtaagca

181 tcatggctgg cagcctcaca ggtttgcttc tacttcaggc agtgtcgtgg gcatcaggtg

241 cccgcccctg catccctaaa agcttcggct acagctcggt ggtgtgtgtc tgcaatgcca

301 catactgtga ctcctttgac ccccegacct ttcctgccct tggtaccttc agccgctatg

361 agagtacacg cagtgggcga cggatggagc tgagtatggg gcccatccag gctaatcaca

421 cgggcacagg cctgctactg accctgcagc cagaacagaa gttccagaaa gtgaagggat

481 ttggaggggc catgacagat gctgctgctc tcaacatcct tgccctgtca ccccctgccc

541 aaaatttgct acttaaatcg tacttctctg aagaaggaat cggatataac atcatccggg

601 tacccatggc cagctgtgac ttctccatcc gcacctacac ctatgcagac acccctgatg

661 atttccagtt gcacaacttc agcctcccag aggaagatac caagctcaag atacccctga

721 ttcaccgagc cctgcagttg gcccagcgtc ccgtttcact ccttgccagc ccctggacat

781 cacccacttg gctcaagacc aatggagcgg tgaatgggaa ggggtcactc aagggacagc

841 ccggagacat ctaccaccag acctgggcca gatactttgt gaagttcctg gatgcctatg

901 ctgagcacaa gttacagttc tgggcagtga cagctgaaaa tgagccttct gctgggctgt

961 tgagtggata ccccttccag tgcctgggct tcacccctga acatcagcga gacttcattg

1021 cccgtgacct aggtcctacc ctcgccaaca gtactcacca caatgtccgc ctactcatgc

1081 tggatgacca acgcttgctg ctgccccact gggcaaaggt ggtactgaca gacccagaag

1141 cagctaaata tgttcatggc attgctgtac attggtacct ggactttctg gctccagcca

1201 aagccaccct aggggagaca caccgcctgt tccccaacac catgctcttt gcctcagagg

1261 cctgtgtggg ctccaagttc tgggagcaga gtgtgcggct aggctcctgg gatcgaggga

1321 tgcagtacag ccacagcatc atcacgaacc tcctgtacca tgtggtcggc tggaccgact

1381 ggaaccttgc cctgaacccc gaaggaggac ccaattgggt gcgtaacttt gtcgacagtc

1441 ccatcattgt agacatcacc aaggacacgt tttacaaaca gcccatgttc taccaccttg

1501 gccacttcag caagttcatt cctgagggct cccagagagt ggggctggtt gccagtcaga

1561 agaacgacct ggacgcagtg gcactgatgc atcccgatgg ctctgctgtt gtggtcgtgc

1621 taaaccgctc ctctaaggat gtgcctctta ccatcaagga tcctgctgtg ggcttcctgg

1681 agacaatctc acctggctac tccattcaca cctacctgtg gcatcgccag tgatggagca

1741 gatactcaag gaggcactgg gctcagcctg ggcattaaag ggacagagtc agctcacacg

1801 ctgtctgtga ctaaagaggg cacagcaggg ccagtgtgag cttacagcga cgtaagccca

1861 ggggcaatgg tttgggtgac tcactttccc ctctaggtgg tgcccagggc tggaggcccc

1921 tagaaaaaga tcagtaagcc ccagtgtccc cccagccccc atgcttatgt gaacatgcgc

1981 tgtgtgctgc ttgctttgga aactngcctg ggtccaggcc tagggtgagc tcactgtccg

2041 tacaaacaca agatcagggc tgagggtaag gaaaagaaga gactaggaaa gctgggccca

2101 aaactggaga ctgtttgtct ttcctagaga tgcagaactg ggcccgtgga gcagcagtgt

2161 cagcatcagg gcggaagcct taaagcagca gcgggtgtgc ccaggcaccc agatgattcc

2221 tatggcacca gccaggaaaa atggcagctc ttaaaggaga aaatgtttga gccca

## Figure 22

**α-galactosidase**

mRNA

1 aggttaatct taaaagccca ggttacccgc ggaaatttat gctgtccggt caccgtgaca

61 atgcagctga ggaacccaga actacatctg ggctgcgcgc ttgcgcttcg cttcctggcc

121 ctcgtttcct gggacatccc tggggctaga gcactggaca atggattggc aaggacgcct

181 accatgggct ggctgcactg ggagcgcttc atgtgcaacc ttgactgcca ggaagagcca

241 gattcctgca tcagtgagaa gctcttcatg gagatggcag agctcatggt ctcagaaggc

301 tggaaggatg caggttatga gtacctctgc attgatgact gttggatggc tccccaaaga

361 gattcagaag gcagacttca ggcagaccct cagcgctttc ctcatgggat tcgccagcta

421 gctaattatg ttcacagcaa aggactgaag ctagggattt atgcagatgt tggaaataaa

481 acctgcgcag gcttccctgg gagttttgga tactacgaca ttgatgccca gacctttgct

541 gactggggag tagatctgct aaaatttgat ggttgttact gtgacagttt ggaaaatttg

601 gcagatggtt ataagcacat gtccttggcc ctgaatagga ctggcagaag cattgtgtac

661 tcctgtgagt ggcctcttta tatgtggccc tttcaaaagc ccaattatac agaaatccga

721 cagtactgca atcactggcg aaattttgct gacattgatg attcctggaa aagtataaag

781 agtatcttgg actggacatc ttttaaccag gagagaattg ttgatgttgc tggaccaggg

841 ggttggaatg acccagatat gttagtgatt ggcaactttg gcctcagctg gaatcagcaa

901 gtaactcaga tggccctctg ggctatcatg gctgctcctt tattcatgtc taatgacctc

961 cgacacatca gccctcaagc caaagctctc cttcaggata aggacgtaat tgccatcaat

1021 caggacccct tgggcaagca agggtaccag cttagacagg gagacaactt tgaagtgtgg

1081 gaacgacctc tctcaggctt agcctgggct gtagctatga taaaccggca ggagattggt

1141 ggacctcgct cttataccat cgcagttgct tccctgggta aaggagtggc ctgtaatcct

1201 gcctgcttca tcacacagct cctccctgtg aaaaggaagc tagggttcta tgaatggact

1261 tcaaggttaa gaagtcacat aaatcccaca ggcactgttt tgcttcagct agaaaataca

1321 atgcagatgt cattaaaaga cttactttaa

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60456378 B
- US 5242687 A
- US 5601828 A
- US 5623056 A
- WO 02102852 A
- US 4954437 A
- US 4703008 A
- US 6610290 B
- US 5672346 A
- US 6143292 A
- US 6534052 B
- US 4518584 A
- US 4737462 A
- US 5605703 A
- US 5811238 A
- US 5873458 A
- US 5830696 A
- US 5939250 A
- US 5763239 A
- US 5965408 A
- US 5945325 A
- WO 9526412 A
- WO 9531566 A
- US 6156497 A
- US 6228646 B
- US 9701019 W
- US 9701048 W
- US 6531456 B


### Non-patent literature cited in the description


- **SAMBHARA ; MILLER.** *Science,* 1991, vol. 252, 1424-1427
- **RAMMENSEE et al.** *Eur. J. Immunol.,* 1982, vol. 12, 930-934
- **FINK et al.** *J. Exp. Med.,* 1983, vol. 157, 141-154
- **RAMMENSEE et al.** *J. Immunol.,* 1984, vol. 132, 668-672
- **HAMBOR et al.** *J. Immunol.,* 1990, vol. 145, 1646-1652
- **HAMBOR et al.** *Intern. Immunol.,* 1990, vol. 2, 8856-8879
- **KAPLAN et al.** *Proc. Natl. Acad. Sci. USA,* 1989, vol. 86, 8512-8515
- **QI et al.** *J. Exp. Med.,* 1996, vol. 183, 1973-1980
- **WEN et al.** *Blood,* 1993, vol. 82, 1507-1516
- **MARTINIUK et al.** *DNA Cell Biol.,* 1990, vol. 9, 85-94
- **MARTINIUK et al.** *Proc. Natl. Acad. Sci. USA,* 1986, vol. 83, 9641-9644
- **HOEFSLOOT et al.** *Eur. Mol. Biol. Organ.,* 1988, vol. 7, 1697-1704
- **DEVEREUX et al.** *Nucleic Acids Res.,* 1984, vol. 12, 387
- **WALDER et al.** *Gene,* 1986, vol. 42, 133
- **BAUER et al.** *Gene,* 1985, vol. 37, 73
- **CRAIK.** *Biotechniques,* January 1995, 12-19
- **LEAHY.** *Faseb J.,* 1995, vol. 9, 17-25
- **LEAHY et al.** *Cell,* 1992, vol. 68, 1145-62
- **NAKAYAMA et al.** *Immunogenetics,* 1989, vol. 30, 393-7
- **GAO ; JAKOBSEN.** *Immunology Today,* 2000, vol. 21, 630-636
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *PNAS USA,* 1988, vol. 85, 2444
- **DEVEREUX et al.** *Nucl. Acid Res.,* 1984, vol. 12, 387-395
- Current Methods in Sequence Comparison and Analysis. Macromolecule Sequencing and Synthesis, Selected Methods and Applications. Alan R. Liss, Inc, 1988, 127-149
- **FENG ; DOOLITTLE.** *J. Mol. Evol.,* 1987, vol. 35, 351-360
- **HIGGINS ; SHARP.** *CABIOS,* 1989, vol. 5, 151-153
- **ALTSCHUL et al.** *J. Mol. Biol.,* 1990, vol. 215, 403-410
- **KARLIN et al.** *PNAS USA,* vol. 90, 5873-5787
- **ALTSCHUL et al.** *Methods in Enzymology,* 1996, vol. 266, 460-480
- **ALTSCHUL et al.** *Nucleic Acids Res.,* vol. 25, 3389-3402
- **POUWELS et al.** Cloning Vectors: A Laboratory Manual. Elsevior, 1985
- **LUCKOW et al.** *Bio/Technology,* 1988, vol. 6, 47

- **SCOPES, R.** Protein Purification. Springer-Verlag, 1982
- **COTTEN et al.** *PNAS (USA,* 1992, vol. 89, 6094-6098
- **CURIEL et al.** *PNAS (USA,* 1991, vol. 88, 8850-8854
- **WAGNER et al.** *PNAS (USA,* 1992, vol. 89, 6099-6103
- **WATKINS et al.** *Targeting Adenovirus-Mediated Gene Delivery with Recombinant Antibodies*
- **YEH ; PERRICAUDET.** *FASEB J.,* 1997, vol. 11, 615
- **BYUN et al.** *Gene Ther.,* 1996, vol. 3 (9), 780-8
- **SIOUD et al.** *J. Mol. Biol.,* 1991, vol. 242, 831-835
- **ROSENFELD et al.** *Science,* 1991, vol. 252, 431-434
- **JAFFE et al.** *Clin. Res.,* 1991, vol. 39 (2), 302A
- **ROSENFELD et al.** *Clin. Res.,* 1991, vol. 39 (2), 311A
- **BERKNER.** *BioTechniques,* 1988, vol. 6, 616-629